# EUROPEAN PATENT APPLICATION

(11) **EP 4 286 401 A1**
(43) Date of publication of application: **06.12.2023**
(21) Application number: 22177185.0
(22) Date of filing: 03.06.2022
(51) Int. Cl.: C07K 14/315, C12N 9/22, C12N 15/62

(54) **USE OF AN ARTIFICIAL PROTEIN OR OF A NUCLEIC ACID ENCODING THE ARTIFICIAL PROTEIN FOR PROVIDING A FUNCTIONAL RIBONUCLEOPROTEIN COMPLEX**

(71) Applicant: Technische Universität Dresden, 01069 Dresden (DE)
(72) Inventor: LINDEMANN, Dirk, 01307 Dresden (DE); LINDEL, Fabian, 01307 Dresden (DE); RICHTER, Stefanie, 01307 Dresden (DE)
(74) Representative: PATERIS Patentanwälte PartmbB

(57) **Abstract**

The invention discloses the use of an artificial protein or of a nucleic acid encoding the artificial protein for providing a functional ribonucleoprotein (RNP) complex, wherein the artificial protein comprises a Csy4 protein, a Cas9 protein, and a linker of 5 to 60 amino acids connecting the Csy4 protein and the Cas9 protein, wherein the linker does not comprise a 2A peptide. Further disclosed is an *in vitro* method for providing a functional RNP complex in a target cell. Further disclosed are the use of a nucleic acid encoding an artificial protein for producing recombinant virus particles, wherein the recombinant virus particles are suitable for providing a functional RNP complex in a target cell, and an *in vitro* method for producing recombinant virus particles, wherein the recombinant virus particles are suitable for providing a functional RNP complex in a target cell.

## Description

### Field of the invention

The present invention relates to the use of an artificial protein or of a nucleic acid encoding the artificial protein for providing a functional ribonucleoprotein (RNP) complex, wherein the artificial protein comprises a Csy4 protein, a Cas9 protein, and a linker of 5 to 60 amino acids connecting the Csy4 protein and the Cas9 protein, wherein the linker does not comprise a 2A peptide. The invention further relates to an *in vitro* method for providing a functional RNP complex in a target cell. The invention further relates to the use of a nucleic acid encoding an artificial protein for producing recombinant virus particles, wherein the recombinant virus particles are suitable for providing a functional RNP complex in a target cell. The invention further relates to an *in vitro* method for producing recombinant virus particles, wherein the recombinant virus particles are suitable for providing a functional RNP complex in a target cell.

### Background of the invention

The adaptation of RNA-guided CRISPR (clustered regularly interspaced short palindromic repeat)/Cas (CRISPR associated protein) systems, a natural defense machinery of bacteria and archaea against invading nucleic acids, has revolutionized genome engineering. In particular, the programmable, easy to handle CRISPR/Cas9 system has become an essential tool for basic research in biology and medicine as well as for clinical applications.

The classical CRISPR/Cas9 system comprises two components, namely a Cas9 deoxyribonuclease, which provides the required catalytic activity for nucleic acid manipulation, and a Cas9-specific single guide RNA (sgRNA), which determines the target nucleic acid sequence specificity.

Tsai et al. 2014 describes a modified CRISPR/Cas9 system that employs a Csy4 ribonuclease as a second enzyme. The Cas9 and Csy4 nucleases are employed as separate proteins. The modified CRISPR/Cas9 system further employs a modified chimeric pre-sgRNA composed of a Cas9-specific sgRNA sequence flanked at least at its 3' end by a Csy4-specific cleavage sequence. The modified pre-sgRNA allows RNA polymerase II mediated expression of the pre-sgRNA. After processing of the pre-sgRNA by the Csy4 nuclease into a mature sgRNA, the mature sgRNA can be recognized by the Cas9 nuclease and assembled into a ribonucleoprotein (RNP) complex that enables editing of the target nucleic acid sequence.

While the modified CRISPR/Cas9 system offers some advantages over the classical CRISPR/Cas9 system, further improvements of the modified CRISPR/Cas9 system are desirable. For example, in certain cell types, a provision of the two nuclease components of the RNP complex is generally difficult so that, if any, only low overall concentrations of the two components in the cells can be achieved. For these and other cases, it is desirable to increase the efficiency of editing of the target nucleic acid sequence.

Another drawback of CRISPR/Cas9 systems used to date is that the RNP complex may lead to unintended permanent genetic modifications in the cell that is to be manipulated.

Therefore, new means for providing a functional RNP complex are needed that overcome the current limitations.

### Summary of the invention

In a first aspect, the present invention relates to the use of an artificial protein or of a nucleic acid encoding the artificial protein for providing a functional ribonucleoprotein (RNP) complex, wherein the artificial protein comprises
a Csy4 protein,
a Cas9 protein, and
a linker of 5 to 60 amino acids connecting the Csy4 protein and the Cas9 protein, wherein the linker does not comprise a 2A peptide.

In a second aspect, the invention relates to an *in vitro* method for providing a functional ribonucleoprotein (RNP) complex in a target cell, the method comprising the step of introducing a component (a) and a component (b) into the target cell, wherein the component (a) is an artificial protein or a nucleic acid encoding the artificial protein, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide;
and wherein the component (b) is
either
   (b-1) a Csy4/Cas9-single guide RNA (Csy4/Cas9-sgRNA), wherein the Csy4/Cas9-sgRNA comprises
      a classical Cas9-sgRNA sequence, and
      a Csy4 cleavage sequence located downstream of the classical Cas9-sgRNA sequence,
         or
   (b-2) a pre-Csy4/Cas9-sgRNA or a nucleic acid encoding the pre-Csy4/Cas9-sgRNA, wherein the pre-Csy4/Cas9-sgRNA comprises
      a classical Cas9-sgRNA sequence,
      a Csy4 cleavage sequence located downstream of the classical Cas9-sgRNA sequence, and
      an additional Csy4 cleavage sequence located directly upstream of the classical Cas9-sgRNA sequence.

In a third aspect, the invention relates to the use of a nucleic acid encoding an artificial protein for producing recombinant virus particles, wherein the recombinant virus particles are suitable for providing a functional ribonucleoprotein (RNP) complex in a target cell, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide.

In a further aspect, the present invention relates to an *in vitro* method for producing recombinant virus particles, wherein the recombinant virus particles are suitable for providing a functional ribonucleoprotein (RNP) complex in a target cell, the method comprising the steps of
(a) introducing
   (i) a nucleic acid encoding an artificial protein, wherein the artificial protein comprises
      a Csy4 protein,
      a Cas9 protein,
      a linker of 5 to 60 amino acids connecting the Csy4 protein and the Cas9 protein, and
      at least one nuclear localization sequence,
      wherein the linker does not comprise a 2A peptide;
         and
   (ii) at least one viral packaging vector
   into a plurality of packaging cells, thereby obtaining a plurality of producer cells;
(b) culturing the producer cells in a medium, thereby obtaining the recombinant virus particles in the medium; and
(c) separating the medium from the producer cells.

### Brief description of the figures

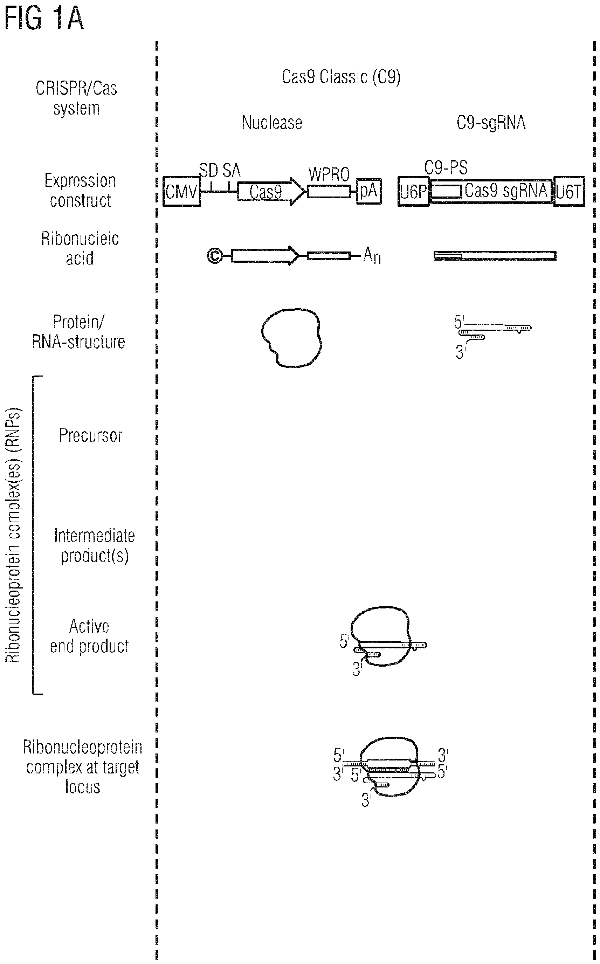

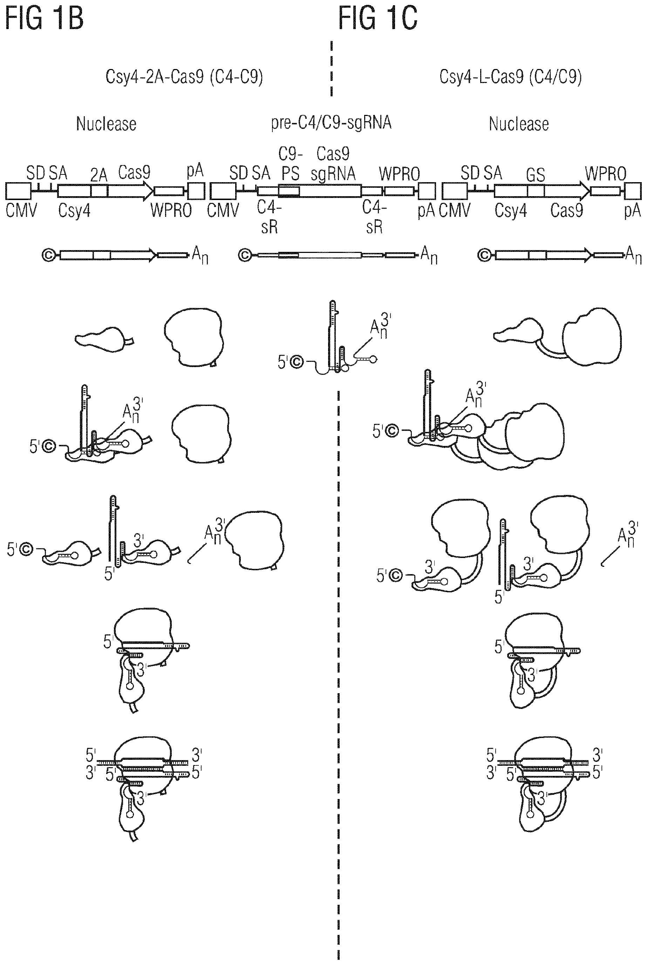

Fig. 1 visualizes the prior art (Fig. 1A, 1B) in comparison to the underlying principle of the present invention (Fig. 1C).

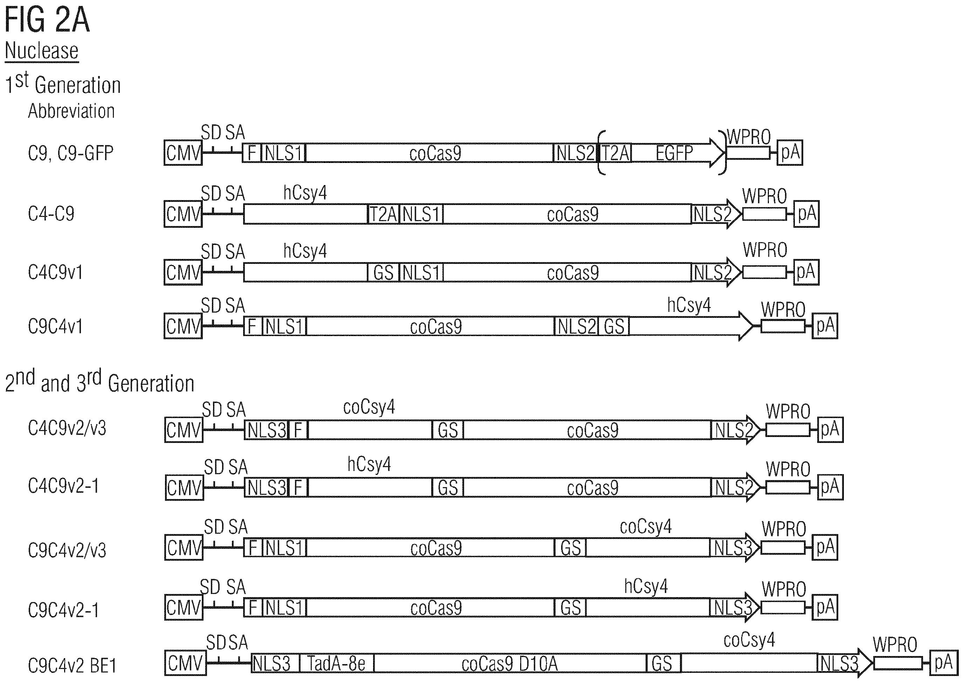

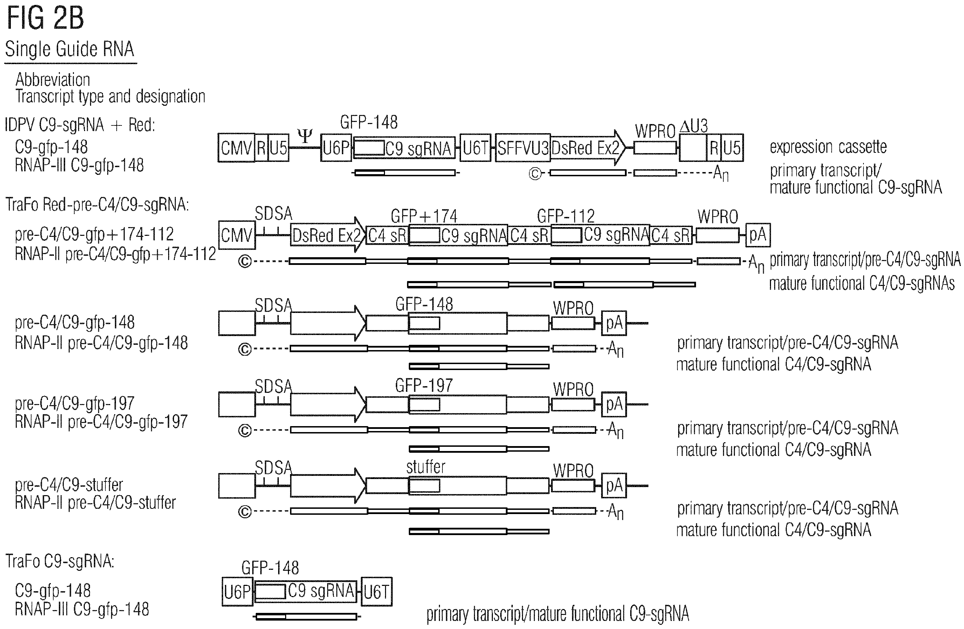

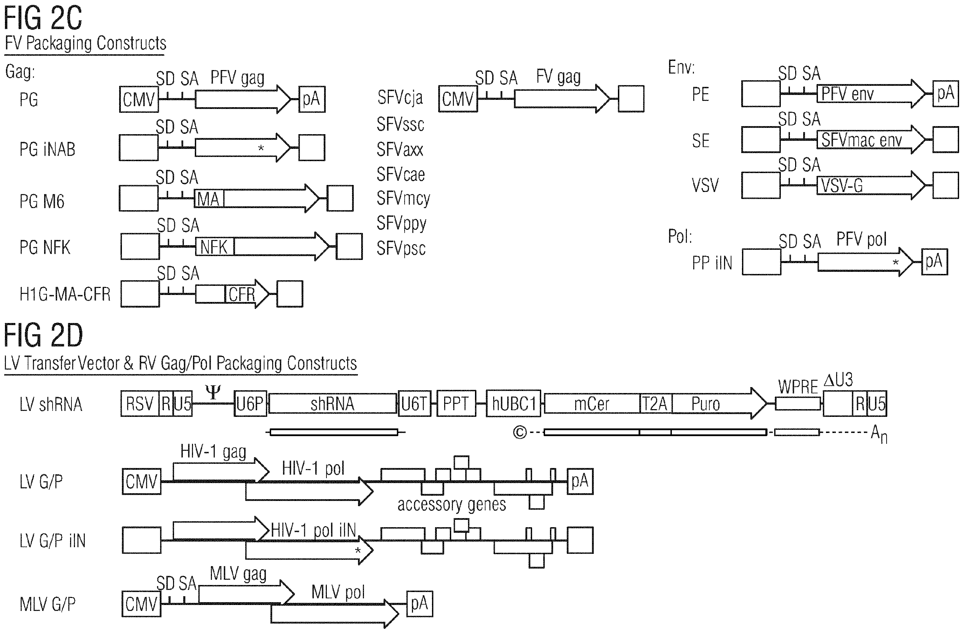

Fig. 2 shows schematic representations of the various constructs used by the inventors. Fig. 2A shows schematic representations of the TraFo vector genome-encoding expression constructs comprising open reading frames (ORFs) for various CRISPR nucleases. Fig. 2B shows schematic representations of the TraFo expression constructs encoding egfp-specific pre-C4/C9-sgRNA mRNAs (TraFo Red-pre-C4/C9-sgRNA) or U6 promoter-driven RNA polymerase III Cas9 sgRNAs (Trafo C9-sgRNA), or IDPV vector genomes comprising U6 promoter-driven Cas9-sgRNAs (IDPV C9-sgRNA + Red) expression cassettes. The primary transcripts derived from the individual expression cassettes, representing the mature functional C9-sgRNAs or the pre-C4/C9-sgRNAs, as indicated, are also shown. Furthermore, the mature functional C4/C9-sgRNAs, derived from the pre-C4/C9-sgRNAs by Csy4-mediated processing, are displayed. Fig. 2C shows schematic representations of the FV packaging constructs used for the generation of TraFo or IDPV vector particles. Fig. 2D shows schematic representations of the lentiviral (LV) transfer vector and retroviral (RV) Gag/Pol packaging constructs used. The meaning of the abbreviations is given in the Example section.

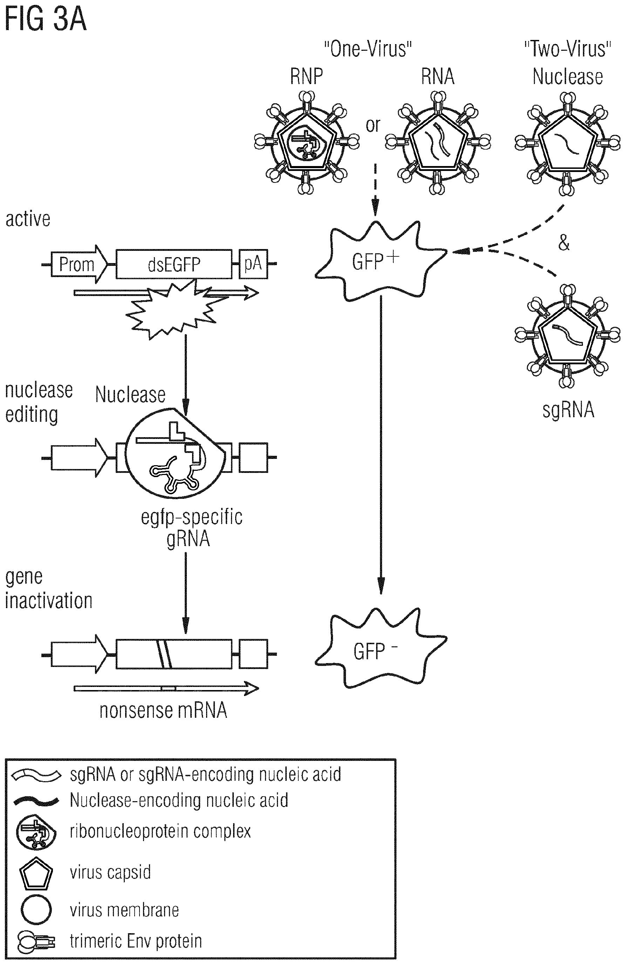

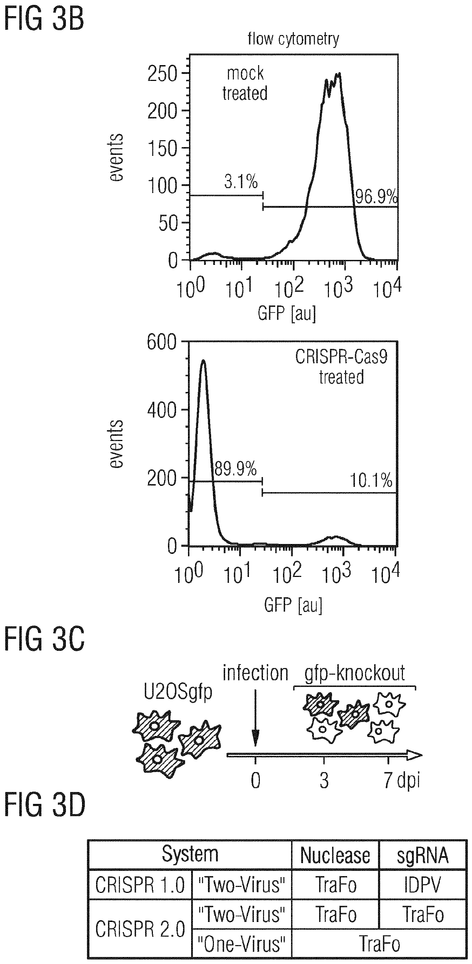

Fig. 3 shows the cell-based, flow cytometric reporter gene inactivation assay that was used for examination and quantitative comparison of genome editing capacities of different CRISPR vector systems. Fig. 3A illustrates the underlying principle of the assay (Prom.: promoter; pA: polyadenylation signal sequence). Fig. 3B shows inactivation of the reporter gene after treatment of the cells with a PFV-derived CRISPR vector system. The percentage of GFP expressing mock treated cells and the percentage of GFP expressing cells after treatment with the CRISPR vector system (CRISPR-Cas9 treated cells) was determined seven days post treatment by flow cytometry (au: arbitrary units). Fig. 3C shows a time scale of the assay (dpi: days post infection). Fig. 3D shows the composition of different CRISPR vector systems used (TraFo: PFV RNA transfer vector; IDPV: integration-deficient PFV vector).

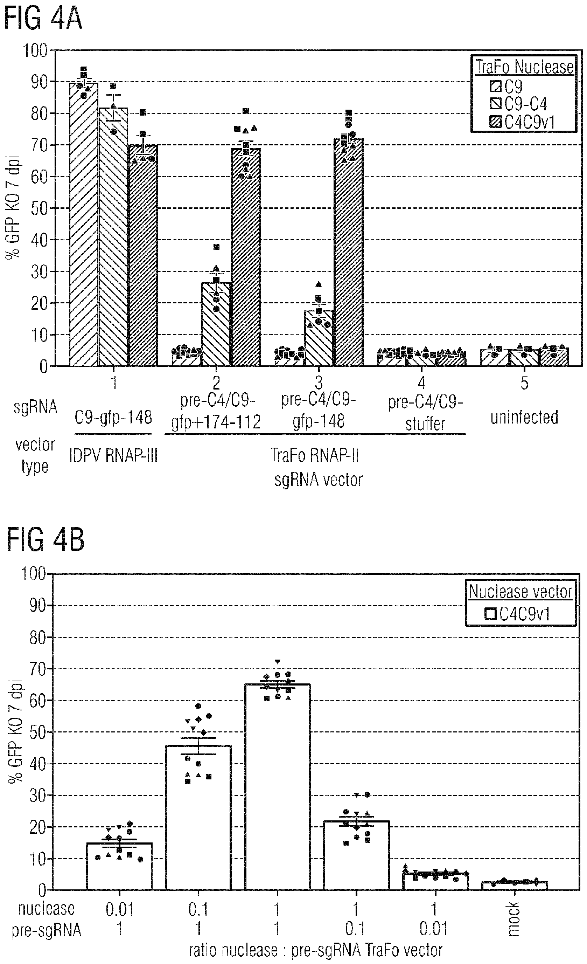

Fig. 4 shows the gene inactivation efficiency of "Two-Virus" CRISPR vector systems using different nuclease - sgRNA combinations and amounts. Fig. 4A shows a comparison of various TraFo nuclease-encoding vectors in combination with TraFo or IDPV sgRNA vectors. U2OS*gfp* cells were incubated with 500 µl of TraFo vector supernatant encoding different nucleases as indicated in combination with 500 µl supernatants containing either TraFo vectors containing different RNAP-II derived pre-C4/C9-sgRNA transcripts as indicated or IDPV vectors encoding a RNAP-III promoter (U6) driven C9-sgRNA gfp-148 expression cassette. GFP inactivation was determined at 7 days post infection (dpi) by flow cytometry. Means ±SEM (n=3-10) are shown (SEM: standard error of the mean). Fig. 4B shows the influence of relative ratios and amounts of TraFo chimeric nuclease vectors to TraFo pre-sgRNA vectors on gene inactivation. U2OS*gfp* cells were incubated with different combinations of 500 µl of plain TraFo sgRNA pre-C4/C9-gfp+174-112 vector supernatant or dilutions thereof as indicated and 500 µl of TraFo C4C9v1 nuclease-encoding vector supernatant or dilutions thereof as indicated. GFP inactivation was determined at 7 days post infection (dpi) by flow cytometry. Means ±SEM (n=6-12) are shown.

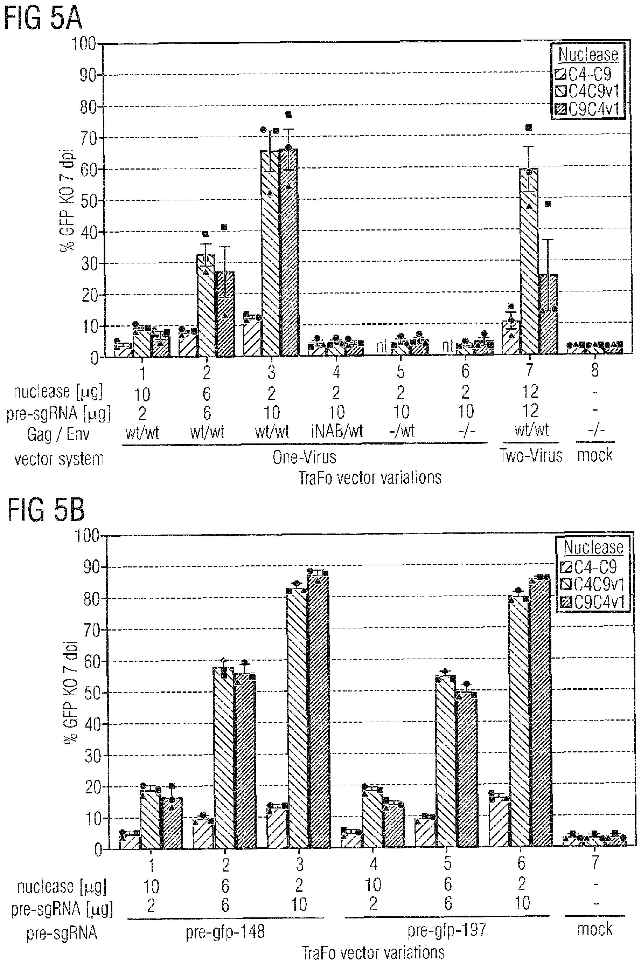

Fig. 5 shows the gene inactivation efficiency of "One-Virus" CRISPR vector systems using different nuclease - sgRNA combinations and amounts. Fig. 5A shows the results of "One-Virus" (One-Virus) TraFo vector supernatants that were produced by transient cotransfection of HEK293T cells with different amounts of various chimeric nuclease encoding expression vectors as indicated in combination with different amounts of pre-C4/C9-sgRNA gfp-148 encoding expression vector and fixed amounts of FV packaging constructs encoding wildtype PFV Gag (wt) or a nucleic acid binding deficient (iNAB) mutant thereof as well as wildtype SFVmac Env (wt). Replacement of one or both FV packaging constructs by non-coding plasmids is indicated by "-". For comparison, "Two-Virus" (Two-Virus) TraFo vector supernatants encoding the same chimeric nucleases or containing pre-C4/C9-sgRNA gfp-148 RNAP-II transcripts were produced in parallel and plain supernatants used at a 1:1 ratio for transduction of U2OS*gfp* cells. GFP inactivation in transduced U2OS*gfp* cells was determined by flow cytometry 7 days post infection (dpi). Means ±SEM (n=3) are shown. Fig. 5B shows the results of "One-Virus" TraFo vector supernatants that were produced by transient cotransfection of HEK293T cells with different amounts of various chimeric nuclease encoding expression vectors as indicated in combination with different amounts of pre-C4/C9-sgRNA gfp-148 (pre-gfp-148) or pre-C4/C9-sgRNA gfp-197 (pre-gfp-197) encoding expression vector and fixed amounts of FV packaging constructs encoding wildtype PFV Gag and wildtype SFVmac Env. GFP inactivation in transduced U2OS*gfp* cells was determined by flow cytometry 7 days post infection (dpi). Means ±SEM (n=3) are shown.

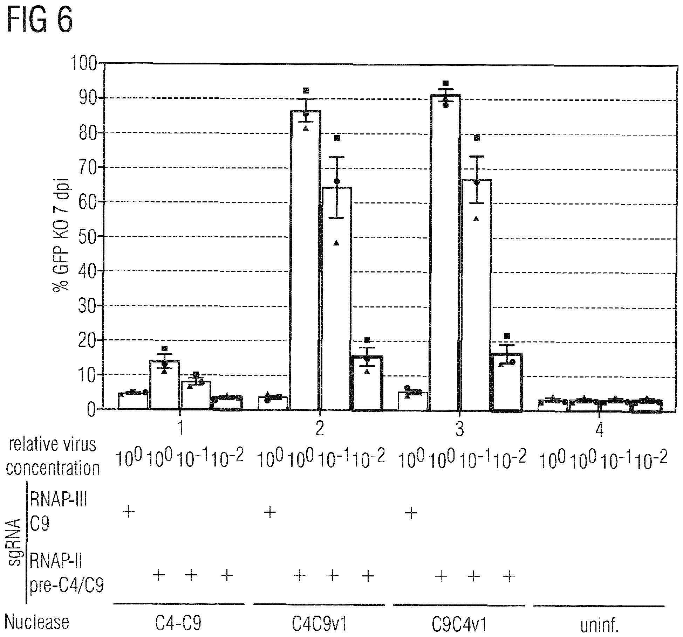

Fig. 6 shows the gene inactivation efficiency of "One-Virus" CRISPR vector systems. "One-Virus" TraFo vector particles encoding different chimeric nucleases were produced in combination with either pFYF1320 EGFP-1 (RNAP-III C9) or pRP2a Red-C4C9gRNA egfp-148 (RNAP-II pre-C4/C9) sgRNA expression constructs at a 2:10 ratio. U2OS*gfp* target cells were incubated with 1 ml of either plain (10⁰),10-fold (10⁻¹) or 100-fold (10⁻²) diluted "One-Virus" TraFo supernatant. GFP inactivation was determined by flow cytometry 7 days post infection (dpi). Means ±SD (n=3) are shown (SD: standard deviation).

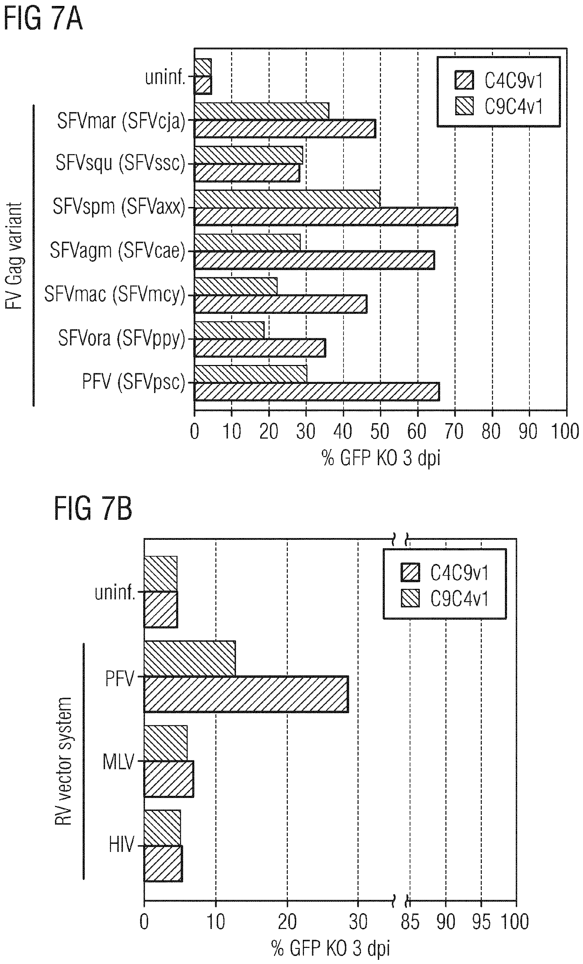

Fig. 7 shows the gene inactivation efficiency of "One-Virus" CRISPR vector systems based on different retrovirus and foamy virus species. "One-virus" "CRISPR 2.0" vector particles encoding C4C9v1- or C9C4v1-combinuclease were produced in combination with corresponding pre-C4/C9-sgRNA gfp-148 transfer vectors at a ratio of 1:11 using packaging components based on different retrovirus species by transient transfection of HEK293T cells. Subsequently, U2OS*gfp* reporter cells were infected with plain cell-free virus supernatant and GFP inactivation was determined 3 days after infection (dpi) by flow cytometry. Fig. 7A shows a comparison of gene inactivation efficiency of "One-Virus" C4C9v1- or C9C4v1-TraFo "CRISPR 2.0" vector supernatants based on different foamy virus species. Identical total amounts (12 µg) of C4C9v1- or C9C4v1-combinuclease and pre-C4/C9-sgRNA gfp-148 transfer vectors in the ratio 1: 11 were used in combination with packaging constructs encoding Gag protein variants of prototype foamy virus (PFV, SFVpsc), Bornean orangutan simian foamy virus (SFVora, SFVppy), Taiwanese macaque simian foamy virus (SFVmac, SFVmcy), Grivet simian foamy virus (SFVagm; SFVcae), Spider monkey simian foamy virus (SFVspm, SFVaxx), Squirrel monkey simian foamy virus (SFVsqu, SFVssc), or White-tufted-ear marmoset simian foamy virus (SFVmar, SFVcja) and each with PFV Env packaging constructs for the production of "One-Virus" TraFo "CRISPR 2.0" vector particles and each with PFV Env packaging constructs were used for the production of "One-Virus" CRISPR vector particles. Shown is GFP gene inactivation efficiency on U2OS*gfp* reporter cells mediated by pure, cell-free virus supernatants determined by flow cytometry 3 dpi (n=1). Fig. 7B shows a comparison of gene inactivation efficiency of "One-Virus" C4C9v1- or C9C4v1-CRISPR vector supernatants based on different retrovirus species. Identical total amounts (7.5 µg) of C4C9v1- or C9C4v1-combinuclease and pre-C4/C9-sgRNAs gfp-148 transfer vectors at a ratio of 1:11 were used in combination with packaging constructs encoding prototype foamy virus (PFV) Gag, mouse leukemia virus (MLV) Gag/Pol, or human immunodeficiency virus type 1 (HIV) Gag/Pol and each with PFV Env packaging constructs were used for the production of "One-Virus" RV-CRISPR vector particles. Shown is the GFP gene inactivation efficiency on U2OS*gfp* reporter cells mediated by plain, cell-free virus supernatants determined by flow cytometry 3 dpi (n=1).

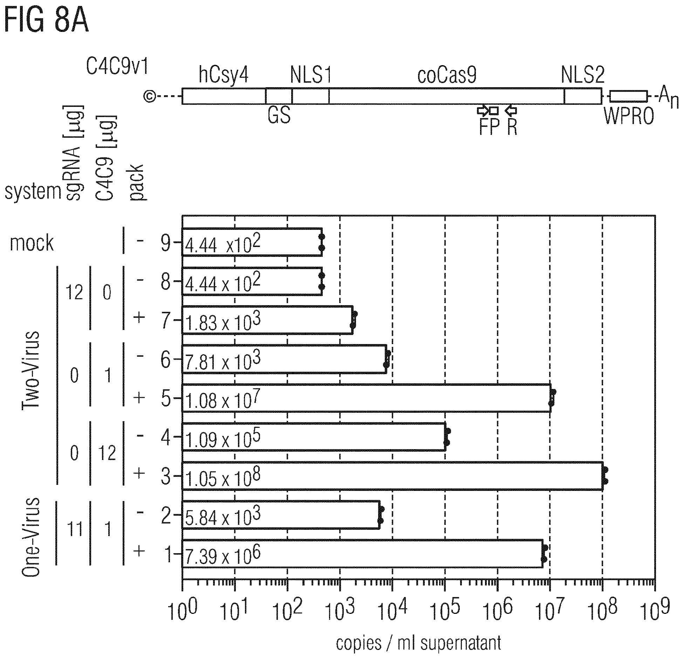

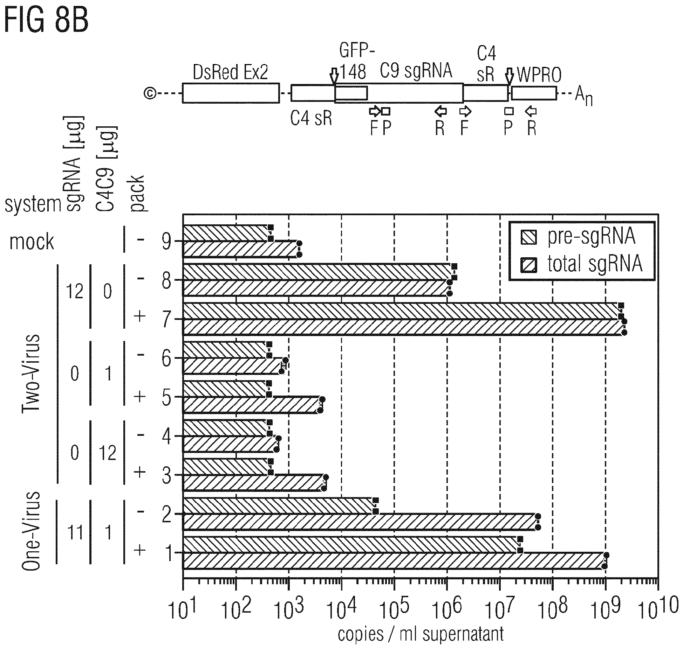

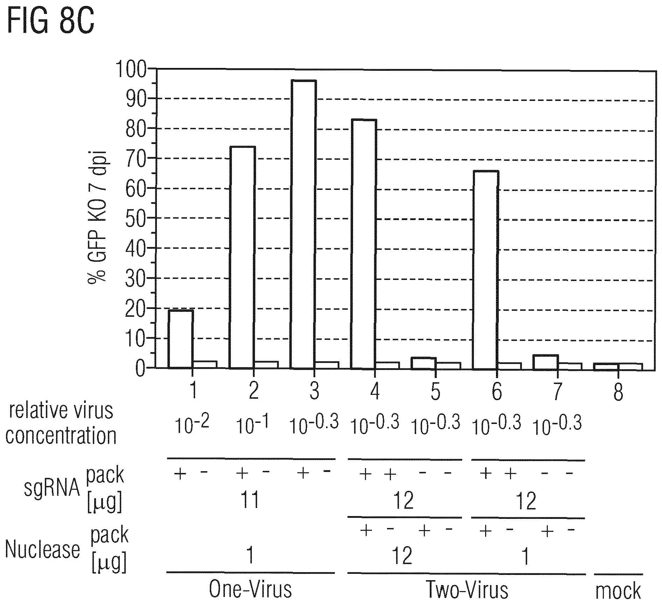

Fig. 8 shows the analysis of the nucleic acid composition of different TraFo "CRISPR 2.0" "One-Virus" or "Two-Virus" vector particles. "One-Virus" (One-Virus) TraFo "CRISPR 2.0" vector particles encoding C4C9v1-combinuclease were produced in combination with corresponding pre-C4/C9-sgRNA gfp-148 transfer vectors at a ratio of 1:11. In case of the "Two-Virus" (Two-Virus) TraFo "CRISPR 2.0" system, separate TraFo nuclease particles with either 1 µg or 12 µg nuclease transfer vector and TraFo pre-C4/C9-sgRNA particles with 12 µg pre-C4/C9-sgRNAs gfp-148 were produced. Corresponding mock supernatants without Gag and Env packaging constructs (pack: -) served as negative controls. For Fig. 8A and Fig. 8B, virus particles or mock particle preparations were obtained from cell-free cell culture supernatants by ultracentrifugation. The intact resuspended mock or viral particle preparations were DNase I digested and then the viral nucleic acids were extracted. In the following, again DNase I digested nucleic acid extracts were subjected to RT-qPCR analysis using Cas9 ORF specific (Fig. 8A) or pre-C4/C9-sgRNA (pre-sgRNA) or total C4/C9-sgRNA (total sgRNA)-specific (Fig. 8B) Taqman primer-probe combinations. In schematic illustrations of the combinuclease or pre-C4/C9sgRNA RNAP-II transcripts, the positions of the Cas9 ORF- or pre-C4/C9-sgRNA and total C4/C9-sgRNA-specific primer-probe combinations used (F: forward primer; R: reverse primer; P: Taqman probe) are indicated. Csy4-mediated processing sites within pre-C4/C9-sgRNA transcripts are indicated with vertical arrows. Shown are the number of nucleic acid copies per ml of plain vector supernatant from technical replicates (n=2) of an individual experiment. Fig. 8C shows the analysis of vector supernatant functionality. U2OS*gfp* target cells were incubated with 1 ml of either 2-fold (10^{-0.3}), 10-fold (10⁻¹), or 100-fold (10⁻²) diluted "One-virus" TraFo supernatant. In the case of the "Two-Virus" vector system, 0.5 ml of plain TraFo combinuclease and 0.5 ml plain TraFo pre-C4/C9-sgRNA supernatant or corresponding control supernatants prepared without packaging constructs (pack: -) were combined. GFP inactivation (% GFP) was determined 7 days post-infection (7 dpi) by flow cytometry. Shown are values obtained using the supernatants of the experiment examined in Fig. 8A and Fig. 8B.

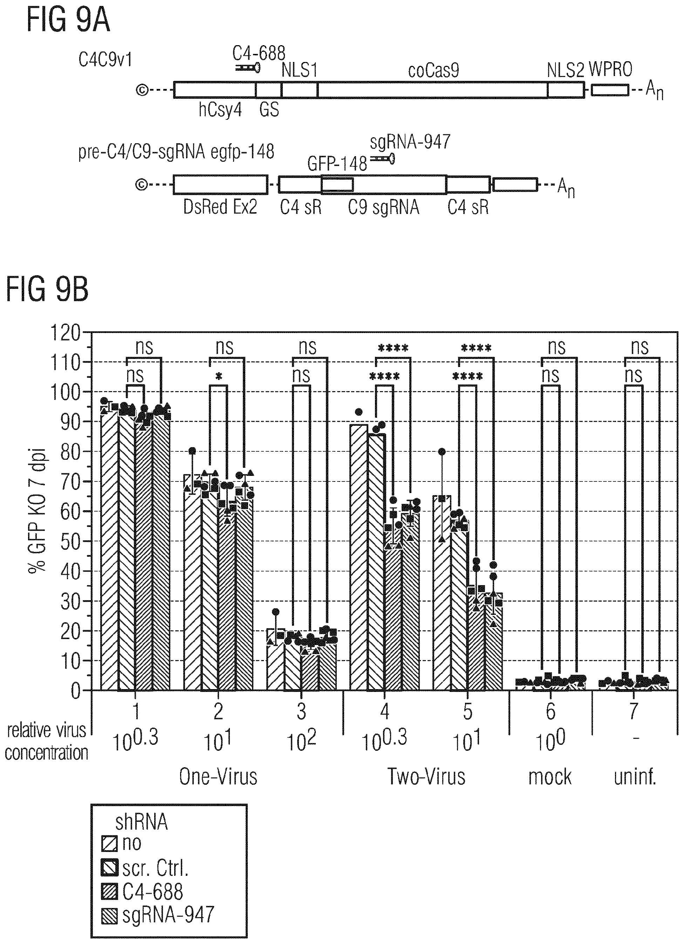

Fig. 9 shows that genome editing by "Two-Virus" but not "One-Virus" TraFo "CRISPR 2.0" vector supernatants is inhibited by target cell expression of CRISPR-Cas9 specific shRNAs. Fig. 9A shows schematic representations of the C4C9v1-combinuclease and pre-C4/C9-sgRNA gfp-148 coding transcripts. The coding or functional regions of the transcripts are shown as different rectangles and the target regions of the shRNAs used, shown as hairpin loops, are indicated. For Fig. 9B, "One-Virus" (One-Virus) or "Two-Virus" (Two-Virus) TraFo vector particles encoding C4C9 nuclease were produced in combination with pre-C4/C9-sgRNA gfp-148 at a ratio of 2:10 or in case of "Two-Virus" TraFo nuclease particles combined with TraFo pre-gfp-148 vector particles. U2OS*gfp* target cell populations stably expressing different CRISPR Cas9-specific shRNA as indicated (no: no shRNA; scr. Ctrl.: scrambled control shRNA; C4-688: Csy4-specific shRNA; sgRNA-947: C9 sgRNA-specific shRNA) following lentiviral vector transduction were incubated with 1 ml of either 2-fold (10^{0.3}),10-fold (10¹) or 100-fold (10²) diluted "One-Virus" TraFo supernatant or various combinations of 500 µl plain (10^{0.3}) or 5-fold diluted (10¹) TraFo nuclease and 500 µl plain (10^{0.3}) or 5-fold (10¹) diluted TraFo pre-sgRNA supernatants. GFP inactivation was determined 7 days post infection (dpi) by flow cytometry. Means ±SEM (n=3-6) derived from 3 independent experiments are shown. Two-way ANOVA with Tukey's multiple-comparisons test was used to assess significance. **p* < 0.05, ***p* < 0.01, ****p* < 0.001, *****p* < 0.0001, ns: not significant (p ≥ 0.05).

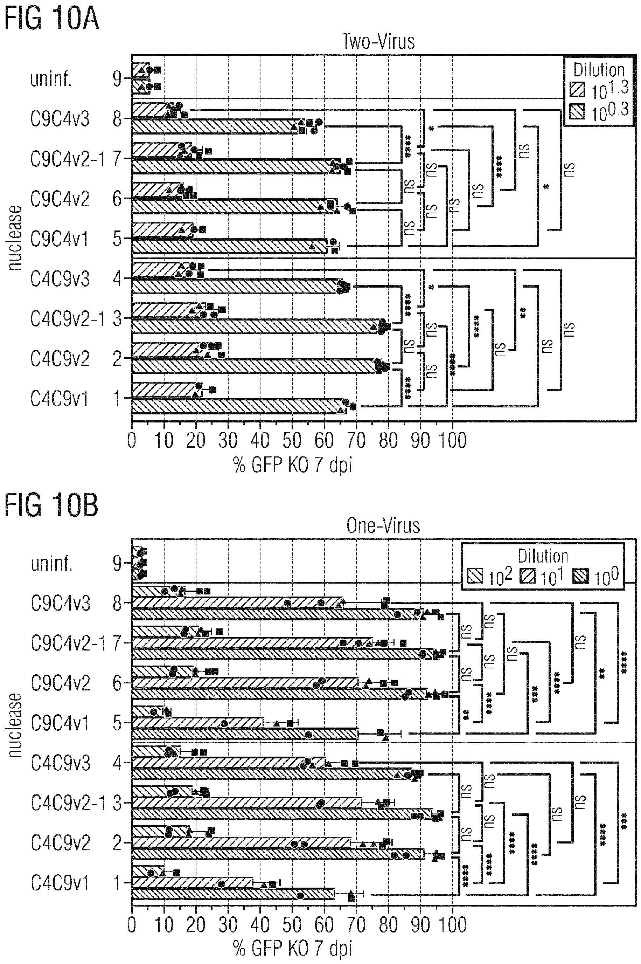

Fig. 10 shows the gene inactivation efficiency of "Two-Virus" and "One-Virus" CRISPR vector systems using different chimeric nucleases. "One-Virus" or "Two-Virus" TraFo vector particles encoding different chimeric Csy4-Cas9 nucleases with different peptide linkers were produced in combination with pre-C4/C9-sgRNA gfp-148 at a 2:10 ratio or in case of "Two-Virus" TraFo nuclease particles combined with TraFo pre-gfp-148 vector particles. U2OS*gfp* target cells were incubated with 1 ml of either various combinations of 500 µl plain or 10-fold (10¹) diluted TraFo nuclease and 500 µl plain or 10-fold (10¹) diluted TraFo pre-sgRNA supernatants (Fig. 8A) or plain (10°), 10-fold (10¹) or 100-fold (10²) diluted "One-Virus" TraFo supernatant (Fig. 8B). GFP inactivation was determined by flow cytometry 7 days post infection (dpi). Means ±SEM (n=3-6) are shown. Two-way ANOVA with Tukey's multiple-comparisons test was used to assess significance. **p* < 0.05, ***p* < 0.01, ****p* < 0.001, *****p* < 0.0001, ns: not significant (p ≥ 0.05).

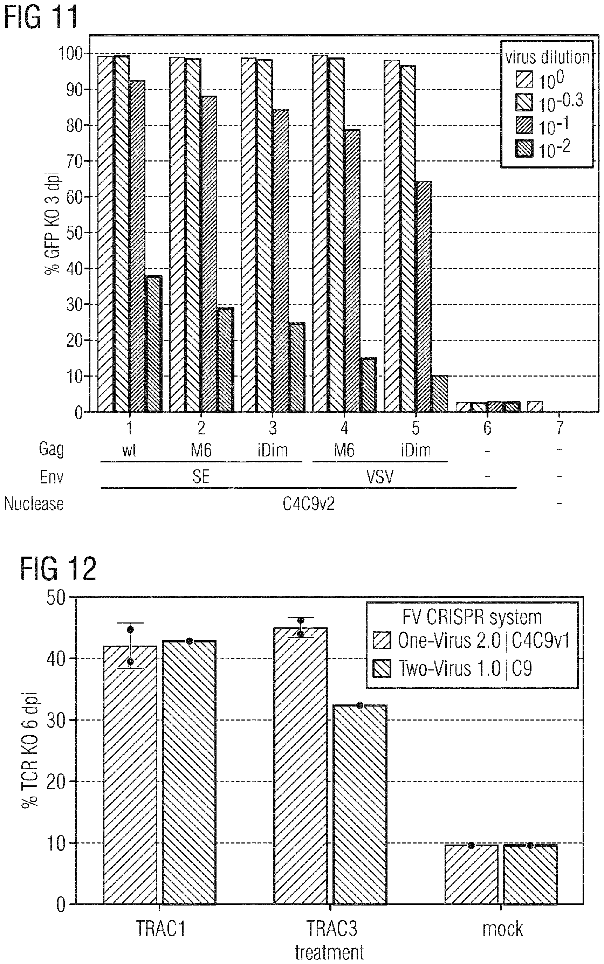

Fig. 11 shows the gene inactivation efficiency of "One-Virus" TraFo pseudotype vector systems. "One-Virus" "CRISPR 2.0" vector particles encoding C4C9v2 nuclease were produced in combination with corresponding pre-C4/C9-sgRNA gfp-148 transfer vectors at a ratio of 1:11 using different packaging components (as described) by transient transfection of HEK293T cells. Subsequently, U2OS*gfp* reporter cells were infected with plain cell-free virus supernatant or dilutions thereof, and GFP inactivation was determined 3 days after infection (dpi) by flow cytometry. Shown are values from one experiment. wt: wildtype PFV Gag; M6: PFV Gag M6 mutant; iDim: iDimerize pseudotype packaging system consisting of PFV Gag-NFK mutant and HIV-1 MA-CFR membrane targeting domain.
Fig. 12 shows inactivation of the T-cell receptor (TCR) by "One-Virus" TraFo "CRISPR 2.0" vector supernatants in Jurkat cells. "One-Virus" TraFo "CRISPR 2.0" vector particles encoding C4C9v1 nuclease (One-Virus 2.0 C4C9v1) were produced in combination with human T-cell receptor alpha chain (TRAC) gene specific pre-C4/C9-sgRNAs (TRAC1 or TRAC3) at a ratio of 2:10. For comparison, "Two-Virus" TraFo "CRISPR 1.0" vector particles (Two-Virus 1.0 C9) composed of TraFo particles encoding Cerulean-tagged Cas9 and integration-deficient lentiviral (IDLV) vector particles encoding a U6 promoter-driven C9-sgRNA (TRAC1 or TRAC3) were produced. Jurkat cells were transduced with plain "One-Virus" TraFo "CRISPR 2.0" supernatants or with a 4:1 mixture (nuclease-encoding to sgRNA-encoding vector supernatant) of plain "Two-Virus" TraFo "CRISPR 1.0" supernatants. TCR inactivation was determined 6 days post infection (dpi) by flow cytometry employing a CD3-specific antibody staining. Shown are values from a single experiment.

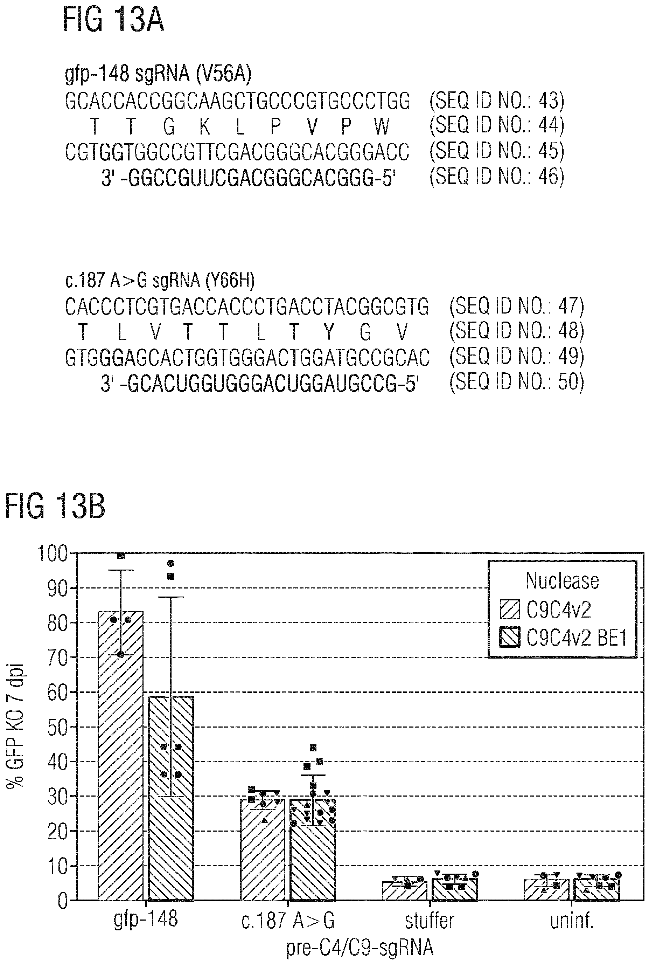

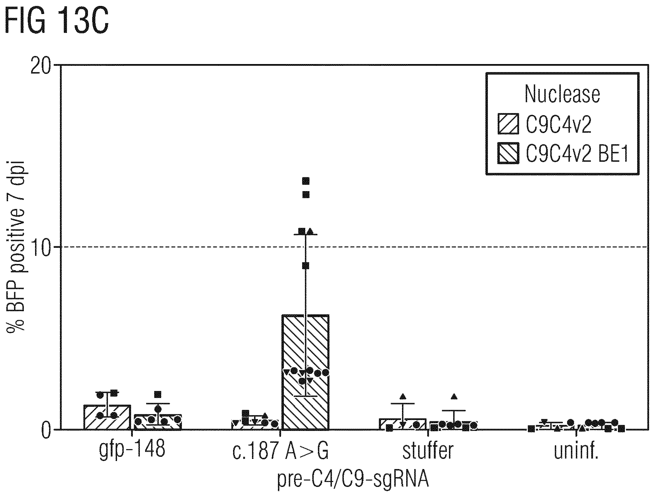

Fig. 13 shows gene editing by "One-Virus" TraFo "CRISPR 2.0" base editor vector supernatants. "One-Virus" TraFo "CRISPR 2.0" vector particles encoding C9C4v2 or C9C4v2 BE1 nuclease were produced in combination with pre-C4/C9-sgRNAs gfp-148 or c.187 A>G (Fig. 13A) at a ratio of 1:10. Plain TraFo supernatants were used to transduce U2OS*gfp* target cells and GFP inactivation (Fig. 13B) or GFP to BFP conversion (Fig. 13C) was determined 7 days post infection (dpi) by flow cytometry. Mean values ±SD (n=4-13) obtained in three independent experiments are shown.

### Detailed description of the invention

In a first aspect, the invention relates to the use of an artificial protein or of a nucleic acid encoding the artificial protein for providing a functional ribonucleoprotein (RNP) complex, wherein the artificial protein comprises
a Csy4 protein,
a Cas9 protein, and
a linker of 5 to 60 amino acids connecting the Csy4 protein and the Cas9 protein, wherein the linker does not comprise a 2A peptide.

The artificial protein is a fusion protein. The artificial protein is a chimeric protein that has the Csy4 protein and the Cas9 protein linked to each other by the linker. Thereby Csy4 protein-mediated and Cas9 protein-mediated activities are combined in a single multi-domain protein. The artificial protein can be expressed from a single nucleic acid molecule harboring coding sequences for the Csy4 protein, the Cas9 protein and the linker, all fused in frame. Accordingly, the nucleic acid encoding the artificial protein comprises a coding sequence for the Csy4 protein, a coding sequence for the Cas9 protein and a coding sequence for the linker, all fused in frame. The coding sequence for the linker is arranged between the coding sequence for the Csy4 protein and the coding sequence for the Cas9 protein. The artificial protein can be provided as a recombinant protein. To do so, the artificial protein may be produced and purified, for example, using standard biotechnological methods such as the expression of the recombinant artificial protein in bacteria.

The artificial protein may be part of a larger multifunctional fusion protein complex. In other words, the chimeric protein that has the Csy4 protein and the Cas9 protein linked to each other by the linker may be fused to further protein domains such as, for example, retroviral capsid protein domains. Fusion to retroviral capsid protein domains may be useful, for example, for generating engineered virus-like particles for packaging and delivering the artificial protein into a cell.

The artificial protein or the nucleic acid encoding the artificial protein is used for providing a functional RNP complex.

The term "RNP complex" as used herein refers to an RNP complex that is formed by two components, namely by the artificial protein and a Cas9-single guide RNA (Cas9-sgRNA). The latter is also designated Cas9-guide RNA (Cas9-gRNA) or Cas9-specific single guide RNA. The term "Cas9-sgRNA" as used herein refers to any sgRNA molecule that comprises at least one classical Cas9-sgRNA sequence and that is capable of assembling with the Cas9 protein of the artificial protein via the at least one classical Cas9-sgRNA sequence. The term "classical Cas9-sgRNA sequence" as used herein refers to a Cas9-sgRNA sequence that is devoid of any additional elements. The classical Cas9-sgRNA sequence is a short artificial RNA sequence that is composed of at least one CRISPR RNA (crRNA) sequence element and a trans-activating CRISPR RNA (tracrRNA) sequence element. The classical Cas9-sgRNA sequence comprises a sequence that is complementary to at least 17 nucleotides, preferably 17-20 nucleotides, of the target nucleic acid.

As mentioned above, the Cas9-sgRNA comprises at least one classical Cas9-sgRNA sequence. Therefore, the Cas9-sgRNA may consist of a classical Cas9-sgRNA sequence. Alternatively, the Cas9-sgRNA may comprise at least one classical Cas9-sgRNA sequence and at least one additional element, provided that the Cas9-sgRNA is still capable of assembling with the Cas9 protein of the artificial protein via the at least one classical Cas9-sgRNA sequence. In the latter case, the classical Cas9-sgRNA sequence is part of a larger sgRNA molecule, in particular of a chimeric sgRNA molecule. The chimeric sgRNA molecule particularly is a Csy4/Cas9-sgRNA. The Csy4/Cas9-sgRNA will be described further below.

The Cas9-sgRNA assembles with the Cas9 protein and directs the Cas9 protein to a specific target nucleic acid. The directing to the target nucleic acid is mediated by a sequence of at least 17-20 nucleotides of the classical Cas9-sgRNA sequence, which are designed to be complementary to and interact via base pair complementarity with the complementary strand of the target nucleic acid that lies next to a protospacer adjacent motif (PAM) sequence. In general, the target nucleic acid is DNA.

The term "functional RNP complex" as used herein refers to an RNP complex that is capable of site-directed binding to the target nucleic acid. In other words, the functional RNP complex is capable of interacting with the target nucleic acid in a site-directed manner. The expressions "binding to the target nucleic acid" and "interacting with the target nucleic acid" are used interchangeably herein. Upon binding, the functional RNP complex is capable of exerting the activity that is required for its intended use. The activity is exerted by the Cas9 protein of the functional RNP complex. Due to the assembly of the Cas9 protein with the Cas9-sgRNA in the functional RNP complex, the activity is exerted in a site-specific manner. The functional RNP complex can also be referred to as biologically active RNP complex. In most cases, the intended use of the functional RNP complex is altering the target nucleic acid. A synonymous term for altering the target nucleic acid is editing the target nucleic acid. The alteration of the target nucleic acid can be, for example, an insertion, a deletion, a modification or a replacement in the target nucleic acid. In most cases, the target nucleic acid is a target gene or a part of a target gene ("gene editing"). The target nucleic acid can also be a part of a target genome ("genome editing"). The intended use of the functional RNP complex can be different from altering the target nucleic acid. For example, the intended use can be activating or repressing the expression of the target nucleic acid, in particular in case the target nucleic acid is a target gene. As another example, the intended use can be gene labelling on chromosomes, e.g. by Cas9-mediated fluorescence in situ hybridization (FISH) using a functional RNP complex in which the Cas9 protein of the artificial protein is further coupled to a fluorescent protein.

The functional RNP complex can be provided in a target cell. The term "target cell" as used herein refers to a cell in which the functional RNP complex is to be provided. The cell can be a cell that is maintained in a laboratory, i.e. outside of a living organism, such as a cell of a cell line or a tissue. The target cell may be, for example, a mammalian cell. Functional RNP complexes are used in target cells, for example, for editing of a target nucleic acid. The functional RNP complex may be formed in the target cell or may be pre-assembled outside of the target cell prior to introduction into the target cell. The two components that are needed for the formation of the functional RNP complex in the target cell may be introduced into the target cell by, for example, viral transduction of the target cell. It is also possible that the target cell is genetically engineered to provide one of the two components so that only the other one of the two components needs to be introduced into the target cell. For example, the target cell may be genetically engineered to provide the Cas9-sgRNA. In particular, the target cell may be a genetically engineered target cell that comprises a nucleic acid encoding a pre-Csy4/Cas9-sgRNA in its genome in a manner that facilitates the expression of the pre-Csy4/Cas9-sgRNA in the target cell. The pre-Csy4/Cas9-sgRNA will be described further below.

Alternatively, the target cell may be a genetically engineered target cell that comprises a nucleic acid encoding a mature Csy4/Cas9-sgRNA in its genome in a manner that facilitates the expression of the mature Csy4/Cas9-sgRNA in the target cell.

Alternatively, the functional RNP complex can be provided in a cell-free solution, i.e. not in a cell. To do so, the artificial protein and the Cas9-sgRNA are each provided in a cell-free solution and incubated with each other, so that the functional RNP complex can assemble in the solution. Functional RNP complexes are used in a cell-free solution, for example, for editing of a target nucleic acid in the solution. Functional RNP complexes provided in this manner may also be of interest for diagnostic applications performed in a cell-free solution, i.e. not in a cell. Further, functional RNP complexes provided in a cell-free solution may be used for delivering the functional RNP complexes into a target cell, for example by means of liposome-mediated transfection or electroporation of the target cell.

The two components of the functional RNP complex may be provided as follows. The artificial protein may be provided, for example, as a recombinant protein. As an alternative of using the artificial protein, the nucleic acid encoding the artificial protein can be used. The nucleic acid encoding the artificial protein may be a DNA molecule that is provided, for example, as part of an RNA polymerase II driven eukaryotic expression construct, resulting in the transcription of a 5'-capped and 3'-polyadenylated mRNA, which is then translated into the artificial protein. As another example, the nucleic acid encoding the artificial protein may be an RNA molecule, for example an *in vitro* (cell-free) transcribed mRNA or a chemically synthesized and modified RNA, that is then translated into the artificial protein.

To provide the Cas9-sgRNA, the Cas9-sgRNA may be produced, for example, by *in vitro* (cell-free) transcription using, for example, T7 RNA polymerase. Alternatively, the Cas9-sgRNA may be provided, for example, as a chemically synthesized and modified RNA. As another alternative, a DNA molecule encoding the Cas9-sgRNA may be used. The DNA molecule encoding the Cas9-sgRNA may be provided, for example, as part of an RNA polymerase III driven eukaryotic expression construct, resulting in the transcription of the Cas9-sgRNA. RNA transcripts of RNA polymerase III are not 5'-capped and are not 3'-polyadenylated, but usually comprise a few additional nucleotides at their 3' end that are due to a 3' termination signal. In case the Cas9-sgRNA is a Csy4/Cas9-sgRNA, the sgRNA can further be provided via a pre-Csy4/Cas9-sgRNA, which can be processed into the mature Csy4/Cas9-sgRNA, or via a nucleic acid encoding the pre-Csy4/Cas9-sgRNA. The nucleic acid encoding the pre-Csy4/Cas9-sgRNA is a DNA molecule that may be provided, for example, as part of an RNA polymerase III driven eukaryotic expression construct, resulting in the transcription of the pre-Csy4/Cas9-sgRNA. Alternatively, the DNA molecule encoding the pre-Csy4/Cas9-sgRNA may be provided, for example, as part of an RNA polymerase II driven eukaryotic expression construct, resulting in the transcription of a 5'-capped and 3'-polyadenylated pre-Csy4/Cas9-sgRNA. The latter option is facilitated by the chimeric design of the pre-Csy4/Cas9-sgRNA.

The artificial protein comprises the Csy4 protein. The Csy4 protein is a member of the CRISPR (clustered regularly interspaced short palindromic repeat)-associated endoribonuclease Cas6 family. The Cys4 protein is also designated "CRISPR-associated endonuclease Cas6/Csy4". The Cys4 protein may be, for example, the Csy4 protein of *Pseudomonas aeruginosa.*

The term "Csy4 protein" as used herein refers to a functional Csy4 protein, i.e. to a Csy4 protein that is capable of exerting its enzymatic nuclease activity. The Csy4 protein can also be referred to as enzymatically active Csy4 protein, Csy4 enzyme, Csy4 nuclease or Csy4 ribonuclease.

The artificial protein further comprises the Cas9 protein. The Cas9 protein is the CRISPR-associated endonuclease Cas9, which is also designated CRISPR associated protein 9. The Cas9 protein may be, for example, the Cas9 protein of *Streptococcus pyogenes.*

The Cas9 protein is a dual RNA-guided DNA endonuclease enzyme. It has two endonuclease domains called the RuvC and HNH domains.

The term "Cas9 protein" as used herein refers to a functional Cas9 protein, i.e. to a Cas9 protein that is capable of exerting the activity that is required for the intended use of the Cas9 protein. In most cases, the intended use of the Cas9 protein is editing of a target nucleic acid and the activity that is required for that use is the enzymatic nuclease activity of the Cas9 protein. In such cases, the Cas9 protein can also be referred to as enzymatically active Cas9 protein, Cas9 enzyme, Cas9 nuclease or Cas9 deoxyribonuclease. Depending on the intended use, the activity that is required for the intended use of the Cas9 protein can be different from its nuclease activity. For example, if the intended use is activating or repressing gene expression, the activity required for the intended use can be limited to the binding of the Cas9 protein to its sgRNA and to the DNA strand that is being targeted as target nucleic acid. Accordingly, the Cas9 protein may be a variant of a native Cas9 protein such as dCas9. The variant termed dCas9, which is also known as dead Cas9 or Cas9 Endonuclease Dead, is a mutant form of the Cas9 protein whose endonuclease activity is removed through point mutations (for example D10A and H840A) in its endonuclease domains. The ability of the catalytically inactivated dCas9 protein to bind to Cas9-sgRNA and the target nucleic acid can be exploited for sequence-specific repression of gene expression (so-called "CRISPR interference") or for RNA-guided recruitment of an heterologous effector domain to a specific target nucleic acid by fusing the dCas9 protein to the effector domain, for example to a transcriptional activation domain for activating gene expression. The dCas9 protein is used, for example, in dCas9-based artificial transcription factors or Cas9 base editors. Base editing using Cas9 base editors is a CRISPR-Cas9-based genome editing technology that enables the direct, irreversible conversion of a specific DNA base into another at a targeted genomic locus without generating double-stranded DNA breaks.

Another example of a variant of a native Cas9 protein that is capable of exerting the activity that is required for the intended use of the Cas9 protein is Cas9 nickase. Cas9 nickase is a variant of the Cas9 protein that differs therefrom by a point mutation (for example D10A) in the RuvC nuclease domain, which enables it to nick, but not cleave, DNA. Cas9 nickase can also be used to generate double-stranded DNA breaks by targeting two genomic loci in close proximity.

The Cas9 protein preferably has its native enzymatic nuclease activity, i.e. the Cas9 protein is a Cas9 nuclease. In that case, the artificial protein comprises a Csy4 nuclease and a Cas9 nuclease. Such an artificial protein can also be referred to as, for example, combinuclease, chimeric nuclease or chimeric enzyme.

The artificial protein further comprises the linker of 5 to 60 amino acids connecting the Csy4 protein and the Cas9 protein. The linker is a peptide linker. The linker covalently joins the Csy4 protein and the Cas9 protein. The linker may be an artificially designed amino acid sequence or a linker derived from a naturally occurring amino acid sequence connecting two protein domains in a protein. The linker can also be composed of a first portion corresponding to a naturally occurring amino acid sequence and a second portion being an artificially designed amino acid sequence. The linker preferably is an artificially designed amino acid sequence.

The linker may be a flexible linker, i.e. a linker that facilitates a flexible movement of the Csy4 protein and the Cas9 protein with respect to one another. Alternatively, the linker may be a rigid linker.

The linker is adapted to ensure a permanent linkage of the Csy4 protein and the Cas9 protein. In other words, the linker permanently connects the Csy4 protein and the Cas9 protein throughout the intended use of the artificial protein. In other words, the linker is stable in a target cell and in a cell-free solution. The linker thus prevents a separation of the Csy4 protein and the Cas9 protein of the artificial protein. In other words, the linker is a linker that does not lead to separate Csy4 and Cas9 proteins. For this reason, the linker does not comprise a 2A peptide.

The term "2A peptide" as used herein refers to a peptide of 18 to 22 amino acids which induces ribosomal skipping during translation of a protein in a cell. As a result, an mRNA encoding a protein that comprises a 2A peptide is translated into two separate proteins. Several 2A peptides such as T2A, P2A, E2A and F2A are known. 2A peptides have a core consensus amino acid sequence which is DxExNPGP (SEQ ID NO.: 1), wherein x is any amino acid. 2A peptides are also referred to as self-cleaving peptides, which is misleading since the resulting two proteins are due to ribosomal skipping during translation and not due to a proteolytic cleavage within the 2A peptide sequence. This means that strictly speaking, on the protein level, a linker comprising a 2A peptide will not be found. However, in the prior art, fusion proteins having linkers that comprise a 2A peptide are often depicted. This is the reason why the feature that the linker does not comprise a 2A peptide is explicitly mentioned in relation to the present invention. In the prior art, linkers comprising a 2A peptide are used in fusion constructs of a first protein and a second protein in case the first and second protein are to be expressed from a single transcript but are not intended to be linked with each other on the protein level. In contrast, according to the invention, the linker permanently connects the Csy4 protein and the Cas9 protein. Accordingly, it is required that the linker does not comprise a 2A peptide.

The amino acid sequences of the most relevant 2A peptides are:

| | |
|---|---|
| T2A peptide: | EGRGSLLTCGDVEENPGP (SEQ ID NO.: 33) |
| P2A peptide: | ATNFSLLKQAGDVEENPGP (SEQ ID NO.: 84) |
| E2A peptide: | QCTNYALLKLAGDVESNPGP (SEQ ID NO.: 85) |
| F2A peptide: | VKQTLNFDLLKLAGDVESNPGP (SEQ ID NO.: 86) |

The absence of a 2A peptide in the linker can be easily verified by known laboratory techniques such as Western blotting using anti-Csy4 antibodies and/or anti-Cas9 antibodies. The size of the proteins detected by Western blotting under denaturing conditions will reveal whether the artificial protein is intact, i.e. whether the Csy4 protein and the Cas9 protein are connected to each other on the protein level.

The nucleic acid encoding the artificial protein can be a DNA molecule or an RNA molecule. In case the nucleic acid encoding the artificial protein is a DNA molecule, the DNA molecule preferably is provided as part of an expression construct, such as an RNA polymerase II driven eukaryotic expression construct that results in the transcription of a 5'-capped and 3'-polyadenylated mRNA, which is then translated into the artificial protein. The expression construct further comprises a promoter sequence, for example a cytomegalovirus (CMV) promoter sequence, and the DNA molecule is operably linked to the promoter sequence. The expression construct preferably is a plasm id or a viral expression vector (also referred to as transfer vector), both of which can be easily introduced into a cell. In case the nucleic acid encoding the artificial protein is an RNA molecule, the RNA molecule can be, for example, an mRNA molecule, such as an *in vitro* (cell-free) transcribed mRNA molecule (transcribed from a DNA molecule encoding the artificial protein), or a synthetic (i.e. chemically synthesized and modified) RNA molecule. As mentioned above, the nucleic acid encoding the artificial protein comprises a coding sequence for the Csy4 protein, a coding sequence for the Cas9 protein, and a coding sequence for the linker, all fused in frame. The coding sequence for the linker is arranged between the coding sequence for the Csy4 protein and the coding sequence for the Cas9 protein.

The inventors surprisingly found that the use of the artificial protein or of the nucleic acid encoding the artificial protein results in a much more efficient formation of the functional RNP complex compared to using the Cas9 protein only or to using the Csy4 and Cas9 proteins as separate proteins. The inventors found that the use of the artificial protein or of the nucleic acid encoding the artificial protein results in a much more efficient assembly of the Cas9 protein with the classical Cas9-sgRNA sequence of a Cas9-sgRNA, in particular a Csy4/Cas9-sgRNA, compared to using the Csy4 and Cas9 proteins as separate proteins. The more efficient formation of the functional RNP complex facilitates a more efficient site-directed binding of the functional RNP complex to the target nucleic acid and thus a more efficient activity of the functional RNP complex, for example a more efficient editing of the target nucleic acid. The more efficient activity is particular advantageous at conditions of low overall concentrations of the two components of the functional RNP complex.

In a preferred embodiment, the artificial protein is the artificial protein of SEQ ID NO.: 51, 53, 55, 57, 59, 61, 63, 65 or 67.

In a further preferred embodiment, the artificial protein is the artificial protein of SEQ ID NO.: 55, 57, 59, 61, 63, 65 or 67.

In a preferred embodiment, the nucleic acid encoding the artificial protein is a DNA molecule, wherein the DNA molecule is the DNA molecule of SEQ ID NO.: 52, 54, 56, 58, 60, 62, 64, 66 or 68. The DNA molecules of SEQ ID NO.: 52, 54, 56, 58, 60, 62, 64, 66 and 68 encode the artificial proteins of SEQ ID NO.: 51, 53, 55, 57, 59, 61, 63, 65 and 67, respectively (Table 3C).

In a further preferred embodiment, the DNA molecule is the DNA molecule of SEQ ID NO.: 56, 58, 60, 62, 64, 66 or 68.

The inventors found that the order of the Csy4 and Cas9 proteins in the artificial protein is not decisive. This means that the C-terminus of the Csy4 protein can be linked to the N-terminus of the Cas9 protein via the linker or vice versa. Accordingly, in a preferred embodiment, the linker links the C-terminus of the Csy4 protein to the N-terminus of the Cas9 protein or the linker links the C-terminus of the Cas9 protein to the N-terminus of the Csy4 protein. In a further preferred embodiment, the linker links the C-terminus of the Csy4 protein to the N-terminus of the Cas9 protein.

In a preferred embodiment, the linker has 5 to 50 amino acids.

In a preferred embodiment, the linker is a flexible linker, i.e. a linker that facilitates a flexible movement of the Csy4 protein and the Cas9 protein with respect to one another. The resulting mobility of the Csy4 protein and the Cas9 protein with respect to one another may promote the assembly of the Cas9 protein of the artificial protein with the classical Cas9-sgRNA sequence of a Csy4/Cas9-sgRNA during the formation of the functional RNP complex. The Csy4/Cas9-sgRNA will be described further below.

A flexible linker is generally rich in small, non-polar (e.g. glycine) or polar (e.g. serine or threonine) amino acids to provide good flexibility and solubility.

In a preferred embodiment, the linker comprises at least one GGGGS (SEQ ID NO.: 2) motif, preferably at least three GGGGS (SEQ ID NO.: 2) motifs, more preferred seven GGGGS (SEQ ID NO.: 2) motifs. Linkers comprising at least one GGGGS (SEQ ID NO.: 2) motif, which is also referred to as G4S motif or G₄S₁ motif or glycine-serine linker motif, are widely used flexible linkers. The linker may comprise more than one G4S motif, for example two, three, four, five, six, seven, eight, nine or ten G4S motifs.

In a preferred embodiment, the linker comprises three, five or seven G4S motifs, preferably seven G4S motifs. The inventors found that the use of a linker comprising three or more G4S motifs leads to an efficient formation of the functional RNP complex and thus to an efficient activity of the functional RNP complex. The inventors further found that increasing the number of G4S motifs, for example from three to seven G4S motifs, may further increase the efficiency of the formation and thus the efficiency of the activity of the functional RNP complex.

In case the linker comprises two or more G4S motifs, the G4S motifs preferably are directly consecutively arranged, i.e. one G4S motif directly follows the other. This arrangement can also be indicated as (G4S)ₙ or (G₄S₁)ₙ with n being the number of repeats.

In a further preferred embodiment, the linker consists of (i) the at least one G4S motif, (ii) one to five, preferably two or three, amino acids on the N-terminal end of the at least one G4S motif, and (iii) one to five, preferably two or three, amino acids on the C-terminal end of the at least one G4S motif. Such a linker is substantially formed by the at least one G4S motif, in particular in case the linker comprises two or more G4S motifs directly consecutively arranged, more particular in case the linker comprises three or more G4S motifs directly consecutively arranged. The amino acids at the N- and C-terminal end of the at least one G4S motif are the amino acids that form the N- and C-terminal end of the linker, respectively. In most cases, these amino acids are due to recognition sequences of restriction enzymes that are present in the nucleic acid encoding the artificial protein and that were used for cloning the DNA molecule encoding the artificial protein.

A further example of a flexible linker is a linker comprising a KESGSVSSEQLAQFRSLE (SEQ ID NO.: 3) motif, a KESGSVSSEQLAQFRSLD (SEQ ID NO.: 4) motif or an EGKSSGSGSESKST (SEQ ID NO.: 5) motif. A further example of a flexible linker is a linker comprising a GGS motif.

In another embodiment, the linker is a rigid linker. One example of a rigid linker is a linker comprising at least one EAAAK (SEQ ID NO.: 6) motif, preferably three, four or five EAAAK (SEQ ID NO.: 6) motifs, wherein the three, four or five EAAAK (SEQ ID NO.: 6) motifs preferably are directly consecutively arranged. A further example of a rigid linker is a linker comprising an (AP)₃A motif or an (AP)sA motif. A further example of a rigid linker is a linker comprising an LPEPEP (SEQ ID NO.: 7) motif.

As mentioned above, the linker of the artificial protein is adapted to ensure a permanent linkage of the Csy4 protein and the Cas9 protein. In other words, the linker is stable in a target cell and in a cell-free solution. This implies that, when the functional RNP complex is provided in a target cell, the linker does not comprise a cleavage sequence that can be cleaved by an intracellular enzyme of the target cell. Intracellular enzymes that are capable of cleaving a specific amino acid sequence (cleavage sequence) are endoproteases. Most intracellular endoproteases such as cathepsins are located in the endosomal and lysosomal compartments of the cell. In most cases, the artificial protein will not enter any endocytic or secretory pathway and thus will not be exposed to intracellular endoproteases. The characteristic of the linker to ensure a permanent linkage of the Csy4 protein and the Cas9 protein likewise implies that when the functional RNP complex is provided in a cell-free solution, the linker does not comprise a cleavage sequence that can be cleaved by an enzyme that may be present in the cell-free solution. Alternatively, the cell-free solution may be composed in such a manner that it does not comprise any enzyme that is capable of cleaving the linker of the artificial protein.

The absence of a cleavage sequence that can be cleaved by an intracellular enzyme of a target cell in the linker can be easily verified by known laboratory techniques such as Western blotting using anti-Csy4 antibodies and/or anti-Cas9 antibodies. The size of the proteins detected by Western blotting under denaturing conditions will reveal whether the artificial protein is intact, i.e. whether the Csy4 protein and the Cas9 protein are connected to each other on the protein level. The same applies to the absence of a cleavage sequence that can be cleaved by an enzyme present in a cell-free solution in case the functional RNP complex is provided in the cell-free solution.

In a preferred embodiment, the functional RNP complex is provided in a target cell and the artificial protein further comprises at least one nuclear localization sequence (NLS).

The NLS is also known as nuclear localization signal. The NLS is an amino acid sequence that facilitates the transport of the artificial protein from the cytoplasm into the nucleus of the target cell. Several NLS such as the NLS of the SV40 large T-antigen (SV40 NLS) and the NLS of nucleoplasmin (nucleoplasmin NLS) are known. In the artificial protein, the at least one NLS may be, for example, the SV40 NLS having the sequence PKKKRKV (SEQ ID NO.: 8), the nucleoplasmin NLS having the sequence KRPAATKKAGQAKKKK (SEQ ID NO.: 9), and/or an SV40 bipartite NLS having the sequence KRTADGSEFESPKKKRKV (SEQ ID NO.: 10). The term "SV40 NLS" as used herein refers to the SV40 monopartite NLS. It is known that the SV40 bipartite NLS shows a much stronger function compared to the SV40 NLS. Accordingly, among these two NLSs, the use of the SV40 bipartite NLS is preferred.

In a preferred embodiment, the at least one NLS is not part of the linker.

In a further preferred embodiment, the at least one NLS is located at the N-terminus of the artificial protein or at the C-terminus of the artificial protein. In that case, the NLS is not part of the linker. The inventors found that arranging the NLS at the N-terminus of the artificial protein or at the C-terminus of the artificial protein rather than in the linker may further increase the efficiency of the formation and thus the efficiency of the activity of the functional RNP complex.

Alternatively, the at least one NLS can be located between the Csy4 protein and the Cas9 protein. In that case, the at least one NLS is part of the linker.

In a preferred embodiment, the artificial protein comprises two NLSs. Compared to one NLS, two NLSs lead to a better transport of the artificial protein from the cytoplasm into the nucleus of the target cell. The two NLSs can be the same NLS or can be different NLSs. It is preferred that the two NLSs are different NLSs. For example, the artificial protein may comprise one SV40 NLS and one SV40 bipartite NLS. As another example, the artificial protein may comprise one nucleoplasmin NLS and one SV40 bipartite NLS. One of the two NLSs may be located at the N-terminus or C-terminus of the artificial protein and the other one of the two NLSs may be part of the linker of the artificial protein.

In a further preferred embodiment, one of the two NLSs is located at the N-terminus of the artificial protein and the other one of the two NLSs is located at the C-terminus of the artificial protein. For example, the artificial protein may comprise one SV40 NLS located at the N-terminus of the artificial protein and one SV40 bipartite NLS located at the C-terminus of the artificial protein. As another example, the artificial protein may comprise one nucleoplasmin NLS located at the C-terminus of the artificial protein and one SV40 bipartite NLS located at the N-terminus of the artificial protein. In another embodiment, the two NLSs are consecutively arranged at the N-terminus of the artificial protein. In another embodiment, the two NLSs are consecutively arranged at the C-terminus of the artificial protein. In all these cases, the linker does not comprise any NLS. The inventors found that arranging the NLSs at the N-terminus of the artificial protein and/or at the C-terminus of the artificial protein rather than in the linker may further increase the efficiency of the formation and thus the efficiency of the activity of the functional RNP complex.

In another embodiment, the artificial protein comprises more than two NLSs, wherein one of the NLSs is preferably located at the N-terminus of the artificial protein and another one of the NLSs is preferably located at the C-terminus of the artificial protein. It is preferred that all NLSs are consecutively arranged at the N- and/or C-terminus of the artificial protein so that the linker does not comprise any NLS. In another embodiment, at least one of the NLSs is part of the linker and the other NLSs are arranged at the N- and/or C-terminus of the artificial protein.

In a preferred embodiment, the artificial protein further comprises an affinity tag. The affinity tag can be, for example, a Flag tag peptide (Flag tag). The affinity tag may be located, for example, at the N-terminus of the artificial protein or in a region of the artificial protein that is close to its N-terminus. The affinity tag facilitates purification of the artificial protein by an affinity technique such as affinity chromatography. The affinity tag is particularly useful in case the artificial protein is produced using standard biotechnological methods such as the expression of the recombinant artificial protein in bacteria.

In a preferred embodiment, the artificial protein is associated with a Cas9-single guide RNA (Cas9-sgRNA). In this case, a functional RNP complex is present. Depending on the design of the functional RNP complex, in particular depending on the type of Cas9 protein used, the functional RNP complex may, for example, alter a target nucleic acid, activate the expression of a target nucleic acid or repress the expression of a target nucleic acid. The target nucleic acid preferably is a target gene or a part of a target gene.

In a further preferred embodiment, the Cas9-sgRNA is a Csy4/Cas9-single guide RNA (Csy4/Cas9-sgRNA), wherein the Csy4/Cas9-sgRNA comprises a classical Cas9-sgRNA sequence and a Csy4 cleavage sequence located downstream of the classical Cas9-sgRNA sequence. In this case, i.e. in case the artificial protein is associated with the Csy4/Cas9-sgRNA, the Csy4 protein of the artificial protein is associated with the Csy4 cleavage sequence of the Csy4/Cas9-sgRNA while the Cas9 protein of the artificial protein is associated with the classical Cas9-sgRNA sequence of the Csy4/Cas9-sgRNA. The Csy4/Cas9-sgRNA is thus capable of assembling with the Cas9 protein of the artificial protein via the classical Cas9-sgRNA sequence. The Csy4/Cas9-sgRNA is a chimeric sgRNA. The classical Cas9-sgRNA sequence comprises a sequence that is complementary to at least 17 nucleotides, preferably 17-20 nucleotides, of the target nucleic acid.

The Csy4 cleavage sequence is located downstream of the classical Cas9-sgRNA sequence. The Csy4 cleavage sequence is also designated Csy4 scaffold region. The Csy4 cleavage sequence has a characteristic RNA secondary structure comprising a stem-loop structure. The secondary structure is important for the recognition of the Csy4 cleavage sequence by the Csy4 protein. The Csy4 protein of the artificial protein can recognize and bind the Csy4/Cas9-sgRNA through recognition of the Csy4 cleavage sequence. Accordingly, the Csy4 cleavage sequence is also designated Csy4 recognition sequence or Csy4 recognition and cleavage sequence.

The term "Csy4/Cas9-sgRNA" as used herein refers to the mature Csy4/Cas9-sgRNA. The Csy4/Cas9-sgRNA may be provided as such or may result from processing of a corresponding pre-C4/C9-sgRNA by the Csy4 protein of the artificial protein. The Csy4/Cas9-sgRNA starts with the first nucleotide of the classical Cas9-sgRNA sequence (which is the first nucleotide of the seed sequence of the classical Cas9-sgRNA sequence) and ends with the last nucleotide of the Csy4 cleavage sequence, i.e. there are no additional nucleotides added to the 3' end of the Csy4 cleavage sequence. The Csy4/Cas9-sgRNA is a chimeric sgRNA.

The term "pre-Csy4/Cas9-sgRNA" as used herein refers to a pre-Csy4/Cas9-sgRNA that comprises a classical Cas9-sgRNA sequence, a Csy4 cleavage sequence located downstream of the classical Cas9-sgRNA sequence, and an additional Csy4 cleavage sequence located directly upstream of the classical Cas9-sgRNA sequence. The Csy4 cleavage sequence located downstream of the classical Cas9-sgRNA sequence is also referred to as downstream Csy4 cleavage sequence. The additional Csy4 cleavage sequence located directly upstream of the classical Cas9-sgRNA sequence is also referred to as upstream Csy4 cleavage sequence. The 3' end of the upstream Csy4 cleavage sequence is directly adjacent to the 5' end of the classical Cas9-sgRNA sequence. The pre-Csy4/Cas9-sgRNA is a chimeric pre-sgRNA. During processing of the pre-Csy4/Cas9-sgRNA into the mature Csy4/Cas9-sgRNA by the Csy4 protein, the pre-Csy4/Cas9-sgRNA is cleaved at the 3' end of both the upstream Csy4 cleavage sequence and the downstream Csy4 cleavage sequence. Therefore, the additional Csy4 cleavage sequence located directly upstream of the classical Cas9-sgRNA sequence leads to a Csy4/Cas9-sgRNA that has a 5' end that is without any additional modification or additional nucleotides. As described, this is due to the Csy4-mediated cleavage at the 3' end of the additional Csy4 cleavage sequence.

Examples of pre-Csy4/Cas9-sgRNAs are the pre-Csy4/Cas9-sgRNAs of SEQ ID NO.: 74, 76, 78 and 82.

The Csy4 cleavage sequence is recognized and bound by the Csy4 protein of the artificial protein. The Csy4 protein has the ability to stay bound with high affinity to the Csy4 cleavage sequence. This even applies after cleavage of a pre-Csy4/Cas9-sgRNA at the 3' end of each Csy4 cleavage sequence by the Csy4 protein during processing of the pre-Csy4/Cas9-sgRNA into the mature Csy4/Cas9-sgRNA. As a consequence, when using the artificial protein, processing of the pre-Csy4/Cas9-sgRNA by the Csy4 protein of the artificial protein or binding of a mature Csy4/Cas9-sgRNA by the Csy4 protein of the artificial protein results in a very high local concentration of mature Csy4/Cas9-sgRNA close to the Cas9 protein of the artificial protein, which is not the case if the Csy4 and Cas9 proteins are used as separate proteins. The inventors assume that the very high local concentration of mature Csy4/Cas9-sgRNA close to the Cas9 protein of the artificial protein results in the much more efficient formation of the functional RNP complex that is observed when using the artificial protein or the nucleic acid encoding the artificial protein for providing the functional RNP complex.

In a preferred embodiment, the Csy4 cleavage sequence of the Csy4/Cas9-sgRNA is located directly downstream of the classical Cas9-sgRNA sequence. In other words, the 3' end of the classical Cas9-sgRNA sequence is directly adjacent to the 5' end of the Csy4 cleavage sequence.

In a preferred embodiment, the Csy4 cleavage sequence comprises the sequence
GUUCACUGCCGUAUAGGCAG (SEQ ID NO.: 11),
GUUCACUGCCGUAUAGGCAGCUAAGAAA (SEQ ID NO.: 12),
GUACACUGCCGUAUAGGCAG (SEQ ID NO.: 13),
GUAAACUGCCGUAUAGGCAG (SEQ ID NO.: 14), or
GUUCCCUGCCGUAUAGGCAG (SEQ ID NO.: 15).

In a further preferred embodiment, the Csy4 cleavage sequence consists of the sequence of SEQ ID NO.: 11, 12, 13, 14 or 15.

In another embodiment, the Cas9-sgRNA consists of a classical Cas9-sgRNA sequence. In other words, the Cas9-sgRNA is a classical Cas9-sgRNA sequence that is devoid of any additional elements. The inventors found that when using separate virus particles for introducing the artificial protein and the Cas9-sgRNA into a target cell, the artificial protein is able to form a functional RNP complex with a Cas9-sgRNA that consists of a classical Cas9-sgRNA sequence in the target cell.

In a preferred embodiment, the artificial protein is associated with a pre-Csy4/Cas9-sgRNA, wherein the pre-Csy4/Cas9-sgRNA comprises a classical Cas9-sgRNA sequence, a Csy4 cleavage sequence located downstream of the classical Cas9-sgRNA sequence, and an additional Csy4 cleavage sequence located directly upstream of the classical Cas9-sgRNA sequence.

In this case, a precursor of a functional RNP complex is present in which two copies of the artificial protein are associated with the pre-Csy4/Cas9-sgRNA. The Csy4 protein of one of the two copies of the artificial protein is associated with the additional Csy4 cleavage sequence located directly upstream of the classical Cas9-sgRNA sequence, while the Csy4 protein of the other one of the two copies of the artificial protein is associated with the Csy4 cleavage sequence located downstream of the classical Cas9-sgRNA sequence. The classical Cas9-sgRNA sequence of the pre-Csy4/Cas9-sgRNA is not associated with any of the Cas9 proteins of the two artificial proteins. The pre-Csy4/Cas9-sgRNA is processed by the Csy4 proteins of the two copies of the artificial protein by cleavage at the 3' end of the Csy4 cleavage sequence. As a consequence, (i) the upstream Csy4 cleavage sequence is separated from the classical Cas9-sgRNA sequence and its downstream Csy4 cleavage sequence, and (ii) additional nucleotides that may be present downstream of the 3' end of the downstream Csy4 cleavage sequence are removed. This processing step leads to the mature Csy4/Cas9-sgRNA. The Cas9 protein of the artificial protein can now associate with the classical Cas9-sgRNA sequence of the Csy4/Cas9-sgRNA. Depending on the design of the precursor of the functional RNP complex, in particular depending on the type of Cas9 protein used, the precursor of the functional RNP complex may be used, for example, for altering a target nucleic acid, activating the expression of a target nucleic acid or repressing the expression of a target nucleic acid. The target nucleic acid preferably is a target gene or a part of a target gene.

It cannot be completely excluded that the Cas9 protein of the artificial protein may also be able to associate with the classical Cas9-sgRNA sequence of the pre-Csy4/Cas9-sgRNA. However, the experimental data of the inventors indicate that in that case, the resulting RNP complex would not be capable of exerting the activity (for example, inducing nicks or DSB in target DNA), that is required for its intended use, i.e. the resulting RNP complex would not be a functional RNP complex. Though the resulting RNP complex may still be functional and capable of exerting other activities (for example transcription activation or repression) that are largely only dependent on target DNA binding.

The chimeric design of the pre-Csy4/Cas9-sgRNA allows an RNA polymerase II driven expression of the pre-Csy4/Cas9-sgRNA from a DNA molecule encoding the pre-Csy4/Cas9-sgRNA. The RNA polymerase II driven expression results in a pre-Csy4/Cas9-sgRNA that comprises a cap structure located at its 5' end and a poly(A) tail located at its 3' end.

In a preferred embodiment, the Csy4 cleavage sequence of the pre-Csy4/Cas9-sgRNA is located directly downstream of the classical Cas9-sgRNA sequence. In other words, the 3' end of the classical Cas9-sgRNA sequence is directly adjacent to the 5' end of the Csy4 cleavage sequence.

In a preferred embodiment, the pre-Csy4/Cas9-sgRNA further comprises a cap structure located at its 5' end. The cap structure generally comprises a guanine nucleotide connected to the RNA via an unusual 5' to 5' triphosphate linkage. This guanosine can be methylated on the 7 position directly after capping by a methyltransferase. It is referred to as a 7-methylguanylate cap, abbreviated m7G. The cap structure may comprise further modifications, for example methylation of the 2'-hydroxy group of the first or the first two ribose sugars of the 5' end of the RNA. The cap structure may alternatively be a 5'-trimethylguanosine cap or a 5'-monomethylphosphate cap. The cap structure provides resistance to 5' exonucleases and thus improves the stability of the pre-Csy4/Cas9-sgRNA.

In a preferred embodiment, the pre-Csy4/Cas9-sgRNA further comprises a polyadenosine tail located at its 3' end. The polyadenosine (poly(A)) tail consists of multiple adenosine monophosphates and is important for the stability of the pre-Csy4/Cas9-sgRNA.

A pre-Csy4/Cas9-sgRNA comprising a cap structure located at its 5' end and/or a poly(A) tail located at its 3' end can be achieved, for example, by providing the nucleic acid encoding the pre-Csy4/Cas9-sgRNA as part of an RNA polymerase II driven eukaryotic expression construct, which results in the transcription of a 5'-capped and 3'-polyadenylated pre-Csy4/Cas9-sgRNA.

In a further preferred embodiment, the pre-Csy4/Cas9-sgRNA further comprises an optimized Woodchuck hepatitis virus posttranscriptional regulatory sequence element (WPRO) located downstream of the Csy4 cleavage sequence that is located downstream of the classical Cas9-sgRNA sequence. The WPRO enhances the expression of a DNA molecule encoding the pre-Csy4/Cas9-sgRNA, in particular in case the DNA molecule is a portion of an expression construct that is a retroviral expression vector.

In a preferred embodiment, the pre-Csy4/Cas9-sgRNA further comprises at least one additional classical Cas9-sgRNA sequence arranged downstream of the Csy4 cleavage sequence that is located downstream of the classical Cas9-sgRNA sequence, wherein each additional classical Cas9-sgRNA sequence is accompanied by an additional Csy4 cleavage sequence located downstream of the additional classical Cas9-sgRNA sequence. Such a pre-Csy4/Cas9-sgRNA is also referred to as multiplex pre-Csy4/Cas9-sgRNA. Multiple additional elements, each comprising an additional classical Cas9-sgRNA sequence and an accompanying additional downstream Csy4 cleavage sequence, can be present. The Csy4 cleavage sequences present in the multiplex pre-Csy4/Cas9-sgRNA can have the same sequence or different sequences. The same applies to the classical Cas9-sgRNA sequences present in the multiplex pre-Csy4/Cas9-sgRNA, however, in most cases, different classical Cas9-sgRNA sequences will be used in order to target different target nucleic acids (multiplexing). This allows multiplex applications which enhance the scope and efficiency of genetic editing and/or gene expression regulation using the CRISPR system. During processing, the multiplex pre-Csy4/Cas9-sgRNA is cleaved at the 3' end of each Csy4 cleavage sequence by the Csy4 protein of the artificial protein, giving rise to separate mature Csy4/Cas9-sgRNAs.

In a preferred embodiment, the nucleic acid encoding the artificial protein is a DNA molecule, wherein the DNA molecule is operably linked to a promoter sequence. The promoter sequence drives the transcription of the DNA molecule encoding the artificial protein. The promoter sequence can be any promoter sequence that is active in a cell or in a cell-free system in which the DNA molecule encoding the artificial protein is to be transcribed. The promoter sequence can be an endogenous promoter sequence or an exogenous promoter sequence. The promoter sequence can be a natural promoter sequence, such as a natural viral promoter sequence, or an engineered (synthetic) promoter sequence. The promoter sequence may be, for example, a cytomegalovirus (CMV) promoter sequence (also referred to as CMV enhancer - promoter sequence). The CMV promoter sequence is a very strong promoter sequence and is thus a preferred promoter sequence. As another example, the promoter sequence may be a Rous sarcoma virus LTR U3 promoter sequence (RSV promoter sequence), a ubiquitin conjugating enzyme E2 K promoter sequence (UBE2K or UBC1 promoter sequence), a CAG promoter sequence, or a simian virus 40 (SV40) late promoter sequence.

The DNA molecule is operably linked to the promoter sequence. This means that the DNA molecule and the promoter sequence are arranged in a manner that allows a functional relationship of the DNA molecule with the promoter sequence, so that the promoter sequence can drive the transcription of the DNA molecule. For example, as is known in the art, the promoter sequence needs to be arranged in an appropriate orientation relative to the DNA molecule. The promoter sequence is preferably located upstream of the DNA molecule.

The advantage of using the DNA molecule compared to an (m)RNA molecule encoding the artificial protein is that the DNA molecule has a greater stability and can be easily provided as part of an expression construct such as a plasmid for introduction into a target cell.

The DNA molecule encoding the artificial protein comprises coding sequences for the Csy4 protein, the Cas9 protein and the linker fused in frame. It is preferred that at least one of the coding sequence for the Csy4 protein (Csy4 coding sequence) and the coding sequence for the Cas9 protein (Cas9 coding sequence), preferably both, are optimized for expression in a human cell. The coding sequence may be, for example, a humanized coding sequence or a coding sequence that is expression-optimized using a suitable algorithm. Humanized coding sequences are based on human codon usage and are thus preferred for use in human cells. Coding sequences that are expression-optimized using a suitable algorithm can be obtained, for example, from commercial providers. They are generally based on an algorithm that utilizes a multifactorial approach to significantly improve protein expression, for example in human cells. The advantage of using humanized coding sequences or coding sequences that are expression-optimized using a suitable algorithm is that a higher expression of the artificial protein, in particular in human cells, can be obtained.

The Csy4 coding sequence preferably is a Csy4 coding sequence that is optimized for expression in a human cell, more preferred a humanized Csy4 coding sequence or a Csy4 coding sequence that is expression-optimized using a suitable algorithm. Likewise, the Cas9 coding sequence preferably is a Cas9 coding sequence that is optimized for expression in a human cell, more preferred a humanized Cas9 coding sequence or a Cas9 coding sequence that is expression-optimized using a suitable algorithm.

In a preferred embodiment, the Csy4 coding sequence is a humanized Csy4 coding sequence and the Cas9 coding sequence is a Cas9 coding sequence that is expression-optimized using a suitable algorithm. In another preferred embodiment, both the Csy4 coding sequence and the Cas9 coding sequence are coding sequences that are expression-optimized using a suitable algorithm.

In case the functional RNP complex is provided in a target cell, the DNA molecule encoding the artificial protein further comprises a coding sequence for the at least one NLS. In this case, the DNA molecule encoding the artificial protein comprises coding sequences for the Csy4 protein, the Cas9 protein, the linker and the at least one NLS fused in frame.

The Csy4 coding sequence and/or the Cas9 coding sequence may also be optimized for expression in a cell that is not a human cell, for example for expression in a bacterial cell (e.g. an E. *coli* cell), a yeast cell or an insect cell. Such types of expression optimization can be achieved, for example, by using a suitable algorithm and may be desirable, for example, for recombinant expression of the artificial protein in the respective cell type.

In a further preferred embodiment, the DNA molecule and the promoter sequence are portions of an expression construct. The expression construct is also referred to as expression vector and is designed for expressing the DNA molecule in a cell. The expression construct preferably is a plasmid or a viral expression vector (also referred to as viral vector or transfer vector), both of which can be easily introduced into a cell. The expression construct preferably is an RNA polymerase II driven eukaryotic expression construct that results in the transcription of a 5'-capped and 3'-polyadenylated mRNA, which is then translated into the artificial protein.

In a further preferred embodiment, the expression construct further comprises an optimized Woodchuck hepatitis virus posttranscriptional regulatory sequence element (WPRO) arranged downstream of the DNA molecule and/or a polyadenylation signal sequence arranged downstream of the DNA molecule. The WPRO enhances the expression of the DNA molecule, in particular in case the expression construct is a retroviral expression vector. The polyadenylation signal sequence leads to an addition of a polyadenosine (poly(A)) tail to the mRNA transcript transcribed from the DNA molecule by RNA polymerase II. The poly(A) tail consists of multiple adenosine monophosphates and is important for nuclear export, translation and stability of the mRNA transcript. In case the expression construct comprises both the WPRO and the polyadenylation signal sequence, the polyadenylation signal sequence is preferably arranged downstream of the WPRO.

In another preferred embodiment, the nucleic acid encoding the artificial protein is an RNA molecule. The RNA molecule may be a chemically synthesized and modified RNA molecule. In a further preferred embodiment, the nucleic acid encoding the artificial protein is an mRNA molecule. The mRNA molecule has been transcribed from a DNA molecule encoding the artificial protein by, for example, transcription in a cell or *in vitro* (cell-free) transcription. The DNA molecule encoding the artificial protein has been described above. Accordingly, the mRNA molecule comprises coding sequences for the Csy4 protein, the Cas9 protein and the linker fused in frame. The coding sequence for the Csy4 protein (Csy4 coding sequence) preferably is a Csy4 coding sequence that is optimized for expression in a human cell, more preferred a humanized Csy4 coding sequence or a Csy4 coding sequence that is expression-optimized using a suitable algorithm. Likewise, the coding sequence for the Cas9 protein (Cas9 coding sequence) preferably is a Cas9 coding sequence that is optimized for expression in a human cell, more preferred a humanized Cas9 coding sequence or a Cas9 coding sequence that is expression-optimized using a suitable algorithm. In case the mRNA molecule results from RNA polymerase II driven transcription in a eukaryotic cell, the mRNA molecule comprises a cap structure located at its 5' end and a polyadenosine tail located at its 3' end. The cap structure and the polyadenosine tail have been described above.

In case the functional RNP complex is provided in a target cell, the mRNA molecule encoding the artificial protein further comprises a coding sequence for the at least one NLS. In this case, the mRNA molecule encoding the artificial protein comprises coding sequences for the Csy4 protein, the Cas9 protein, the linker and the at least one NLS fused in frame.

The advantage of using the (m)RNA molecule compared to a DNA molecule encoding the artificial protein is that the step of transcription of the DNA molecule is not necessary and that RNA viruses or retroviruses can be used for introduction of the nucleic acid encoding the artificial protein into a target cell. Another advantage of using the (m)RNA molecule compared to a DNA molecule encoding the artificial protein is that in case the functional RNP complex is provided in an eukaryotic target cell, no recombination of a DNA molecule introduced into the target cell and the DNA genome of the target cell can occur.

The functional RNP complex can be provided in a target cell. The target cell may be any cell in which the RNP complex is capable of site-directed binding to a target nucleic acid. The target cell preferably is a eukaryotic cell, such as, for example, a plant cell, a fungal cell or an animal cell. In a preferred embodiment, the target cell is a mammalian cell. The mammalian cell may be a human cell or a non-human cell. Many mammalian human and non-human cells and cell lines in which the RNP complex can bind to a target nucleic acid and then exert an intended activity such as altering the target nucleic acid or activating or repressing the expression of the target nucleic acid are known. Among these cells, cancer cells and immune cells are of particular importance. Accordingly, blood cells like T-lymphocytes, B-lymphocytes, monocytes, macrophages or natural killer cells are important mammalian target cells. Further examples of important mammalian target cells are stem cells such as hematopoietic stem cells, neuronal stem cells or induced pluripotent stem cells. In a preferred embodiment, the mammalian cell is a human cell.

In a preferred embodiment, the functional RNP complex binds to a target nucleic acid. The binding of the functional RNP complex to the target nucleic acid occurs in a site-specific manner. Depending on the intended use and thus the design of the functional RNP complex, the binding to the target nucleic acid may lead, for example, to altering the target nucleic acid or to activating or repressing expression of the target nucleic acid.

In a preferred embodiment, the functional RNP complex alters the target nucleic acid. The alteration of the target nucleic acid can be, for example, an insertion, a deletion, a modification or a replacement in the target nucleic acid. The target nucleic acid is altered by the functional RNP complex in a site-specific manner.

The target nucleic acid may be provided in a cell-free solution or may be present in the target cell. The target nucleic acid preferably is a target gene or a part of a target gene. In general, the target nucleic acid is DNA.

In a second aspect, the invention relates to an *in vitro* method for providing a functional ribonucleoprotein (RNP) complex in a target cell, the method comprising the step of introducing a component (a) and a component (b) into the target cell, wherein the component (a) is an artificial protein or a nucleic acid encoding the artificial protein, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide;
and wherein the component (b) is
either
   (b-1) a Csy4/Cas9-single guide RNA (Csy4/Cas9-sgRNA), wherein the Csy4/Cas9-sgRNA comprises
      a classical Cas9-sgRNA sequence, and
      a Csy4 cleavage sequence located downstream of the classical Cas9-sgRNA sequence,
         or
   (b-2) a pre-Csy4/Cas9-sgRNA or a nucleic acid encoding the pre-Csy4/Cas9-sgRNA, wherein the pre-Csy4/Cas9-sgRNA comprises
      a classical Cas9-sgRNA sequence,
      a Csy4 cleavage sequence located downstream of the classical Cas9-sgRNA sequence, and
      an additional Csy4 cleavage sequence located directly upstream of the classical Cas9-sgRNA sequence.

The term *"in vitro"* as used herein in relation to the method for providing a functional RNP complex in a target cell is to be understood as "outside a living organism".

In the method, the two components that are intended to provide the functional RNP complex in the target cell, i.e. the component (a) and the component (b), are introduced into the target cell. The first component of the functional RNP complex is the artificial protein. The artificial protein can be introduced into the target cell as such or via introducing the nucleic acid encoding the artificial protein into the target cell. The artificial protein may be provided, for example, as a recombinant protein. The nucleic acid encoding the artificial protein may be a DNA molecule or an RNA molecule. In case the nucleic acid encoding the artificial protein is a DNA molecule, the DNA molecule is operably linked to a promoter sequence. The DNA molecule encoding the artificial protein and the promoter sequence may be provided, for example, as part of an RNA polymerase II driven eukaryotic expression construct, resulting in the transcription of a 5'-capped and 3'-polyadenylated mRNA, which is then translated into the artificial protein. In case the nucleic acid encoding the artificial protein is an RNA molecule, the RNA molecule preferably is an mRNA molecule, such as an *in vitro* (cell-free) transcribed mRNA molecule (transcribed from a DNA molecule encoding the artificial protein). Alternatively, the RNA molecule may be a chemically synthesized and modified RNA molecule.

In the method of the invention, the second component of the functional RNP complex is the Csy4/Cas9-sgRNA. As mentioned above, the term "Csy4/Cas9-sgRNA" as used herein refers to the mature Csy4/Cas9-sgRNA. The Csy4/Cas9-sgRNA can be introduced into the target cell as such or via introducing the pre-Csy4/Cas9-sgRNA or the nucleic acid encoding the pre-Csy4/Cas9-sgRNA into the target cell. To provide the Csy4/Cas9-sgRNA or the pre-Csy4/Cas9-sgRNA, the respective sgRNA may be produced, for example, by *in vitro* (cell-free) transcription using, for example, T7 RNA polymerase. Alternatively, the respective sgRNA may be provided, for example, as a chemically synthesized and modified RNA. The nucleic acid encoding the pre-Csy4/Cas9-sgRNA may be a DNA molecule, wherein the DNA molecule is operably linked to a promoter sequence. The DNA molecule encoding the pre-Csy4/Cas9-sgRNA and the promoter sequence may be provided, for example, as part of an RNA polymerase III driven eukaryotic expression construct, resulting in the transcription of the pre-Csy4/Cas9-sgRNA. RNA transcripts of RNA polymerase III are not 5'-capped and are not 3'-polyadenylated. Alternatively, the DNA molecule encoding the pre-Csy4/Cas9-sgRNA and the promoter sequence may be provided, for example, as part of an RNA polymerase II driven eukaryotic expression construct, resulting in the transcription of a 5'-capped and 3'-polyadenylated pre-Csy4/Cas9-sgRNA. The latter option is facilitated by the chimeric design of the pre-Csy4/Cas9-sgRNA.

The classical Cas9-sgRNA sequence is also referred to as first classical Cas9-sgRNA sequence.

The pre-Csy4/Cas9-sgRNA is processed by the Csy4 protein of the artificial protein into the mature Csy4/Cas9-sgRNA through recognition and binding of the Csy4 cleavage sequence and subsequent cleavage at the 3' end of each Csy4 cleavage sequence by the ribonuclease activity of the Csy4 protein.

In case the mature Csy4/Cas9-sgRNA is provided by other means, the Csy4 protein of the artificial protein recognizes and binds the mature Csy4/Cas9-sgRNA through recognition and binding of the Csy4 cleavage sequence.

The Csy4 protein has the ability to stay bound with high affinity to the Csy4 cleavage sequence, even after the cleavage at the 3' end of each Csy4 cleavage sequence in case a pre-Csy4/Cas9-sgRNA has been used.

The mature Csy4/Cas9-sgRNA can be bound by the Cas9 protein of the artificial protein through recognition and binding of the classical Cas9-sgRNA sequence of the Csy4/Cas9-sgRNA. This leads to the formation of the functional RNP complex.

The inventors surprisingly found that the method for providing a functional RNP complex in the target cell results in a much more efficient formation of the functional RNP complex compared to using the Csy4 and Cas9 proteins as separate proteins. In other words, the inventors found that the method results in a much more efficient assembly of the Cas9 protein with the classical Cas9-sgRNA sequence of the Csy4/Cas9-sgRNA, compared to using the Csy4 and Cas9 proteins as separate proteins. The more efficient formation of the functional RNP complex facilitates a more efficient site-directed binding of the functional RNP complex to the target nucleic acid and thus a more efficient activity of the functional RNP complex, for example a more efficient editing of the target nucleic acid. The more efficient activity is particular advantageous at conditions of low overall concentrations of the two components of the functional RNP complex.

In a preferred embodiment, the functional RNP complex binds to a target nucleic acid. As described above, the binding to the target nucleic acid may lead, for example, to altering the target nucleic acid or to activating or repressing expression of the target nucleic acid. The target nucleic acid preferably is a target gene or a part of a target gene. In general, the target nucleic acid is DNA.

In a preferred embodiment, the functional RNP complex alters the target nucleic acid. In this case, the method for providing the functional RNP complex in the target cell presents a method for altering a target nucleic acid in the target cell.

In another embodiment, the functional RNP complex activates the expression of the target nucleic acid. In this case, the method for providing the functional RNP complex in the target cell presents a method for activating the expression of a target nucleic acid in the target cell.

In another embodiment, the functional RNP complex represses the expression of the target nucleic acid. In this case, the method for providing the functional RNP complex in the target cell presents a method for repressing the expression of a target nucleic acid in the target cell.

The features and embodiments, in particular the preferred embodiments, described above with respect to the use of the artificial protein or of the nucleic acid encoding the artificial protein for providing a functional RNP complex, also apply to the method for providing a functional RNP complex. This particularly concerns features and embodiments described above regarding the artificial protein, the nucleic acid encoding the artificial protein, the Csy4/Cas9-sgRNA, the pre-Csy4/Cas9-sgRNA, the nucleic acid encoding the pre-Csy4/Cas9-sgRNA, and the target cell.

Examples of pre-Csy4/Cas9-sgRNAs are the pre-Csy4/Cas9-sgRNAs of SEQ ID NO.: 74, 76, 78 and 82.

Examples of nucleic acids encoding pre-Csy4/Cas9-sgRNAs, namely of DNA molecules encoding pre-Csy4/Cas9-sgRNAs, are the DNA molecules of SEQ ID NO.: 75, 77, 79 and 83. The DNA molecules of SEQ ID NO.: 75, 77, 79 and 83 encode the pre-Csy4/Cas9-sgRNAs of SEQ ID NO.: 74, 76, 78 and 82, respectively (Table 3D).

The component (a) and the component (b) can be introduced (delivered) into the target cell by various means as described below. The component (a) and the component (b) can be separately introduced into the target cell or the component (a) and the component (b) can be introduced into the target cell together. In the latter case, the two components may be pre-assembled to the functional RNP complex or to a precursor of the functional RNP complex prior to introduction into the target cell. In the precursor of the functional RNP complex, the Csy4 protein of the artificial protein is associated with the Csy4 cleavage sequence of the pre-Csy4/Cas9-sgRNA while the Cas9 protein of the artificial protein is not yet associated with the classical Cas9-sgRNA sequence.

As mentioned above, it cannot be completely excluded that the Cas9 protein of the artificial protein may also be able to associate with the classical Cas9-sgRNA sequence of the pre-Csy4/Cas9-sgRNA. However, the experimental data of the inventors indicate that in that case, the resulting RNP complex would not be capable of exerting the activity that is required for its intended use, i.e. the resulting RNP complex would not be a functional RNP complex.

Various means for introducing a protein and/or a nucleic acid into a target cell are known. The artificial protein may be introduced into the target cell, for example, by injection, electroporation, non-viral protein transduction or viral transduction of the target cell. In case the artificial protein is to be introduced into the target cell by non-viral protein transduction of the artificial protein through the plasma membrane of the target cell, the artificial protein may be fused to a protein transduction domain such as a Tat protein transduction domain of the HIV-1 Tat protein. For introducing a nucleic acid into the target cell, i.e. for introducing any of (i) the nucleic acid encoding the artificial protein, (ii) the Csy4/Cas9-sgRNA, (iii) the pre-Csy4/Cas9-sgRNA or (iv) the nucleic acid encoding the pre-Csy4/Cas9-sgRNA, the target cell may be subject to transfection or transduction.

The term "transfection of the target cell" as used herein refers to the process of introducing one or more foreign nucleic acids into the target cell using non-viral physical or chemical methods. Several transfection techniques are known, for example electroporation, cationic liposome-mediated transfection, calcium phosphate coprecipitation, or methods using cationic polymers such as DEAE-dextran or poly-ethylenimine.

In contrast, the term "transduction of the target cell" as used herein refers to the process of transferring one or more nucleic acids or other cargo molecules such as heterologous viral proteins, non-viral proteins, nucleoprotein complexes or ribonucleoprotein complexes, into the target cell using a DNA virus, an RNA virus or a retrovirus. The virus is typically a recombinant virus that has been produced by a suitable packaging cell. The DNA virus may be, for example, an adenovirus or an adeno-associated virus. The RNA virus may be, for example, a vesicular stomatitis virus or a sendai virus. The retrovirus may be, for example, a foamy virus, a lentivirus or another retrovirus.

The form in which the component (a) and the component (b) are introduced into the target cell primarily depends on the means chosen for their introduction into the target cell. For example, in case a DNA virus is used for introducing the component (a) into the target cell, then for (a), the nucleic acid encoding the artificial protein is selected, wherein the nucleic acid encoding the artificial protein is a DNA molecule. Likewise, in case a DNA virus is used for introducing the component (b) into the target cell, then for (b), the nucleic acid encoding the pre-Csy4/Cas9-sgRNA is selected, wherein the nucleic acid encoding the pre-Csy4/Cas9-sgRNA is a DNA molecule.

In a preferred embodiment, the component (a) and the component (b) are introduced into the target cell by transduction of the target cell using at least one recombinant virus.

In a preferred embodiment, the component (a) and the component (b) are separately introduced into the target cell by using a first virus and a second virus for the transduction of the target cell. The first virus is used for introducing the component (a) into the target cell and the second virus is used for introducing the component (b) into the target cell. The component (a) and the component (b) are thus transferred into the target cell in separate viral vector particles. This "split-virus design" results in the independent introduction of the component (a) and the component (b) into the target cell. The first virus and the second virus are typically co-transduced into the target cell. Alternatively, the target cell may be consecutively transduced with the first virus and the second virus, in particular first with the first virus and second with the second virus. The combined use of the first virus and the second virus is also referred to as "Two-Virus" vector system.

In a further preferred embodiment, the first virus is a first retrovirus and the second virus is a second retrovirus. The first retrovirus is used for introducing the component (a) into the target cell and the second retrovirus is used for introducing the component (b) into the target cell. Retroviruses are particularly suitable for introducing nucleic acids or other cargo molecules into the target cell. The first and the second retrovirus preferably are based on the same type of retrovirus (i.e. differing in their cargo only). This facilitates their co-transduction into the target cell.

In a further preferred embodiment, the first retrovirus is an integrase-deficient retrovirus and/or the second retrovirus is an integrase-deficient retrovirus. The advantages of using an integrase-deficient retrovirus will be explained with respect to an integrase-deficient prototype foamy virus further below.

In a further preferred embodiment, the first retrovirus is a reverse transcriptase-deficient retrovirus and/or the second retrovirus is a reverse transcriptase-deficient retrovirus. The advantages of using a reverse transcriptase-deficient retrovirus will be explained with respect to an integrase-deficient prototype foamy virus further below.

In a further preferred embodiment, the first retrovirus is a foamy virus and the second retrovirus is a foamy virus. Foamy virus is also referred to as spumavirus. Spuma- or foamy viruses (FV) are a special type of retroviruses that have been grouped into several genera of a separate retrovirus subfamily, the spumaretrovirinae, as a consequence of unique features in their replication strategy. Foamy virus is a particularly suitable virus for introducing the two components into the target cell since it has an extremely broad tropism, i.e. it can infect almost every eukaryotic cell type, and since it has the capacity to package large mRNA amounts both specific and unspecific (cargo nucleic acids). In addition to the efficient packaging (encapsidation) of non-viral RNAs as cargo nucleic acids, foamy virus has a further unique capability, namely the efficient transfer of the non-viral RNAs. The efficient transfer refers to the release of the cargo nucleic acids in the target cell. The release of the cargo nucleic acids is important for the intracellular processes following the transduction of the target cell, for example for the translation of released mRNA into the respective protein. As an additional advantage, foamy virus is apparently apathogenic in natural hosts as well as in zoonotically infected humans.

The foamy virus may be, for example, of the genus of *Simiispumaviruses* of Ape origin such as the prototype foamy virus (PFV, SFVpsc_huHSRV.13) isolate, or of Old World monkey origin such as the macaque simian foamy virus (macaque SFV, SFVmcy_FV21) isolate, or of New World monkey origin such as the spider monkey simian foamy virus (SFVspm, SFVaxx_Hooks40) isolate. The foamy virus may be, for example, prototype foamy virus (PFV), Bornean orangutan simian foamy virus, Taiwanese macaque simian foamy virus, Grivet simian foamy virus, Spider monkey simian foamy virus, Squirrel monkey simian foamy virus, or White-tufted-ear marmoset simian foamy virus.

The foamy virus preferably is prototype foamy virus (PFV). A major advantage of using PFV is that it can facilitate a substantially transient genetic modification of the target cell.

The term "substantially transient genetic modification" as used herein refers to the predominantly transient presence of the cargo nucleic acids in the target cell. In other words, the components delivered by the virus are predominantly present within a narrow time frame. The substantially transient genetic modification of the target cell requires the use of an integrase-deficient PFV vector, for example of a PFV vector that has an integrase-deficient PFV Pol protein. The mechanism underlying the substantially transient genetic modification of the target cell is as follows: Upon packaging of the PFV vector RNA genome (vgRNA), it will be reverse transcribed by the co-encapsidated integrase-deficient PFV Pol protein into double-stranded vector DNA (vgDNA). Due to the enzymatically inactive integrase of the PFV Pol protein employed to deliver vgDNA into the target cell, the vgDNA cannot be actively inserted into target cell chromosomes. The vgDNA is largely present transiently in episomal form in the nucleus of the transduced target cell. This results in a predominantly transient nuclear expression of the one or more cargo nucleic acids transferred by the PFV. Accordingly, the PFV preferably is an integrase-deficient PFV. This leads to a substantially transient genetic modification of the target cell. The substantially transient genetic modification of the target cell is highly desirable in order to minimize any accumulation of unintended off-target effects. This increases the specificity of the intended effect on the target nucleic acid in the target cell. Accordingly, in a preferred embodiment, the first retrovirus is an integrase-deficient PFV and the second retrovirus is an integrase-deficient PFV. This allows a substantially transient presence of both component (a) and component (b) in the target cell. In another embodiment, the first retrovirus or the second retrovirus is an integrase-deficient PFV. This allows a substantially transient presence of one of the two components (a) and (b) in the target cell.

The substantially transient genetic modification of the target cell that is achieved by using an integrase-deficient PFV is substantially transient, but not completely transient. The reason for this is that, at low frequency, a long-term expression of the one or more nucleic acids transferred by the PFV is possible due to non-viral mediated chromosome integration of episomal vgDNA as a consequence of non-homologous recombination events. Therefore, the genetic modification of the target cell is not completely transient and poses a residual risk for unintended permanent genetic modifications of the target cell.

The substantially transient genetic modification of the target cell can also be achieved by using a different integrase-deficient retrovirus. Accordingly, as mentioned above, it is preferred that the first retrovirus is an integrase-deficient retrovirus and/or the second retrovirus is an integrase-deficient retrovirus. The integrase-deficient retrovirus preferably is a foamy virus, more preferred a prototype foamy virus.

Another major advantage of using PFV is that it can facilitate a completely transient genetic modification of the target cell. The term "completely transient genetic modification" as used herein refers to the completely transient presence of the cargo nucleic acids in the target cell. It is also referred to as exclusively transient genetic modification, fully transient genetic modification or purely transient genetic modification. Non-viral mediated chromosome integration of the cargo nucleic acids does not occur. Accordingly, the components delivered by the virus are present within a narrow time frame. The completely transient genetic modification of the target cell requires the use of a reverse transcriptase-deficient PFV. The reverse transcriptase-deficient PFV may have a mutated reverse transcriptase that is inactive or may completely lack the Pol protein. The RNA transferred by the reverse transcriptase-deficient PFV can be translated in the cytoplasm of the target cell and is degraded over time. Due to the deficient reverse transcriptase, no recombination of nucleic acids transferred by the PFV with the target cell genome is possible. Therefore, a genetic modification of the target cell cannot occur.

The completely transient genetic modification of the target cell prevents any unintended off-target effects since there is no residual risk for unintended permanent genetic modifications of the target cell. This further increases the specificity of the intended effect on the target nucleic acid in the target cell. Accordingly, in a preferred embodiment, the first retrovirus is a reverse transcriptase-deficient PFV and the second retrovirus is a reverse transcriptase-deficient PFV. This allows a completely transient presence of both component (a) and component (b) in the target cell. In another embodiment, the first retrovirus or the second retrovirus is a reverse transcriptase-deficient. This allows a completely transient presence of one of the two components (a) and (b) in the target cell.

The completely transient genetic modification of the target cell can also be achieved by using a different reverse transcriptase-deficient retrovirus. Accordingly, as mentioned above, it is preferred that the first retrovirus is a reverse transcriptase-deficient retrovirus and/or the second retrovirus is a reverse transcriptase-deficient retrovirus. The reverse transcriptase-deficient retrovirus preferably is a foamy virus, more preferred a prototype foamy virus.

It is preferred that the reverse transcriptase-deficient PFV is based on a minimal FV vector system. Suitable minimal FV vector systems are, for example, described in WO 2012/152632 A1. The minimal FV vector system comprises at least one viral packaging vector and a minimal transfer vector, wherein the minimal transfer vector comprises a non-viral cargo nucleic acid and does not comprise any sequences of the FV. The minimal transfer vector is also referred to as non-viral transfer vector since it may be devoid or substantially devoid of any viral sequences. Since the minimal transfer vector does not have any foamy viral sequences, the packaged RNA that is transferred into the target cell likewise does not have any foamy viral sequences. It can thereby facilitate a completely transient genetic modification of the target cell. The at least one viral packaging vector comprises sequences encoding at least a FV Gag protein and an Env protein, both of which are essential for the production of recombinant PFV particles that are suitable for providing a functional RNP complex in the target cell. The term "FV Gag protein" as used herein refers to a FV Gag protein that has its natural nucleic acid binding activity. The Env protein (envelope protein) may be, for example, a FV Env protein or VSV-G. A DNA sequence encoding a reverse transcriptase-deficient PFV Pol protein is not required, but may be comprised by the at least one viral packaging vector. The minimal FV vector system is based on the previous finding that in an FV vector system, a minimal transfer vector, which does not comprise any nucleotide sequences derived from the FV in question, can be used, and that such a minimal transfer vector can be used for a completely transient expression of a cargo nucleic acid in a target cell. The minimal FV vector system shows an efficient packaging of the non-viral cargo nucleic acid and a functional introduction thereof into the target cell.

Accordingly, in a preferred embodiment, the first retrovirus is a reverse transcriptase-deficient PFV that is based on a minimal PFV vector system and the second retrovirus is a reverse transcriptase-deficient PFV that is based on a minimal PFV vector system. This allows a completely transient presence of both component (a) and component (b) in the target cell. In another embodiment, the first retrovirus or the second retrovirus is a reverse transcriptase-deficient PFV that is based on a minimal PFV vector system. This allows a completely transient presence of one of the two components (a) and (b) in the target cell. The minimal PFV vector system is also referred to as transient PFV RNA transfer vector system or transient PFV vector system (TraFo). The minimal PFV vector system comprises at least one viral packaging vector and a minimal transfer vector, wherein the at least one viral packaging vector comprises sequences encoding at least a FV Gag protein and an Env protein, wherein the minimal transfer vector comprises a nucleic acid encoding the artificial protein or a nucleic acid encoding the pre-Csy4/Cas9-sgRNA, as applicable, and wherein the minimal transfer vector does not comprise any sequences of the PFV. The term "PFV that is based on a minimal PFV vector system" as used herein refers to a PFV that has been produced using the minimal PFV vector system.

A reverse transcriptase-deficient PFV that is based on a minimal PFV vector system has been previously used in connection with a classical CRISPR/Cas9 system, namely for introducing an mRNA encoding the Cas9 protein into a target cell, leading to a completely transient presence of the Cas9 mRNA in the target cell (Lindel et al. 2019). The PFV has been used in combination with an integrase-deficient PFV for introducing a Cas9-sgRNA that consists of a classical Cas9-sgRNA sequence into the target cell, leading to a substantially transient presence of the Cas9-sgRNA in the target cell. It was found that the combination enhances efficacy and specificity of the inactivation of a target gene in the target cell compared to respective lentiviral vector systems.

In another embodiment, the first retrovirus is a reverse transcriptase-deficient PFV that is based on a minimal PFV vector system and the second retrovirus is an integrase-deficient PFV. This allows a completely transient presence of component (a) and a substantially transient presence of component (b) in the target cell. In another embodiment, the first retrovirus is an integrase-deficient PFV and the second retrovirus is a reverse transcriptase-deficient PFV that is based on a minimal PFV vector system. This allows a substantially transient presence of component (a) and a completely transient presence of component (b) in the target cell.

In another embodiment, the first and the second retrovirus is a lentivirus, preferably human immunodeficiency virus (HIV), more preferred HIV type 1 (HIV-1).

In another embodiment, the first and the second retrovirus is a gammaretrovirus, preferably murine leukemia virus (MLV).

In another embodiment, the first retrovirus is a reverse transcriptase-deficient PFV that is based on a minimal PFV vector system and the second retrovirus is a lentivirus or a gamma-retrovirus. In another embodiment, the first retrovirus is a lentivirus or a gamma-retrovirus and the second retrovirus is a reverse transcriptase-deficient PFV that is based on a minimal PFV vector system.

The inventors found that the effect of the "Two-Virus" vector system on a target nucleic acid is influenced in its efficiency by the relative ratio of the first virus and the second virus during their co-transduction into the target cell, in particular in case the first virus introduces an mRNA molecule encoding the artificial protein (component (a)) into the target cell and the second virus introduces the pre-Csy4/Cas9-sgRNA (component (b)) into the target cell. In this case, the highest efficiency was obtained using a relative ratio of the first virus to the second virus of 1:1. Therefore, according to the inventors' findings, the relative ratio of the first virus to the second virus during their co-transduction into the target cell preferably is 0.1:1 to 1:0.1, more preferred 1:1, wherein the first virus introduces an mRNA molecule encoding the artificial protein into the target cell and the second virus introduces the pre-Csy4/Cas9-sgRNA into the target cell. The preferred relative ratios have been established using a pre-Csy4/Cas9-sgRNA that has a single classical Cas9-sgRNA sequence. In case a pre-Csy4/Cas9-sgRNA that comprises two or more classical Cas9-sgRNA sequences is used, a different relative ratio may show the highest efficiency. The best relative ratio of the first virus to the second virus during their co-transduction into the target cell can be determined for each setting by testing different relative ratios as shown in the Examples following the detailed description of the invention.

The inventors further found that the effect of the "Two-Virus" vector system on a target nucleic acid may be influenced in its efficiency by the design of the linker of the artificial protein. In particular, the efficiency of the "Two-Virus" vector system may be further increased by increasing the number of G4S motifs in the linker.

In another preferred embodiment, the component (a) and the component (b) as defined in the method of the invention are introduced into the target cell together by using one virus for the transduction of the target cell. In this case, only one virus is needed. The component (a) and the component (b) are thus transferred into the target cell in the same viral vector particle. The use of one virus is also referred to as "One-Virus" vector system. It provides an all-in-one transfer system. The "One-Virus" vector system offers several advantages, especially in case of target cells that generally show a poor target cell transduction. For example, lymphocytes (primary lymphocytes or tumor cells) show a generally poor transduction efficiency for retroviral vector particles. The inventors found that T-cells of a human tumor cell line can be successfully transduced with the "One-Virus" vector system using a foamy virus.

It is preferred that the virus is a foamy virus. The advantages of using foamy virus have been described above and also apply to "One-Virus" vector system.

The foamy virus may be, for example, of the genus of *Simiispumaviruses* of Ape origin such as the prototype foamy virus (PFV, SFVpsc_huHSRV.13) isolate, or of Old World monkey origin such as the macaque simian foamy virus (macaque SFV, SFVmcy_FV21) isolate, or of New World monkey origin such as the spider monkey simian foamy virus (SFVspm, SFVaxx_Hooks40) isolate. The foamy virus may be, for example, prototype foamy virus (PFV), Bornean orangutan simian foamy virus, Taiwanese macaque simian foamy virus, Grivet simian foamy virus, Spider monkey simian foamy virus, Squirrel monkey simian foamy virus, or White-tufted-ear marmoset simian foamy virus.

The foamy virus preferably is prototype foamy virus (PFV). The PFV preferably is an integrase-deficient PFV or a reverse transcriptase-deficient PFV. The advantages of using PFV and in particular an integrase-deficient PFV or a reverse transcriptase-deficient PFV have been described above and also apply to "One-Virus" vector system.

It is further preferred that the PFV is a reverse transcriptase-deficient PFV that is based on a minimal PFV vector system. For the "One-Virus" vector system, the minimal PFV vector system comprises at least one viral packaging vector, a first minimal transfer vector and a second minimal transfer vector, wherein the at least one viral packaging vector comprises sequences encoding at least a FV Gag protein and an Env protein, wherein the first minimal transfer vector comprises a nucleic acid encoding the artificial protein, wherein the second minimal transfer vector comprises a nucleic acid encoding the pre-Csy4/Cas9-sgRNA, and wherein the first and the second minimal transfer vector do not comprise any sequences of the PFV. As an alternative to the first minimal transfer vector and the second minimal transfer vector, only one minimal transfer vector that comprises a nucleic acid encoding both the artificial protein and the pre-Csy4/Cas9-sgRNA may be used, wherein the minimal transfer vector does not comprise any sequences of the PFV. The advantages of using the reverse transcriptase-deficient PFV that is based on a minimal PFV vector system have been described above and also apply to "One-Virus" vector system.

Importantly, the "One-Virus" vector system established by the inventors that uses a reverse transcriptase-deficient PFV that is based on a minimal PFV vector system is the first vector system with which a completely transient gene editing in human T-cells could be demonstrated.

The inventors found that the effect of the "One-Virus" vector system on a target nucleic acid is influenced in its efficiency by the relative ratio of a first expression construct to a second expression construct during virus production, wherein the first expression construct encodes the artificial protein (component (a)) and the second expression construct encodes the pre-Csy4/Cas9-sgRNA (component (b)). During virus production, the first and the second expression construct are co-transfected into a plurality of packaging cells. The efficiency of the "One-Virus" vector system could be further increased by reducing the relative ratio of the first expression construct to the second expression construct during virus production. The highest efficiency was obtained using a relative ratio of 1:5. This indicates that rather little amounts of the first expression construct relative to the second expression construct are sufficient for obtaining a highly efficient "One-Virus" vector system. This has been a surprising finding. Therefore, according to the inventors' findings, the relative ratio of the first expression construct to the second expression construct during virus production preferably is 1:1 to 1:5, more preferred 1:5, wherein the first expression construct encodes the artificial protein and the second expression construct encodes the pre-Csy4/Cas9-sgRNA. The preferred relative ratios have been established using a pre-Csy4/Cas9-sgRNA that has a single classical Cas9-sgRNA sequence. In case a pre-Csy4/Cas9-sgRNA that comprises two or more classical Cas9-sgRNA sequences is used, a different relative ratio may show the highest efficiency. The best relative ratio of the first expression construct to the second expression construct during virus production can be determined for each setting by testing different relative ratios as shown in the Examples following the detailed description of the invention.

In another preferred embodiment, the relative ratio of the first expression construct to the second expression construct during virus production is 1:1 to 1:11, more preferred 1:10 or 1:11, wherein the first expression construct encodes the artificial protein and the second expression construct encodes the pre-Csy4/Cas9-sgRNA.

The inventors further found that the effect of the "One-Virus" vector system on a target nucleic acid is influenced in its efficiency by the design of the linker of the artificial protein. In particular, the efficiency of the "One-Virus" vector system could be further increased by using a linker that is devoid of any NLS compared to a linker that comprises an NLS.

The inventors also found that the "One-Virus" vector system mainly introduces the component (a) and the component (b) as parts of a functional RNP complex into the target cell. This implies that the artificial protein and the Csy4/Cas9-sgRNA are pre-assembled to the functional RNP complex prior to introduction into the target cell. Accordingly, for (a), the artificial protein is selected, and for (b), the Csy4/Cas9-sgRNA is selected, wherein the artificial protein is associated with the Csy4/Cas9-sgRNA. The assembly of the functional RNP complex takes places during virus production, i.e. in a packaging cell used for virus production. This is due to the surprisingly efficient assembly of the functional RNP complex when using the artificial protein and a Csy4/Cas9-sgRNA or a pre-Csy4/Cas9-sgRNA as has been described with respect to the target cell above. The highly efficient formation of the functional RNP complex also applies to the packaging cell. It facilitates an efficient packaging of the component (a) and the component (b) into "One-Virus" vector particles and, upon transduction of a target cell, a highly efficient site-directed binding of the functional RNP complex to the target nucleic acid in the target cell and thus a more efficient activity of the functional RNP complex, for example a more efficient editing of the target nucleic acid.

The advantage of transferring a functional RNP complex into the target cell is that the activity of the artificial protein is available earlier, since no translation of the artificial protein is required. This may be particularly relevant in cases in which the target cell has reduced or completely inhibited translational capacity. In addition, it is conceivable that directly transferring a functional RNP complex into the target cell leads to a greater number of functional RNP complexes in the target cell compared to the scenario in which the functional RNP complex is first formed in the target cell. A further advantage of transferring a functional RNP complex into the target cell is that a completely transient genetic modification of the target cell is achieved.

In a preferred embodiment, the virus, in particular the retrovirus, comprises a foamy virus envelope glycoprotein (FV Env) or a vesicular stomatitis virus glycoprotein (VSV-G). The glycoprotein can be the natural envelope glycoprotein of the virus. For example, the virus can be PFV and comprise the FV Env of PFV. Alternatively, the virus particle can comprise a viral envelope glycoprotein that is different from its natural envelope proteins (pseudotyped virus particle). For example, the virus can be PFV pseudotyped with FV Env of macaque SFV or pseudotyped with VSV-G. Pseudotyping of virus particles is commonly used to alter the tropism of the virus particles. The possibility of pseudotyping broadens the potential application of the virus particles. The inventors found that pseudotyped virus particles can be used for introducing the component (a) and/or the component (b) into the target cell.

The FV Env may be, for example, of the genus of *Simiispumaviruses* of Ape origin such as the prototype foamy virus (PFV, SFVpsc_huHSRV.13) isolate, or of Old World monkey origin such as the macaque simian foamy virus (macaque SFV, SFVmcy_FV21) isolate, or of New World monkey origin such as the spider monkey simian foamy virus (SFVspm, SFVaxx_Hooks40) isolate. The foamy virus may be, for example, prototype foamy virus (PFV), Bornean orangutan simian foamy virus, Taiwanese macaque simian foamy virus, Grivet simian foamy virus, Spider monkey simian foamy virus, Squirrel monkey simian foamy virus, or White-tufted-ear marmoset simian foamy virus. In a preferred embodiment, the FV Env is the FV Env of PFV or the FV Env of macaque SFV.

In a preferred embodiment, the virus is a foamy virus that comprises a non-foamy virus envelope glycoprotein. This is useful in case the broad tropism mediated by FV Env proteins is not suitable for a specific target cell and thus the FV Env needs to be replaced by alternative viral glycoproteins such as VSV-G or by retargeted glycoproteins with cell-specific receptor use as for example on the basis of measles- or Nipah virus glycoproteins. The non-foamy virus envelope glycoprotein preferably is VSV-G. VSV-G pseudotyped foamy virus, in particular VSV-G pseudotyped PFV, allows, for example, the transduction of T-cells.

In a preferred embodiment, the Csy4 cleavage sequence comprises the sequence
GUUCACUGCCGUAUAGGCAG (SEQ ID NO.: 11),
GUUCACUGCCGUAUAGGCAGCUAAGAAA (SEQ ID NO.: 12),
GUACACUGCCGUAUAGGCAG (SEQ ID NO.: 13),
GUAAACUGCCGUAUAGGCAG (SEQ ID NO.: 14), or
GUUCCCUGCCGUAUAGGCAG (SEQ ID NO.: 15).

In a further preferred embodiment, the Csy4 cleavage sequence consists of the sequence of SEQ ID NO.: 11, 12, 13, 14 or 15.

In a preferred embodiment, the pre-Csy4/Cas9-sgRNA further comprises a cap structure located at its 5' end and/or a polyadenosine tail located at its 3' end. This can be achieved, for example, by providing the nucleic acid encoding the pre-Csy4/Cas9-sgRNA as part of an RNA polymerase II driven eukaryotic expression construct, which results in the transcription of a 5'-capped and 3'-polyadenylated pre-Csy4/Cas9-sgRNA. The cap structure, the polyadenosine tail and their advantages have been described above and also apply to the method for providing a functional RNP complex.

In a preferred embodiment, the pre-Csy4/Cas9-sgRNA further comprises an optimized Woodchuck hepatitis virus posttranscriptional regulatory sequence element (WPRO) located downstream of the Csy4 cleavage sequence. The WPRO enhances the expression of a DNA molecule encoding the pre-Csy4/Cas9-sgRNA, in particular in case the DNA molecule is a portion of an expression construct that is a retroviral expression vector.

In a preferred embodiment, the pre-Csy4/Cas9-sgRNA further comprises at least one additional classical Cas9-sgRNA sequence arranged downstream of the Csy4 cleavage sequence, wherein each additional classical Cas9-sgRNA sequence is accompanied by an additional Csy4 cleavage sequence located downstream of the additional classical Cas9-sgRNA sequence. Such a pre-Csy4/Cas9-sgRNA is also referred to as multiplex pre-Csy4/Cas9-sgRNA. Multiple additional elements, each comprising an additional classical Cas9-sgRNA sequence and an accompanying downstream Csy4 cleavage sequence, can be present. The Csy4 cleavage sequences present in the multiplex pre-Csy4/Cas9-sgRNA can have the same sequence or different sequences. The same applies to the classical Cas9-sgRNA sequences, however, in most cases, different classical Cas9-sgRNA sequences will be used in order to target different target nucleic acids (multiplexing). This allows multiplex applications which enhance the scope and efficiency of genetic editing and/or gene expression regulation using the CRISPR system. During processing, the multiplex pre-Csy4/Cas9-sgRNA is cleaved at the 3' end of each Csy4 cleavage sequence by the Csy4 protein of the artificial protein, giving rise to separate mature Csy4/Cas9-sgRNAs.

In a preferred embodiment, for the component (a), the artificial protein is selected, and for the component (b), the Csy4/Cas9-sgRNA or the pre-Csy4/Cas9-sgRNA is selected, wherein the artificial protein is associated with the Csy4/Cas9-sgRNA or with the pre-Csy4/Cas9-sgRNA. In case the artificial protein is associated with the Csy4/Cas9-sgRNA, the Csy4 protein of the artificial protein is associated with the downstream Csy4 cleavage sequence of the Csy4/Cas9-sgRNA and the Cas9 protein of the artificial protein is associated with the classical Cas9-sgRNA sequence of the Csy4/Cas9-sgRNA. In this case, a functional RNP complex is introduced into the target cell. In case the artificial protein is associated with the pre-Csy4/Cas9-sgRNA, the Csy4 protein of the artificial protein is associated with the downstream Csy4 cleavage sequence of the pre-Csy4/Cas9-sgRNA, while the Cas9 protein of the artificial protein is not associated with the classical Cas9-sgRNA sequence of the pre-Csy4/Cas9-sgRNA. In this case, a precursor of the functional RNP complex is introduced into the target cell. In the target cell, the pre-Csy4/Cas9-sgRNA will be processed by the Csy4 protein of the artificial protein. This leads to the mature Csy4/Cas9-sgRNA and thus to the assembly of the functional RNP complex.

As mentioned above, it cannot be completely excluded that the Cas9 protein of the artificial protein may also be able to associate with the classical Cas9-sgRNA sequence of the pre-Csy4/Cas9-sgRNA. However, the experimental data of the inventors indicate that in that case, the resulting RNP complex would not be capable of exerting the activity that is required for its intended use, i.e. the resulting RNP complex would not be a functional RNP complex.

In another preferred embodiment, for the component (a), the nucleic acid encoding the artificial protein is selected, wherein the nucleic acid encoding the artificial protein is an mRNA molecule, and for the component (b), the Csy4/Cas9-sgRNA or the pre-Csy4/Cas9-sgRNA is selected. In this case, the component (a) and the component (b) are both introduced as RNA molecules into the target cell. In this case, the component (a) and the component (b) may be introduced into the target cell by transduction of the target cell using one or two RNA virus(es) or retrovirus(es). In the target cell, the mRNA encoding the artificial protein will be translated into the artificial protein. The artificial protein will then associate with the Csy4/Cas9-sgRNA or with the pre-Csy4/Cas9-sgRNA, leading to the functional RNP complex as described above.

In a further preferred embodiment, for the component (a), the nucleic acid encoding the artificial protein is selected, wherein the nucleic acid encoding the artificial protein is an mRNA molecule, and for the component (b), the pre-Csy4/Cas9-sgRNA is selected.

In another preferred embodiment, for the component (a), the nucleic acid encoding the artificial protein is selected, wherein the nucleic acid encoding the artificial protein is a DNA molecule, and for the component (b), the nucleic acid encoding the pre-Csy4/Cas9-sgRNA is selected, wherein the nucleic acid encoding the pre-Csy4/Cas9-sgRNA is a DNA molecule. In this case, the component (a) and the component (b) are both introduced as DNA molecules into the target cell. In this case, the component (a) and the component (b) may be introduced into the target cell by transduction of the target cell using one or two DNA virus(es). Each DNA molecule preferably is part of a respective expression construct, for example a viral transfer vector (viral expression vector). In the target cell, the DNA encoding the artificial protein will be transcribed and translated into the artificial protein, while the DNA encoding the pre-Csy4/Cas9-sgRNA will be transcribed into the pre-Csy4/Cas9-sgRNA. The artificial protein will then associate with the pre-Csy4/Cas9-sgRNA, leading to the functional RNP complex as described above.

In a third aspect, the invention relates to the use of a nucleic acid encoding an artificial protein for producing recombinant virus particles, wherein the recombinant virus particles are suitable for providing a functional ribonucleoprotein (RNP) complex in a target cell, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide.

The features and embodiments, in particular the preferred embodiments, described above with respect to the use of the artificial protein or of the nucleic acid encoding the artificial protein for providing a functional RNP complex, also apply to the use of the nucleic acid encoding the artificial protein for producing recombinant virus particles. This particularly concerns features and embodiments described above regarding the artificial protein, the nucleic acid encoding the artificial protein, the Csy4/Cas9-sgRNA, the pre-Csy4/Cas9-sgRNA, the nucleic acid encoding the pre-Csy4/Cas9-sgRNA, and the target cell.

Likewise, the features and embodiments, in particular the preferred embodiments, described above with respect to the method for providing a functional RNP complex in a target cell, also apply to the use of the nucleic acid encoding the artificial protein for producing recombinant virus particles. This particularly concerns features and embodiments described above regarding the recombinant virus, the "Two-Virus" vector system and the "One-Virus" vector system.

Recombinant virus particles are generally produced in a plurality of packaging cells. The term "packaging cell" as used herein refers to a cell that is suitable for viral packaging, i.e. for the *in vitro* production of recombinant virus particles. The term *"in vitro"* as used herein in relation to the use of a nucleic acid encoding an artificial protein for producing recombinant virus particles is to be understood as "outside a living organism". The packaging cell will be further described further below. In general, the packaging cell is transfected with at least one viral packaging vector and at least one transfer vector to produce and release recombinant virus particles. The production of recombinant virus particles will be further described further below. The recombinant virus particles are suitable for providing a functional RNP complex in the target cell. In other words, transduction of a target cell using the recombinant virus particles is suitable for providing a functional RNP complex in the target cell. The transduction of a target cell for providing the functional RNP complex in the target cell has been described above with respect to the method for providing a functional RNP complex in the target cell and also applies to the recombinant virus particles obtained by the use of the nucleic acid encoding the artificial protein for producing the same.

In a preferred embodiment, the nucleic acid encoding the artificial protein is a DNA molecule. The DNA molecule preferably is part of a transfer vector. The DNA molecule is operably linked to a promoter sequence.

The transfer vector is an expression vector that may comprise viral sequences. For retrovirus particles, the viral sequences of the transfer vector are typically long terminal repeat (LTR) regions that are flanking the cargo nucleic acid. However, in a preferred embodiment, the transfer vector is devoid or substantially devoid of any viral sequences. The transfer vector preferably is a minimal transfer vector. The minimal transfer vector has been described above with respect to the method for providing a functional RNP complex in the target cell. The features and embodiments described above regarding the minimal transfer vector also apply to the use of the nucleic acid encoding the artificial protein for producing recombinant virus particles.

Depending on the type of virus produced, the virus particles may be packaged with a DNA molecule encoding the artificial protein or with an mRNA molecule encoding the artificial protein. The DNA molecule encoding the artificial protein and the mRNA molecule encoding the artificial protein have been described above.

In case a Csy4/Cas9-sgRNA, a pre-Csy4/Cas9-sgRNA or a nucleic acid encoding the pre-Csy4/Cas9-sgRNA is provided in the packaging cell, the virus particles may be further packaged with the Csy4/Cas9-sgRNA, the pre-Csy4/Cas9-sgRNA or the nucleic acid encoding the pre-Csy4/Cas9-sgRNA. The nucleic acid encoding the pre-Csy4/Cas9-sgRNA may be a DNA molecule, wherein the DNA molecule is operably linked to a promoter sequence. Alternatively, the virus particles may be packaged with the functional RNP complex or a precursor of the functional RNP complex. The precursor of the functional RNP complex is formed by the artificial protein and the pre-Csy4/Cas9-sgRNA. The functional RNP complex and the precursor of the functional RNP complex have been described above.

As mentioned before, the inventors surprisingly found that the use of the artificial protein or of the nucleic acid encoding the artificial protein results in a much more efficient formation of the functional RNP complex in a target cell compared to using the Cas9 protein only or to using the Csy4 and Cas9 proteins as separate proteins.

Accordingly, the use of the nucleic acid encoding the artificial protein for producing recombinant virus particles leads to recombinant virus particles that, upon transduction of the target cell using the virus particles, enable a much more efficient formation of the functional RNP complex in the target cell. The more efficient formation of the functional RNP complex facilitates a more efficient site-directed binding of the functional RNP complex to the target nucleic acid and thus a more efficient activity of the functional RNP complex, for example a more efficient editing of the target nucleic acid.

In addition, the inventors surprisingly found that the use of the nucleic acid encoding the artificial protein for producing recombinant virus particles can also lead to an efficient formation of the functional RNP complex in the packaging cell used for virus production. This requires (i) expression of the artificial protein in the packaging cell, and (ii) the provision of a Csy4/Cas9-sgRNA, a pre-Csy4/Cas9-sgRNA or a nucleic acid encoding the pre-Csy4/Cas9-sgRNA in the packaging cell. The latter can be achieved, for example, by transfecting the packaging cell with a transfer vector comprising the nucleic acid encoding the pre-Csy4/Cas9-sgRNA. For each option, the assembly of the functional RNP complex has been described above with respect to the target cell and also applies to the assembly in the packaging cell. The efficient assembly of the functional RNP complex in the packaging cell facilitates the production of virus particles packaged with the functional RNP complex. The inventors found that an efficient packaging of the functional RNP complex into "One-Virus" vector particles is achieved, and that, upon transduction of the target cell, a highly efficient site-directed binding of the functional RNP complex to the target nucleic acid in the target cell takes place. This facilitates an efficient activity of the functional RNP complex, for example an efficient editing of the target nucleic acid.

For providing the Csy4/Cas9-sgRNA, the pre-Csy4/Cas9-sgRNA or the nucleic acid encoding the pre-Csy4/Cas9-sgRNA in the packaging cell, various means are possible. For example, as mentioned above, the packaging cell may be transfected with a transfer vector comprising the nucleic acid encoding the pre-Csy4/Cas9-sgRNA. In this case, the nucleic acid encoding the pre-Csy4/Cas9-sgRNA is a DNA molecule that is part of the transfer vector and that is operably linked to a promoter sequence. In another example, the packaging cell may be a genetically engineered packaging cell that comprises the nucleic acid encoding the pre-Csy4/Cas9-sgRNA in its genome in a manner that facilitates the expression of the pre-Csy4/Cas9-sgRNA in the target cell. In this case, the packaging cell expresses the pre-Csy4/Cas9-sgRNA and does not need to be transfected with a respective transfer vector.

The term "genetically engineered cell" as used herein refers to a modified cell that is derived from a parental cell, in which one or more modifications have been artificially introduced on the gene level (i.e. in the genome) of the cell. The one or more modifications are stably introduced into the cell and passed on to subsequent generations of the cells. Due to the one or more modifications introduced, the genetically engineered cell differs from the parental cell.

The recombinant virus can be any type of virus that is suitable for introducing a cargo nucleic acid and/or another cargo such as the functional RNP complex or a precursor thereof into the target cell. In a preferred embodiment, the recombinant virus is a DNA virus, an RNA virus or a retrovirus. In a further preferred embodiment, the recombinant virus is a retrovirus, wherein the retrovirus preferably is a foamy virus. The features and advantages of using foamy virus have been described above and also apply to the use of the nucleic acid encoding the artificial protein for producing recombinant virus particles.

The foamy virus preferably is prototype foamy virus (PFV). The PFV preferably is an integrase-deficient PFV or a reverse transcriptase-deficient PFV. The features and advantages of using PFV and in particular an integrase-deficient PFV or a reverse transcriptase-deficient PFV have been described above and also apply to the use of the nucleic acid encoding the artificial protein for producing recombinant virus particles.

It is further preferred that the PFV is a reverse transcriptase-deficient PFV that is based on a minimal PFV vector system. The features and advantages of using the reverse transcriptase -deficient PFV that is based on a minimal PFV vector system have been described above and also apply to the use of the nucleic acid encoding the artificial protein for producing recombinant virus particles.

In another preferred embodiment, the retrovirus is a lentivirus, preferably human immunodeficiency virus (HIV), more preferred HIV type 1 (HIV-1), or the retrovirus is a gammaretrovirus, preferably murine leukemia virus (MLV).

In a further aspect, the present invention relates to an *in vitro* method for producing recombinant virus particles, wherein the recombinant virus particles are suitable for providing a functional ribonucleoprotein (RNP) complex in a target cell, the method comprising the steps of
(a) introducing
   (i) a nucleic acid encoding an artificial protein, wherein the artificial protein comprises
      a Csy4 protein,
      a Cas9 protein,
      a linker of 5 to 60 amino acids connecting the Csy4 protein and the Cas9 protein, and
      at least one nuclear localization sequence,
      wherein the linker does not comprise a 2A peptide;
         and
   (ii) at least one viral packaging vector
   into a plurality of packaging cells, thereby obtaining a plurality of producer cells;
(b) culturing the producer cells in a medium, thereby obtaining the recombinant virus particles in the medium; and
(c) separating the medium from the producer cells.

The term *"in vitro"* as used herein in relation to the method for producing recombinant virus particles, wherein the recombinant virus particles are suitable for providing a functional RNP complex in a target cell, is to be understood as "outside a living organism".

In the method for producing recombinant virus particles, the nucleic acid encoding the artificial protein and at least one viral packaging vector are introduced into the plurality of packaging cells. The term "packaging cell" as used herein refers to a cell that is suitable for viral packaging, i.e. for the *in vitro* production of recombinant virus particles. This implies that the packaging cell can be provided with the nucleic acid encoding the artificial protein and one or more viral packaging vectors to produce and release recombinant virus particles. Once the nucleic acid encoding the artificial protein and the at least one viral packaging plasmid are introduced into the packaging cells, the packaging cells are also referred to as "producer cells". The virus particles are released into the medium in which the cells are cultivated. The term "supernatant" as used herein refers to the medium in which the recombinant virus particles are present due to their release from the producer cells. The supernatant is also referred to as viral supernatant, vector supernatant or viral vector supernatant.

In step (a), the packaging cells are provided with all components required for viral packaging. The nucleic acid encoding the artificial protein may be a DNA molecule or an RNA molecule. The DNA molecule encoding the artificial protein and the RNA molecule encoding the artificial protein have been described above. The DNA molecule is operably linked to a promoter sequence and may be part of a transfer vector. The transfer vector can also be referred to as transfer plasmid, transfer vector plasmid, expression vector or expression plasmid. The transfer vector can be introduced into the packaging cells by, for example, transfection of the packaging cells. In case the nucleic acid encoding the artificial protein is an RNA molecule, such as an *in vitro* (cell-free) transcribed mRNA molecule or a synthetic RNA molecule, it can be introduced into the packaging cells by, for example, RNA transfection of the packaging cells. Further means of introducing the nucleic acid encoding the artificial protein into the packaging cells are known, for example electroporation. In addition to the nucleic acid encoding the artificial protein, at least one viral packaging vector harboring the elements required for packaging and encapsulation of the cargo is introduced into the packaging cells. Typically, for example for producing recombinant retrovirus particles, one, two or three viral packaging vectors are used. For retrovirus particles, for example, one packaging vector may comprise the Gag and Pol genes, and, if applicable, the Rev gene, while a different packaging vector may comprise the Env gene, each preferably in an expression-optimized version. Alternatively, three packaging vectors for the viral structural components Gag, Pol and Env may be used, each preferably in an expression-optimized version. For producing recombinant foamy virus particles, it is preferred that the at least one viral packaging vector comprises sequences encoding at least a FV Gag protein and an Env protein, each preferably in an expression-optimized version. A sequence encoding a Pol protein is not required, but may be comprised by the at least one viral packaging vector.

In a preferred embodiment, the nucleic acid encoding the artificial protein and at least one viral packaging vector are introduced into the plurality of packaging cells by transfection of the packaging cells. For the transfection of packaging cells to produce recombinant virus particles, calcium phosphate coprecipitation or cationic polymer-based transfection reagents, in particular polyethylenimine, are commonly used.

During the culture of the producer cells (step (b)), the cells produce recombinant virus particles and release the virus particles into the medium in which the cells are cultivated. In this way, the recombinant virus particles are obtained in the medium. Most cells are cultivated at a temperature of 37°C, a CO₂ concentration of 5% and a relative humidity of 95% in a suitable medium (cell culture medium). Cell culture conditions may be adjusted, for example depending on the type of cell line used.

After a sufficient period of time for virus production (for example about 48, 72 or 96 hours), the medium (supernatant) is separated from the producer cells (step (c)). This separation step serves to harvest the recombinant virus particles produced. The separation step can be performed by commonly used techniques such as collecting the medium and preferably centrifuging the collected medium to pellet any producer cells that may have been taken up during medium collection. In this way, cell-free (i.e. released) virus particles are harvested.

The separation step can alternatively be performed by freeze-thawing of the producer cells together with the medium and subsequent removal of cell debris by low-speed centrifugation and/or sterile filtration. In this way, cell-bound virus particles are also harvested.

Optionally, the virus particles may then be recovered from the medium. The term "recovery" as used herein refers to the concentration of the medium in which the virus particles are present in order to obtain a higher virus titer. The recovery or concentration step can be performed by commonly used techniques such as ultracentrifugation or ultrafiltration.

The features and embodiments, in particular the preferred embodiments, described above with respect to the use of the artificial protein or of the nucleic acid encoding the artificial protein for providing a functional RNP complex, also apply to the method for producing recombinant virus particles. This particularly concerns features and embodiments described above regarding the artificial protein, the nucleic acid encoding the artificial protein, the Csy4/Cas9-sgRNA, the pre-Csy4/Cas9-sgRNA, the nucleic acid encoding the pre-Csy4/Cas9-sgRNA, and the target cell.

Likewise, the features and embodiments, in particular the preferred embodiments, described above with respect to the method for providing a functional RNP complex in a target cell, also apply to the method for producing recombinant virus particles. This particularly concerns features and embodiments described above regarding the recombinant virus, the "Two-Virus" vector system and the "One-Virus" vector system.

Likewise, the features and embodiments, in particular the preferred embodiments, described above with respect to the use of a nucleic acid encoding an artificial protein for producing recombinant virus particles, also apply to the method for producing recombinant virus particles.

As mentioned before, the inventors surprisingly found that the use of the artificial protein or of the nucleic acid encoding the artificial protein results in a much more efficient formation of the functional RNP complex in a target cell compared to using the Cas9 protein only or to using the Csy4 and Cas9 proteins as separate proteins. Accordingly, the method for producing recombinant virus particles leads to recombinant virus particles that, upon transduction of the target cell using the virus particles, enable a much more efficient formation of the functional RNP complex in the target cell. The more efficient formation of the functional RNP complex facilitates a more efficient site-directed binding of the functional RNP complex to the target nucleic acid and thus a more efficient activity of the functional RNP complex, for example a more efficient editing of the target nucleic acid.

In a preferred embodiment, step (a) further comprises introducing either
(iii-1) a Csy4/Cas9-single guide RNA (Csy4/Cas9-sgRNA), wherein the Csy4/Cas9-sgRNA comprises
   a classical Cas9-sgRNA sequence, and
   a Csy4 cleavage sequence located downstream of the classical Cas9-sgRNA
   sequence,
      or
(iii-2) a pre-Csy4/Cas9-sgRNA or a nucleic acid encoding the pre-Csy4/Cas9-sgRNA, wherein the pre-Csy4/Cas9-sgRNA comprises
   a classical Cas9-sgRNA sequence,
   a Csy4 cleavage sequence located downstream of the classical Cas9-sgRNA sequence, and
   an additional Csy4 cleavage sequence located directly upstream of the classical Cas9-sgRNA sequence,
into the plurality of packaging cells.

The Csy4/Cas9-sgRNA or the pre-Csy4/Cas9-sgRNA can be introduced into the packaging cells by, for example, RNA transfection or electroporation of the packaging cells. The nucleic acid encoding the pre-Csy4/Cas9-sgRNA may be a DNA molecule that is operably linked to a promoter sequence and that is part of a transfer vector. The transfer vector can be introduced into the packaging cells by, for example, transfection or electroporation of the packaging cells. In a preferred embodiment, the Csy4/Cas9-sgRNA, the pre-Csy4/Cas9-sgRNA or the nucleic acid encoding the pre-Csy4/Cas9-sgRNA are introduced into the plurality of packaging cells by transfection of the packaging cells.

As mentioned above, the inventors surprisingly found that the use of the nucleic acid encoding the artificial protein for producing recombinant virus particles can also lead to an efficient formation of the functional RNP complex in the packaging cell used for virus production. This requires the provision of the Csy4/Cas9-sgRNA, the pre-Csy4/Cas9-sgRNA or the nucleic acid encoding the pre-Csy4/Cas9-sgRNA in the packaging cell. For each option, the assembly of the functional RNP complex has been described above with respect to the target cell and also applies to the assembly in the packaging cell. The efficient assembly of the functional RNP complex in the packaging cell facilitates the production of virus particles packaged with the functional RNP complex. The inventors found that an efficient packaging of the functional RNP complex into "One-Virus" vector particles is achieved, and that, upon transduction of the target cell, a highly efficient site-directed binding of the functional RNP complex to the target nucleic acid in the target cell takes place. This facilitates an efficient activity of the functional RNP complex, for example an efficient editing of the target nucleic acid.

In a preferred embodiment, the nucleic acid encoding the pre-Csy4/Cas9-sgRNA is introduced into the plurality of packaging cells.

In another preferred embodiment, the packaging cell is a genetically engineered packaging cell that comprises the nucleic acid encoding the pre-Csy4/Cas9-sgRNA in its genome in a manner that facilitates the expression of the pre-Csy4/Cas9-sgRNA in the target cell. In this case, the packaging cell expresses the pre-Csy4/Cas9-sgRNA and the step of introducing the Csy4/Cas9-sgRNA, the pre-Csy4/Cas9-sgRNA or the nucleic acid encoding the pre-Csy4/Cas9-sgRNA into the packaging cells is not needed.

The recombinant virus can be any type of virus that is suitable for introducing a cargo nucleic acid and/or another cargo such as the functional RNP complex or a precursor thereof into the target cell. In a preferred embodiment, the recombinant virus is a DNA virus, an RNA virus or a retrovirus. In a further preferred embodiment, the recombinant virus is a retrovirus, wherein the retrovirus preferably is a foamy virus. The features and advantages of using foamy virus have been described above and also apply to the method for producing recombinant virus particles.

The foamy virus preferably is prototype foamy virus (PFV). The PFV preferably is an integrase-deficient PFV or a reverse transcriptase-deficient PFV. The features and advantages of using PFV and in particular an integrase-deficient PFV or a reverse transcriptase-deficient PFV have been described above and also apply to the method for producing recombinant virus particles.

It is further preferred that the PFV is a reverse transcriptase-deficient PFV that is based on a minimal PFV vector system. The features and advantages of using the reverse transcriptase-deficient PFV that is based on a minimal PFV vector system have been described above and also apply to the method for producing recombinant virus particles.

In another preferred embodiment, the retrovirus is a lentivirus, preferably human immunodeficiency virus (HIV), more preferred HIV type 1 (HIV-1), or the retrovirus is a gammaretrovirus, preferably murine leukemia virus (MLV).

In a preferred embodiment, the Csy4 cleavage sequence comprises the sequence
GUUCACUGCCGUAUAGGCAG (SEQ ID NO.: 11),
GUUCACUGCCGUAUAGGCAGCUAAGAAA (SEQ ID NO.: 12),
GUACACUGCCGUAUAGGCAG (SEQ ID NO.: 13),
GUAAACUGCCGUAUAGGCAG (SEQ ID NO.: 14), or
GUUCCCUGCCGUAUAGGCAG (SEQ ID NO.: 15).

In a further preferred embodiment, the Csy4 cleavage sequence consists of the sequence of SEQ ID NO.: 11, 12, 13, 14 or 15.

In a preferred embodiment, the pre-Csy4/Cas9-sgRNA further comprises an additional Csy4 cleavage sequence located directly upstream of the classical Cas9-sgRNA sequence. In other words, the 3' end of the additional Csy4 cleavage sequence is directly adjacent to the 5' end of the classical Cas9-sgRNA sequence. The advantages of using such a pre-Csy4/Cas9-sgRNA have been described above and also apply to the method for producing recombinant virus particles.

Further preferred embodiments regarding the pre-Csy4/Cas9-sgRNA, for example a multiplex pre-Csy4/Cas9-sgRNA, have been described above and also apply to the method for producing recombinant virus particles.

In a preferred embodiment, the packaging cells are cells of a cell line, wherein the cell line is selected from the group consisting of human embryonic kidney 293T (HEK293T) cell line, human embryonic kidney 293 (HEK293) cell line, HeLa cell line, D-17 cell line, HT1080 cell line, TE671 cell line, MV-1-Lu cell line and HOS cell line.

These cell lines are known to serve as suitable mammalian packaging cell lines for producing recombinant virus particles.

In addition, the cell line may be a variant of the indicated cell lines. Variants of the cell lines that can also serve as suitable mammalian packaging cell lines for producing recombinant virus particles are known. For example, the variant may be a variant that lacks cell surface expression of heparan sulfate, for example due to inactivation of the B3GAT3 gene encoding the B3GAT3 protein. Such variants are described in EP 3 822 346 A1 and are particularly useful for producing FV Env-comprising retroviral vector particles. In a preferred embodiment, the cell line is a HEK293T cell line in which the B3GAT3 gene encoding the B3GAT3 protein is inactivated.

The HEK293T cell line is a human cell line derived from the HEK293 cell line by modifying the HEK293 cell line to express the SV40 large T antigen to allow improved replication and expression of transfected plasmids containing the SV40 origin of replication. The HEK293T cell line is a very commonly used packaging cell line for producing recombinant virus particles, in particular retrovirus particles. HEK293T cells are easy to handle, can be easily transfected using standard protocols and have a good transfection efficiency.

HEK293 cells can be used as packaging cells for, for example, recombinant adenovirus (AV) and adeno-associated virus (AAV) particles.

HeLa cells are human epithelial cells derived from cervical cancer. HeLa cells can be used as packaging cells for, for example, recombinant AAV particles.

The D-17 cell line is a canine osteosarcoma cell line. D-17 cells can be used as packaging cells for, for example, recombinant retrovirus particles.

The HT1080 cell line is a human fibroblast cell line. HT1080 cells can be used as packaging cells for, for example, recombinant retroviruses such as murine leukemia viruses (MLV).

The TE671 cell line is a human cancer cell line. The MV-1-Lu (also referred to as Mv1Lu) cell line is a mink lung epithelial cell line. The HOS cell line is a human osteosarcoma cell line. TE671, MV-1-Lu and HOS cells can each be used as packaging cells for, for example, recombinant retroviruses such as murine leukemia viruses (MLV).

Further disclosed is a recombinant virus particle obtainable by the method of the invention for producing recombinant virus particles. The recombinant virus particle has been described above. The features and embodiments, in particular the preferred embodiments, described above with respect to the method for producing recombinant virus particles, also apply to the recombinant virus particle obtainable by the method.

In a preferred embodiment, the recombinant virus particle comprises a functional RNP complex.

As mentioned above, the inventors surprisingly found that the use of the nucleic acid encoding the artificial protein for producing recombinant virus particles facilitates the production of virus particles packaged with the functional RNP complex due to the efficient assembly of the functional RNP complex in the packaging cell. The inventors found that an efficient packaging of the functional RNP complex into "One-Virus" vector particles is achieved, and that, upon transduction of the target cell, a highly efficient site-directed binding of the functional RNP complex to the target nucleic acid in the target cell takes place. This facilitates an efficient activity of the functional RNP complex, for example an efficient editing of the target nucleic acid.

Accordingly. for the "One-Virus" vector system, the *in vitro* method for providing a functional ribonucleoprotein (RNP) complex in a target cell preferably comprises the steps of
(a) introducing
   (i) a nucleic acid encoding an artificial protein, wherein the artificial protein comprises
      a Csy4 protein,
      a Cas9 protein,
      a linker of 5 to 60 amino acids connecting the Csy4 protein and the Cas9 protein, and
      at least one nuclear localization sequence,
      wherein the linker does not comprise a 2A peptide;
   (ii) at least one viral packaging vector;
      and
   (iii) either
      (iii-1) a Csy4/Cas9-single guide RNA (Csy4/Cas9-sgRNA), wherein
         the Csy4/Cas9-sgRNA comprises
         a classical Cas9-sgRNA sequence, and
         a Csy4 cleavage sequence located downstream of the classical Cas9-sgRNA sequence,
            or
      (iii-2) a pre-Csy4/Cas9-sgRNA or a nucleic acid encoding the pre-Csy4/Cas9-sgRNA, wherein the pre-Csy4/Cas9-sgRNA comprises
         a classical Cas9-sgRNA sequence,
         a Csy4 cleavage sequence located downstream of the classical Cas9-sgRNA sequence, and
         an additional Csy4 cleavage sequence located directly upstream of the classical Cas9-sgRNA sequence,
   into a plurality of packaging cells, thereby obtaining a plurality of producer cells;
(b) culturing the producer cells in a medium, thereby obtaining recombinant virus particles in the medium;
(c) separating the medium from the producer cells; and
(d) transducing the target cell with the medium obtained in step (c), wherein the medium is optionally diluted or concentrated before transducing the target cell with the medium.

Further disclosed is the use of a DNA virus harboring a DNA molecule encoding an artificial protein for providing a functional ribonucleoprotein (RNP) complex in a target cell, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide.

Further disclosed is the use of an RNA virus harboring an mRNA molecule encoding an artificial protein for providing a functional ribonucleoprotein (RNP) complex in a target cell, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide.

Further disclosed is the use of a retrovirus harboring an mRNA molecule encoding an artificial protein for providing a functional ribonucleoprotein (RNP) complex in a target cell, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide.

In a preferred embodiment, the retrovirus is a foamy virus, wherein the foamy virus further harbors a pre-Csy4/Cas9-sgRNA, wherein the pre-Csy4/Cas9-sgRNA comprises
a classical Cas9-sgRNA sequence,
a Csy4 cleavage sequence located downstream of the classical Cas9-sgRNA sequence, and
an additional Csy4 cleavage sequence located directly upstream of the classical Cas9-sgRNA sequence.

The features and embodiments, in particular the preferred embodiments, described above with respect to the use of the artificial protein or of the nucleic acid encoding the artificial protein for providing a functional RNP complex, also apply to the disclosed use of a DNA virus, RNA virus and retrovirus, respectively. This particularly concerns features and embodiments described above regarding the artificial protein, the nucleic acid encoding the artificial protein, the Csy4/Cas9-sgRNA, the pre-Csy4/Cas9-sgRNA, the nucleic acid encoding the pre-Csy4/Cas9-sgRNA, and the target cell.

Likewise, the features and embodiments, in particular the preferred embodiments, described above with respect to the method for providing a functional RNP complex in a target cell, also apply to the disclosed use of a DNA virus, RNA virus and retrovirus, respectively. This particularly concerns features and embodiments described above regarding the recombinant virus, the "Two-Virus" vector system and the "One-Virus" vector system.

Likewise, the features and embodiments, in particular the preferred embodiments, described above with respect to the use of a nucleic acid encoding an artificial protein for producing recombinant virus particles and with respect to the method for producing recombinant virus particles, also apply to the disclosed use of a DNA virus, RNA virus and retrovirus, respectively.

Further disclosed is an *in vivo* method for providing a functional ribonucleoprotein (RNP) complex in a target cell, the method comprising the step of introducing a component (a) and a component (b) into the target cell,
wherein the component (a) is an artificial protein or a nucleic acid encoding the artificial protein, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide;
and wherein the component (b) is
either
   (b-1) a Csy4/Cas9-single guide RNA (Csy4/Cas9-sgRNA), wherein the Csy4/Cas9-sgRNA comprises
      a classical Cas9-sgRNA sequence, and
      a Csy4 cleavage sequence located downstream of the classical Cas9-sgRNA sequence,
         or
   (b-2) a pre-Csy4/Cas9-sgRNA or a nucleic acid encoding the pre-Csy4/Cas9-sgRNA, wherein the pre-Csy4/Cas9-sgRNA comprises
      a classical Cas9-sgRNA sequence,
      a Csy4 cleavage sequence located downstream of the classical Cas9-sgRNA sequence, and
      an additional Csy4 cleavage sequence located directly upstream of the classical Cas9-sgRNA sequence.

The term *"in vivo"* as used herein in relation to the disclosed *in vivo* method for providing a functional RNP complex in a target cell is to be understood as "inside a living organism".

The features and embodiments, in particular the preferred embodiments, described above with respect to the use of the artificial protein or of the nucleic acid encoding the artificial protein for providing a functional RNP complex, also apply to the disclosed *in vivo* method for providing a functional RNP complex in a target cell. This particularly concerns features and embodiments described above regarding the artificial protein, the nucleic acid encoding the artificial protein, the Csy4/Cas9-sgRNA, the pre-Csy4/Cas9-sgRNA, the nucleic acid encoding the pre-Csy4/Cas9-sgRNA, and the target cell.

Likewise, the features and embodiments, in particular the preferred embodiments, described above with respect to the *in vitro* method for providing a functional RNP complex in a target cell, also apply to the disclosed *in vivo* method for providing a functional RNP complex in a target cell. This particularly concerns features and embodiments described above regarding the recombinant virus, the "Two-Virus" vector system and the "One-Virus" vector system.

Further disclosed is the use of a producer cell for producing recombinant virus particles, wherein the recombinant virus particles are suitable for providing a functional ribonucleoprotein (RNP) complex in a target cell, wherein the producer cell comprises
(i) a nucleic acid encoding an artificial protein, wherein the artificial protein comprises
   a Csy4 protein,
   a Cas9 protein,
   a linker of 5 to 60 amino acids connecting the Csy4 protein and the Cas9 protein, and
   at least one nuclear localization sequence,
   wherein the linker does not comprise a 2A peptide;
      and
(ii) at least one viral packaging vector.

The features and embodiments, in particular the preferred embodiments, described above with respect to the use of a nucleic acid encoding an artificial protein for producing recombinant virus particles and with respect to the method for producing recombinant virus particles, also apply to the disclosed use of a producer cell for producing recombinant virus particles.

Further aspects of the invention will be apparent to the person skilled in the art by the enclosed description of the examples, in particular the scientific results.

### Examples

The following examples relate to the use of an artificial protein or of a nucleic acid encoding the artificial protein for providing a functional ribonucleoprotein (RNP) complex in a target cell. The functional RNP complex is intended to alter a target gene in the target cell ("gene editing"). The artificial protein used comprises a Csy4 protein, a Cas9 protein, an amino acid linker connecting the Csy4 protein and the Cas9 protein, and two nuclear localization sequences. In the following, the Csy4 protein is also referred to as, inter alia, Csy4 nuclease, Csy4 ribonuclease or C4. The Cas9 protein is a Cas9 protein that has its native enzymatic nuclease activity. In the following, it is also referred to as, inter alia, Cas9 nuclease, Cas9 deoxyribonuclease or C9. The artificial protein is also referred to as, inter alia, C4/C9-combinuclease, combinuclease, chimeric C4/C9-nuclease, chimeric nuclease or chimeric C4/C9-enzyme. The linker is a glycine-serine rich linker that comprises several G4S motifs. The linker does not comprise a 2A peptide.

The pre-Cas9-sgRNA used is a pre-Csy4/Cas9-sgRNA comprising a single classical Cas9-sgRNA sequence, a Csy4 cleavage sequence located downstream of the classical Cas9-sgRNA sequence, and an additional Csy4 cleavage sequence located directly upstream of the classical Cas9-sgRNA sequence. The Csy4 cleavage sequence is also referred to as Csy4 scaffold region, C4-specific sgRNA scaffold region, C4-sR or sR. The pre-Csy4/Cas9-sgRNA is also referred to as pre-C4/C9-sgRNA or chimeric pre-sgRNA. The mature Csy4/Cas9-sgRNA is also referred to as C4/C9-sgRNA or chimeric sgRNA.

The Cas9-sgRNA used in some experiments is a classical, non-chimeric Cas9-sgRNA that consists of a single classical Cas9-sgRNA sequence. The Cas9-sgRNA is also referred to as C9-sgRNA.

The following examples also relate to the method for providing a functional RNP complex in the target cell. The two components needed to provide the functional RNP complex in the target cell (i.e. the nuclease component and the sgRNA component) are introduced into the target cell by viral transduction of the target cell, in particular using prototype foamy virus (PFV).

In the "One-Virus" CRISPR vector system ("One-Virus" vector system), the nuclease component (such as the C4/C9-combinuclease or a nucleic acid encoding the same) and the sgRNA component (such as a pre-C4/C9-sgRNA or a nucleic acid encoding the same) of the CRISPR system are transferred into the target cell in the same viral vector particle. In the "Two-Virus" CRISPR vector system ("Two-Virus" vector system), the nuclease component (such as the C4/C9-combinuclease or a nucleic acid encoding the same) and the sgRNA component (such as a pre-C4/C9-sgRNA or a nucleic acid encoding the same) of the CRISPR system are transferred into the target cell in separate viral vector particles.

The terms "TraFo", "TraFo vector", "TraFo virus vector particle", "TraFo supernatant" and the like refer to a prototype foamy virus (PFV) RNA transfer vector system that facilitates transient genetic modification of target cells by transfer of non-viral RNAs.

The following examples also relate to the use of a nucleic acid encoding an artificial protein for producing recombinant virus particles, wherein the recombinant virus particles are suitable for providing a functional RNP complex in the target cell. For producing recombinant virus particles, in particular recombinant PFV particles, the nucleic acid encoding the artificial protein is a DNA molecule that is part of a transfer vector that is used for transfecting a HEK293T packaging cell.

The following examples also relate to the method for producing recombinant virus particles, wherein the recombinant virus particles are suitable for providing a functional RNP complex in the target cell.

### Materials and methods

### Cells and cell culture conditions

The human embryonic kidney cell line HEK293T (ATCC CRL-1573), the human osteosarcoma U-2 OS cell line (ATCC HTB-96) variant U2OS*gfp* harboring a single lentiviral vector genome integrant with a constitutively expressed, CMV promotor-driven destabilized EGFP-PEST (dsEGFP) reporter protein ORF were cultivated in Dulbecco's modified Eagle's medium (DMEM) supplemented with 10% heat-inactivated fetal calf serum and antibiotics. The human T lymphocyte Jurkat cell line (ATCC TIB-152) was cultivated in RPMI supplemented with 10% heat-inactivated fetal calf serum, 1% non-essential amino acids, 1% sodium pyruvate and 1% L-Alanine, L-Glutamate. Cells were cultivated in cell culture flasks in an incubator (37°C, 5% CO₂, 95% humidity). All work with cells and cell lines was conducted under sterile conditions.

### Recombinant plasmid DNAs

Integration-competent (ICPV) or integration-deficient (IDPV) recombinant PFV vector particles were generated with a four-component vector system, which has been described previously (Hamann et al. 2014). Briefly, it consists of the expression-optimized packaging constructs pcoPG4 (PFV Gag), pcoPE (PFV Env) or pcoSE (SFVmcy Env), pcoPP (Pol), and different variants of the PFV transfer vector puc2MD9. In some experiments, a previously described variant of the PFV Pol packaging construct, containing an ORF with catalytically inactive integrase (pcoPP3, Pol ilN, D₉₃₆A mutation), was used. In addition, a recently described three-component PFV RNA transfer vector system (TraFo) for transient genetic modification of target tissue by transfer of non-viral RNAs was used (Hamann et al. 2014, Lindel et al. 2019). For production of TraFo particles pseudotyped with VSV-G, an expression-optimized version of a PFV Gag packaging plasmid (pcoPG4 M6), encoding a PFV Gag protein with an N-terminal HIV-1 Gag matrix domain (MA) (PG M6) enabling PFV Env independent particle egress, and pcziVSV-G were used (Ho et al. 2012). Alternatively, instead of pcoPG4 M6 a combination of an expression construct encoding a HIV-1 Gag MA domain with C-terminal FR (CFR) heterodimerization domain and an expression construct encoding a PFV Gag with N-terminal FK (NFK) heterodimerization domain were employed for VSV-G pseudotyped PFV vector production (Ho et al. 2012). A schematic outline of the packaging components is shown in Fig. 2C, D. The SpCas9 ORF used in this study was derived from pL-CRISPR.ESF.tRFP (a gift from Benjamin Ebert, Addgene plasmid #57819). The hCsy4 ORF used in this study was derived from pSQT1601 (a gift from Keith Joung, Addgene plasmid #53371 (Tsai et al. 2014)). It is expression-optimized for human cells. The coCsy4 ORF used in this study was independently designed and synthesized by "Geneart" (Thermo Fisher Scientific). U6 promoter-driven sgRNA expression cassettes were derived from pFYF1320 EGFP Site#1 (a gift from Keith Joung, Addgene plasmid #47511). The C4/C9-gfp+174-112 fragment used to generate the TraFo transfer vector pre-C4/C9-gfp+174-112 (pRP2a Red-C4C9gRNA egfp+174-112) was derived from RFN_EGFP_81 (a gift from Keith Joung, Addgene plasmid #60073). Targeting sequences of the sgRNAs used in this study are listed in Table 1. Schematic outlines of the expression constructs encoding the different Cas9 and/or sgRNA encoding vector genomes or the lentiviral shRNA transfer vectors are shown in Fig. 2A, B, D. The schematic outline of all newly generated expression constructs, encoding various combinations of Csy4 (C4) and Cas9 (C9) nucleases in different arrangements in context of the pczi vector backbone, as well as various sgRNAs and pre-sgRNAs driven by different promoters in context of pRP2a Red, pFYF1320 or puc2MD9 vector backbones, is illustrated in Fig. 2A and B. All new constructs were verified by diagnostic digest and sequencing analysis.

Integration-competent (ICLV) or integration-deficient (IDLV) lentiviral particles pseudotyped with SFVmcy Env or VSV-G were produced using the packaging vectors pcoSE03, encoding a mutant SFVmcy Env or pcziVSV-G in combination with the wildtype HIV-1 Gag-Pol expressing packaging vector pCD/NL-BH (for ICLVs) or its integrase-deficient variant pCD/NL-BH ΔIN (Lindel et al. 2019) (for IDLVs) and an HIV-1 transfer vector. MLV particles were produced using the MLV Gag-Pol expressing packaging vector pHIT60 in combination with glycoprotein packaging plasmids and transfer vectors.

### PFV packaging plasmids

pcoPG4: Eukaryotic expression plasmid for codon-optimized PFV gag, CMV enhancer/promoter, based on pcDNA 3.1 zeo⁺, includes CMV intron A for expression of spliced mRNAs.

pcoPP: Eukaryotic expression plasmid for codon-optimized PFV pol, CMV enhancer/promoter, based on pcziPol, includes CMV intron A for expression of spliced mRNAs.

pcoPE: Eukaryotic expression plasmid for codon-optimized PFV env, CMV enhancer/ promoter, based on pcDNA 3.1 zeo⁺, includes CMV intron A for expression of spliced mRNAs.

pcoSE: Eukaryotic expression plasmid for codon-optimized SFVmac (SFVmcy) env, CMV enhancer/promoter, based on pcDNA 3.1 zeo⁺, includes CMV intron A for expression of spliced mRNAs.

### HIV or MLV packaging plasmids

pCD/NL-BH: Eukaryotic expression plasmid for HIV-1 Gag/Pol, Rev, CMV enhancer/promoter, based on pcDNA.

pHIT60: Eukaryotic expression plasmid for MLV Gag/Pol, CMV enhancer/promoter, based on pBR322.

pcoPE01: Eukaryotic expression plasmid for codon-optimized PFV env variant for efficient pseudotyping, CMV enhancer/promoter, based on pcDNA 3.1 zeo⁺, includes CMV intron A for expression of spliced mRNAs.

pcoSE03: Eukaryotic expression plasmid for codon-optimized SFVmac (SFVmcy) env variant for efficient pseudotyping, CMV enhancer/promoter, based on pcDNA 3.1 zeo⁺, includes CMV intron A for expression of spliced mRNAs.

**Table 1**

| sgRNA | Target gene/ locus | C9-sgRNA targeting sequence with PAM (*) | SEQ ID NO.: |
|---|---|---|---|
| stuffer | | CGAGACGAGCTTGCGTCTCG NGG | 16 |
| gfp-148 | EGFP | GGGCACGGGCAGCTTGCCGG TGG | 17 |
| gfp-197 | EGFP | GCTGAAGCACTGCACGCCGT AGG | 18 |
| gfp-112 | EGFP | CAGGGTCAGCTTGCCGTAGG TGG | 19 |
| gfp+174 | EGFP | CCGGCAAGCTGCCCGTGCCC TGG | 20 |
| gfp c.187 A>G | EGFP | GCCGTAGGTCAGGGTGGTCACG AGG | 21 |
| sgTRAC1 | hTRAC | gCAGGGTTCTGGA T ATCTGT GGG | 22 |
| sgTRAC3 | hTRAC | gAGAGTCTCTCAGCTGGTACA CGG | 23 |

| | | | |
|---|---|---|---|
| (*) Lowercase indicates non-matching additional 5' G residues. Base editor target nucleotides are highlighted in bold. The PAM is underlined. | | | |

### Viral vector production and target cell transduction

Cell culture supernatants containing recombinant viral particles were generated by transfection of the corresponding plasmids into HEK293T cells (packaging cells) using polyethyleneimine (PEI) as described previously (Hamann et al. 2014). Briefly, the individual plasmids of the different retroviral vector systems (16 µg total DNA) were transiently cotransfected into 6 x 10⁶ HEK293T cells, plated 18 to 24 h in advance in 10 cm culture dishes, using 48 µg PEI. For ICPV and IDPV supernatants, transfer vector as well as Gag, Pol and Env packaging components were used at a ratio of 14.25 : 3.25 : 1.5 : 1. For TraFo supernatants, transfer vector(s), Gag and Env packaging components were used at a ratio of 15 : 4 : 1. Lentiviral and MLV vector supernatants were produced using transfer vector to Gag/Pol and Env packaging construct ratios of 7 : 7 : 1. If necessary, empty pUC19 plasmid was used in all experiments as stuffer to keep the total DNA amount of transfected DNA constant. At 26 to 30 hours post transfection, the medium was exchanged for fresh culture medium and cell-free viral vector supernatant was harvested 48 h post transfection by using 0.45 µm sterile filters. Plain, cell-free viral supernatants in complete RPMI 1640 (for Jurkat) or DMEM (all other cells) were used either freshly harvested or stored frozen at -80°C.

U2OS*gfp* target cells were seeded at a density of 2.5 × 10⁴ cells/ml in 12-well plates 16 to 24 h prior to transduction. For transduction, target cells were incubated with 1 ml viral vector supernatants for 4-5 h. Combined virus infections were conducted simultaneously at 1:1 volume ratio of plain viral vector supernatants if not stated otherwise. Following aspiration of vector supernatants, transduced target cells were cultivated in fresh growth medium for different time periods as indicated.

For transduction of Jurkat cells, 2 × 10⁵ cells in 10 µl volume were added to 500 µl of viral supernatant in complete RPMI 1640 medium in 48-well plates. Cell - virus mixtures were spinoculated (600 g, 2 h, 36°C) and subsequently cultivated overnight at 37°C, 5% CO₂. On the next day, cells were pelleted and resuspended in fresh RPMI medium. Cells were cultured at a density of approx. 5 × 10⁵ cells/ml every other day until the day of analysis. Anti-CD3 cell surface staining analyzed by flow cytometry on day 7 post infection was used as read-out for successful gene engineering.

### Gene inactivation and editing analysis

For all viral transductions of CRISPR/Cas9 components into U2OS*gfp* target cells, the percentage of non-fluorescent cells, indicating successful gene inactivation, was analyzed by flow cytometry at various time points post-transduction as indicated. Flow cytometry analysis was conducted on a MACSQuant analyzer (Miltenyi Biotec). All transduction experiments were performed at least three times using viral supernatants derived from independent transfections if not stated otherwise. The efficiency of *egfp* ORF inactivation in U2OS*gfp* cells was calculated by the percentage of non-fluorescent cells at the given time point. For T cell receptor KO analysis 1 × 10⁵ Jurkat cells were once washed with staining buffer (PBS, 2% FCS) and resuspended in 50 µl of anti-CD3 antibody solution (80 ng/µl; mouse UCHT1; IgG1 isotype; Biolegend). Cells were kept on ice for 60 min without light and washed 3 × with staining buffer. Subsequently, cells were diluted and examined by flow cytometry analysis.

### Purification of particulate material by ultracentrifugation

Supernatant generated by transient transfection (as described above) was harvested, passed through a 0.45 µm filter and centrifuged at 4°C and 25,000 rpm for 2 h in a SW32Ti rotor (Beckman) through a 20% sucrose cushion. The particulate material was resuspended in phosphate-buffered saline (PBS) resulting in a 100-fold (v/v) concentration.

### Quantitative PCR analysis

For qPCR analysis of viral particle nucleic acid composition DNase I (0.35 U/µl final concentration) and a 1/10^{th} volume of 10x DNase I Buffer were added to the pelleted viral particle preparations resuspended in PBS. After incubation for 1 h at 37°C nucleic acids were extracted using the Quick-RNA Viral Kit (Zymo Research) according to the manufacturer's instructions. Viral nucleic acids from 7 ml pelleted plain vector supernatant were eluted in 15 µl DNase- and RNase-free ddH₂O. Ten microliter of the nucleic acid eluate were DNase I (50 mU/µl final concentration) digested in 20 µl total volume for 30 min at 37°C using the RapidOut DNA Removal Kit (ThermoFisher Scientific) according to the manufacturer's instructions. After DNase inactivation samples were diluted 1 : 10 with DNase- and RNase-free ddH₂O. Five microliter of this diluted DNase-I digested nucleic acid solution was used in a 25 µl RT-qPCR amplification reaction employing the Luna Universal probe One-Step RT qPCR Kit according the manufacturer's instructions. Primers, Taqman probes and cycling conditions for specific quantification of Cas9 mRNA, pre-C4/C9-sgRNAs, total C4/C9-sgRNAs are summarized in Table 2. All values obtained were referred to a standard curve consisting of 10-fold serial dilutions of respective reference plasmids containing the target sequences. All sample values included were in the linear range of the standard curves with a span from 10² to 10⁹ copies per amplification reaction and subsequently normalized per ml plain vector supernatant.

**Table 2**

| Target | Primer/ Probe | Sequence (5'-3') | Cycle Conditions |
|---|---|---|---|
| Cas9 | fwd | GGACCAGGAACTGGACATCA (SEQ ID NO.: 24) | 95 °C, 1 min, 1x |
| | | | 95 °C, 10 s, 40x |
| | rev | TCGATGGAGTCGTCCTTCAG (SEQ ID NO.: 25) | 56 °C, 1 min, 40x |
| | probe | FAM- CCACATCGTAGTCGGACAGCCGG T-BHQ1 (SEQ ID NO.: 26) | |
| pre-C4/C9-sgRNA | fwd | CGGTGCGTTCACTGCCGTAT (SEQ ID NO.: 27) | 95 °C, 1 min, 1x |
| | | | 95 °C, 10 s, 40x |
| | rev | AGCAGCGTATCCACATAGCGT (SEQ ID NO.: 28) | 60 °C, 1 min, 40x |
| | probe | ATTO550- TTGTCGACGCGGCCGCTGCC- BHQ2 (SEQ ID NO.: 29) | |
| C9-sgRNA | fwd | GTTTTAGAGCTAGAAATAGCAAGT (SEQ ID NO.: 30) | 95 °C, 1 min, 1x |
| | | | 95 °C, 10 s, 40x |
| | rev | CCGACTCGGTGCCACTTTT (SEQ ID NO.: 31) | 60 °C, 1 min, 40x |
| | probe | FAM-ACGGACTAGCCTTATTTTA- MGB (SEQ ID NO.: 32) | |

| | | | |
|---|---|---|---|
| (fwd: forward primer; rev: reverse primer; FAM: fluorescein; BHQ1: black hole quencher 1; BHQ2: black hole quencher 2; ATTO550: ATTO 550 fluorescent label (ATTO-TEC GmbH, Siegen, Germany); MGB: minor groove binder) | | | |

### Statistical analysis

If not stated otherwise, data are shown as means ± SEM. Bar charts are shown with scatter plots of individual data points. Statistical analysis was performed using Graphpad Prism V9.2.0 using the tests as indicated in the individual figure legends. Significance levels in each figure are indicated by asterisks (*p ≤ 0.05, **p < 0.01, ***p < 0.005, ****p < 0.001).

### Results

Before the scientific results are described in detail, it is referred to Fig. 1 which visualizes the prior art (Fig. 1A, 1B) in comparison to the underlying principle of the present invention (Fig. 1C). The classical CRISPR/Cas9 genome editing tool comprises two components (Cas9 Classic (C9), Fig. 1A). First, a protein, the Cas9 deoxyribonuclease (C9), which provides the required catalytic activity for nucleic acid manipulation. Second, a ribonucleic acid, the Cas9-specific single guide RNA (C9-sgRNA), which is an artificial chimeric RNA composed of CRISPR RNA (cRNA) and trans-activating CRISPR RNA (tracrRNA) sequence elements, and determines the target sequence specificity. The C9-nuclease can be provided by various means as for example, in the context of an RNA polymerase II (RNAP-II) driven eukaryotic expression cassette resulting in the transcription of a capped and polyadenylated mRNA, as *in vitro* transcribed or chemically synthesized and modified (m)RNA, or as recombinant protein. The C9-sgRNA on the other side can be provided for example, in the context of a RNAP-III driven eukaryotic expression cassette resulting in transcripts lacking cap and poly-A tail modifications, or as *in vitro* transcribed or chemically synthesized and modified RNA. Both CRISPR/Cas9 components can be assembled into the functional ribonucleoprotein (RNP) complex either in the target cell or tissue, or pre-assembled *in vitro* prior to target cell or tissue introduction by various means. The C9-sgRNA component is very sensitive to modifications in respective to functionality to assemble with C9 into an enzymatically active RNP complex. For example, C9-sgRNA sequences embedded in a RNAP-II transcript do not yield a functional RNP complex due to its capping and polyadenylation. Similarly, multifunctional RNAs harboring other sequence elements in addition to the C9-sgRNA sequence, or RNAP-III transcripts harboring multiple C9-sgRNA sequences in tandem that would allow multiplex applications, do not form functional RNP complexes.

The latter C9-sgRNA limitations are addressed by a modified CRISPR/Cas9 genome editing system (Csy4-2A-Cas9 (C4-C9), Fig. 1B) that employs the use of a second enzyme, Csy4 (C4) ribonuclease, and additional C4-specific sgRNA scaffold region (sR) ribonucleotide sequences (Fig. 1B; Fig. 2A) (Tsai et al. 2014). The ORFs of both nucleases, C4 and C9, are fused in frame and connected by a 2A-peptide coding sequence. This bicistronic-like transcript results in translation of separate C4- and C9-nucleases, mediated by a ribosomal skipping mechanism. Furthermore, the system employs a modified chimeric sgRNA (pre-C4/C9-sgRNA) composed of C9-specific sgRNA sequences flanked on both sides by C4-specific sR sequences (Fig. 1B; Fig. 2B). This chimeric design allows RNAP-II mediated expression of pre-C4/C9-sgRNAs with one or multiple embedded C9-sgRNAs. The embedded C9-sgRNAs are processed by C4 through recognition of C4-sR and subsequent cleavage at their 3' end into one or multiple mature C4/C9-sgRNAs. These consist of a 5' C9-sgRNA element with 3' extension encoding a C4-sR element and can be recognized by C9 and assembled into a functional C9-RNP enabling dsDNA editing (Fig. 1B).

The inventors have developed a new combinuclease CRISPR genome editing system which employs various chimeric C4/C9-enzymes, having C4- and C9-proteins covalently linked by a peptide linker (Csy4-L-Cas9 (C4/C9), Fig. 1C, Fig. 2A). Thereby C4-mediated ribonuclease and C9-mediated deoxyribonuclease catalytic activities are combined in a single multi-domain protein. These C4/C9-combinucleases can be expressed from single transcripts harboring coding sequences for C4, C9 and peptide linkers fused in frame in various configurations (Fig. 2A; C4C9, C9C4). When combined with pre-C4/C9-sgRNA-transcripts or -RNAs, generated by different synthesis approaches (e.g. RNAP-II or RNAP-III driven transcription, in *vitro* transcription, chemical synthesis), these can be recognized and bound by the C4-domain of the respective C4/C9-combinuclease and processed into the mature C4/C9-sgRNAs by its C4-ribonuclease activity. Alternatively, if mature C4/C9-sgRNAs are provided by other means, the C4-domain of the respective C4/C9-combinuclease can recognize and bind the mature C4/C9-sgRNAs. C4 has the ability to stay bound with high affinity to its C4-sR even after 3' nuclease cleavage. As a consequence, processing of pre-C4/C9-sgRNA by the respective C4/C9-combinuclease results in a very high local concentration of mature C4/C9-sgRNA close to the C9-domain of the C4/C9-combinuclease (Fig. 1C), which is not the case if C4- and C9-nucleases are translated as separate proteins as is the case for the C4-C9 system (Fig. 1B). Thereby the C4/C9-combinuclease system results in a much more efficient formation of functional C4/C9-RNP complexes, having the C9-element of the C4/C9-sgRNA bound by C9-nuclease, than the C4-C9 system. Thereby a more efficient C9-RNP-mediated genome editing is enabled, in particular at conditions of low overall concentrations of both components.

In Fig. 1, the functional RNP complex is designated "active end product".

### Nuclease-encoding and sgRNA-encoding expression constructs and further constructs used

Fig. 2A shows schematic representations of the TraFo vector genome-encoding expression constructs comprising ORFs for various CRISPR nucleases. The C9 expression construct has a codon-optimized version of a C9 ORF encoding the C9 nuclease with N-terminal Flag and SV40 nuclear localization signal (NLS) tag (NLS1) and C-terminal nucleoplasmin NLS tag (NLS2) (Fig. 2A, C9). The C9-GFP expression construct additionally has an EGFP ORF fused in frame and connected by a 2A-peptide coding sequence, resulting in translation of separate C9 (Flag-NLS1-Cas9-NLS2) and EGFP proteins, mediated by a ribosomal skipping mechanism (Fig. 2A, C9-GFP). In the C4-C9 expression construct, the ORFs of both nucleases, C4 and C9, are fused in frame and connected by a 2A-peptide coding sequence (Fig. 2A, C4-C9). This bicistronic-like transcript results in translation of separate C4- and C9-nucleases, mediated by a ribosomal skipping mechanism. The 2A-peptide coding sequence used encodes a T2A peptide that has the following amino acid sequence: EGRGSLLTCGDVEENPGP (SEQ ID NO.: 33). The constructs C9, C9-GFP and C4-C9 are, at least in principle, known from the prior art.

A first generation (v1) of RNAP-II driven C4/C9-combinuclease expression constructs was generated with different N- to C-terminal fusions of C4- and C9-ORFs connected by a sequence encoding a (G₄S₁)₃ peptide linker (Fig. 2A, C4C9v1, C9C4v1). The v1 C4/C9-combinucleases also had one out of two nuclear localization signals placed in the peptide linker sequence covalently connecting C4- and C9-nucleases.

A second and third generation (v2, v3) of RNAP-II driven C4/C9-combinuclease expression constructs was generated. Unlike the first generation (v1), they encoded peptide linkers that were largely composed of different numbers of G₄S₁ peptide modules (v2, v2-1: seven; v3: three) and lacked NLS peptide sequences. The latter were relocated from the peptide linker to either the N- or C-terminus of the C4/C9-combinuclease. Furthermore, these C4/C9-combinucleases had the original humanized C4-ORF (hCsy4) of the first generation (v1) replaced by a C4-ORF (coCsy4), which had been expression-optimized by a different algorithm. To allow the examination of the influence of C4-ORF optimization alone on any phenotypes observed, the inventors generated variants (v2-1) with the original humanized C4-ORF for the respective C4/C9-combinuclease variants with the longest peptide linker (v2).

Further, an expression construct for a C4/C9-combinuclease that has an additional functional protein domain was generated (C9C4v2 BE1). The C9C4v2 BE1 combinuclease will be described further below.

Abbreviations used in Fig. 2A: CMV: cytomegalovirus enhancer - promoter; SD: splice donor; SA: splice acceptor; hCsy4: humanized Csy4 ORF; coCsy4: expression-optimized Csy4 ORF; coCas9: expression-optimized Cas9 ORF; coCas9 D10A: expression-optimized Cas9 D10A nickase ORF; T2A: thosea asigna virus 2A peptide; NLS: Nuclear localization signal peptide sequence; NLS1: SV40 NLS; NLS2: nucleoplasmin NLS; NLS3: SV40 bipartite NLS; WPRO: Optimized Woodchuck hepatitis virus posttranscriptional regulatory sequence element; pA: Polyadenylation signal sequence; F: Flag tag peptide sequence; GS: Glycine-serine peptide linker; TadA-8e: *Escherichia coli* tRNA adenosine deaminase variant 8e.

The complete linker sequences of the combinucleases are indicated in Table 3A.

**Table 3A**

| Construct | Linker sequence (N-terminal to C-terminal) (*) |
|---|---|
| C4C9v1 | FEGGGGSGGGGSGGGGSARMAPKKKRKVGIHGVPAA (SEQ ID NO.: 34) |
| C9C4v1 | KRPAATKKAGQAKKKKASGSGLQFEGGGGSGGGGSGGGGS AS (SEQ ID NO.: 35) |
| C4C9v2, | FEGGGGSGGGGSGGGGSGGGGSGGGGSGGGGSGGGGSG T (SEQ ID NO.: 36) |
| C4C9v2-1, | |
| C4C9v3 | FEGGGGSGGGGSGGGGSGT (SEQ ID NO.: 37) |
| C9C4v2, | NCLGGGGSGGGGSGGGGSGGGGSGGGGSGGGGSGGGGS TG (SEQ ID NO.: 38) |
| C9C4v2-1 | |
| C9C4v2 BE1 | |
| C9C4v3 | NCLGGGGSGGGGSGGGGSTG (SEQ ID NO.: 39) |

| | |
|---|---|
| (*) Amino acids of glycine-serine linker motifs (G4S motifs) are underlined once. NLS amino acid sequences are underlined twice. Amino acids that are not underlined are miscellaneous amino acids due to recognition sequences of restriction enzymes that were used for cloning the DNA molecules encoding the combinucleases. | |

The NLS sequences used are indicated in Table 3B.

**Table 3B**

| NLS(*) | NLS sequence (N-terminal to C-terminal) |
|---|---|
| NLS1 | PKKKRKV (SEQ ID NO.: 8) |
| NLS2 | KRPAATKKAGQAKKKK (SEQ ID NO.: 9) |
| NLS3 | KRTADGSEFESPKKKRKV (SEQ ID NO.: 10) |

| | |
|---|---|
| (*) NLS1: SV40 NLS; NLS2: Nucleoplasmin NLS; NLS3: SV40 bipartite NLS. | |

The SEQ ID NO.s of the amino acid sequences of the C4/C9-combinuclease proteins and the SEQ ID NO.s of the corresponding DNA sequences encoding the C4/C9-combinuclease proteins are indicated in Table 3C.

**Table 3C**

| C4/C9-combinuclease | SEQ ID NO. of the amino acid sequence of the C4/C9-combinuclease | SEQ ID NO. of the DNA sequence encoding the C4/C9-combinuclease |
|---|---|---|
| C4C9v1 | 51 | 52 |
| C9C4v1 | 53 | 54 |
| C4C9v2 | 55 | 56 |
| C4C9v3 | 57 | 58 |
| C4C9v2-1 | 59 | 60 |
| C9C4v2 | 61 | 62 |
| C9C4v3 | 63 | 64 |
| C9C4v2-1 | 65 | 66 |
| C9C4v2 BE1 | 67 | 68 |

The DNA sequence encoding C9, resulting in the translation of C9 nuclease protein, is provided as SEQ ID NO.: 69.

The DNA sequence encoding C9-GFP, resulting in the translation of separate C9 nuclease and EGFP proteins, is provided as SEQ ID NO.: 70.

The DNA sequence encoding C4-C9, resulting in the translation of separate C4 nuclease and C9 nuclease proteins, is provided as SEQ ID NO.: 71.

Fig. 2B shows schematic representations of the TraFo expression constructs encoding egfp-specific pre-C4/C9-sgRNA mRNAs (TraFo Red-pre-C4/C9-sgRNA) or U6 promoter-driven RNA polymerase III Cas9 sgRNAs (TraFo C9-sgRNA), or IDPV vector genomes comprising U6 promoter-driven Cas9-sgRNAs (IDPV C9-sgRNA + Red) expression cassettes. The primary transcripts derived from the individual expression cassettes, representing the mature functional C9-sgRNAs or the pre-C4/C9-sgRNAs, as indicated, are also shown. Furthermore, the mature functional C4/C9-sgRNAs, derived from the pre-C4/C4-sgRNAs by Csy4-mediated processing, are displayed. The constructs IDPV C9-sgRNA + Red and Trafo C9-sgRNA are, at least in principle, known from the prior art.

Additional abbreviations used in Fig. 2B: R: long terminal repeat sequence; U5: long terminal repeat unique 5' sequence; ΔU3: enhancer-promoter deleted LTR unique 3' region; Psi: retroviral packaging signal sequence; U6P: RNAP-III U6 promoter; U6T: U6 terminator; SFFV U3: Spleen focus forming virus U3 promoter; DsRed Ex2: DsRed Express 2 ORF; ^{©}: cap structure; Aₙ: poly A tail; C9 sgRNA: Cas9 single guide RNA; C4 sR: Csy4 scaffold region; RNAP-III: RNA polymerase III; RNAP-II: RNA polymerase III.

The SEQ ID NO.s of the C9-sgRNAs and pre-C4/C9-sgRNAs and the SEQ ID NO.s of the corresponding DNA sequences encoding the C9-sgRNAs and the pre-C4/C9-sgRNAs are indicated in Table 3D.

**Table 3D**

| C9-sgRNA or pre-C4/C9-sgRNA | SEQ ID NO. of the C9-sgRNA or pre-C4/C9-sgRNA sequence | SEQ ID NO. of the DNA sequence encoding the C9-sgRNA or pre-C4/C9-sgRNA |
|---|---|---|
| IDPV C9-gfp-148 (C9-gfp-148 of "IDPV C9-sgRNA + Red" construct) | 72 | 73 |
| pre-C4/C9-gfp+174-112 | 74 | 75 |
| pre-C4/C9-gfp-148 | 76 | 77 |
| pre-C4/C9-gfp-197 | 78 | 79 |
| pre-C4/C9-stuffer | 80 | 81 |
| pre-C4/C9-gfp c.187 A>G | 82 | 83 |
| TraFo C9-gfp-148 (C9-gfp-148 of "TraFo C9-sgRNA" construct) | 72 | 73 |

Fig. 2C shows schematic representations of the FV packaging constructs used for the generation of TraFo or IDPV vector particles. The constructs are, at least in principle, known from the prior art. Standard integration-deficient (IDPV) PFV vector particles are composed of PFV Gag (PG), FV Env (PE, SE), and integrase-deficient PFV Pol (PP ilN) proteins encapsidating regular, reverse transcription-competent PFV RNA vector genomes, which comprise non-coding PFV packaging (P) RNA genome sequences and harbor sgRNA expression cassettes (IDPV C9-sgRNA + Red). TraFo FV vector particles are composed of FV Gag (PG, SFVcja, SFVssc, SFVaax, SFVcae, SFVmcy, SFVppy, SFVpsc), FV Env protein (PE, SE) only and encapsidate non-viral mRNAs, which encode nucleases (C9, C9-GFP, C4-C9, C4C9, C9C4), or sgRNA (e.g. U6-gfp-148), pre-sgRNA (e.g. pre-C4/C9-gfp-148) transcripts, which encode a sgRNA with the specific target sequence of choice. For pseudotyping of TraFo vector particles with non-FV glycoproteins (e.g. VSV-G, VSV), a PFV Gag variant (PG M6) with an N-terminal membrane targeting domain tag (HIV-1 Gag matrix [MA] domain) was used.

Additional abbreviations used in Fig. 2C: PG: PFV Gag; PG iNAB: nucleic acid binding defective PFV Gag; PG M6: PFV Gag with N-terminal HIV-1 Gag matrix domain fusion; PG NFK: PFV Gag with N-terminal FK heterodimerization domain fusion; H1G-MA-CFR: HIV-1 Gag matrix domain with C-terminal FR heterodimerization domain fusion; SFVxxx: Gag of various Simian Foamy Virus species; PE: PFV Env; SE: SFVmac/SFVmcy Env; VSV: vesicular stomatitis virus glycoprotein (VSV-G); PP ilN: integrase-inactive PFV Pol.

Fig. 2D shows schematic representations of the lentiviral (LV) transfer vector and retroviral (RV) Gag/Pol packaging constructs used. The constructs are, at least in principle, known from the prior art. LV: lentiviral human immunodeficiency virus 1 (HIV-1); RV: retroviral; MLV: Murine leukemia virus; ilN: enzymatically inactive integrase variant.

Additional abbreviations used in Fig. 2D: hUBC1: human UBC1 promoter; RSV: Rous sarcoma virus promoter; shRNA: short hairpin RNA; PPT: poly purine tract; mCer: mCerulean; Puro: Puromycin resistance; WPRE: Woodchuck hepatitis virus posttranscriptional regulatory element.

### Reporter gene inactivation assay

In many experiments of this study, a cell-based, flow cytometric reporter gene inactivation assay was used (Fig. 3). The assay allows examination and quantitative comparison of genome editing capacities of different CRISPR systems. The assay employs an *egfp* reporter gene encoding an enhanced green fluorescent protein (EGFP) and is also referred to as "GFP KO assay" (green fluorescent protein knockout assay). The *egfp* reporter gene acts as target gene. The assay uses a human osteosarcoma cell line (U2OS*gfp*) comprising a single, chromosomally integrated, lentiviral vector genome. The stably integrated sequence consists of a cytomegalovirus (CMV) promoter-driven reporter gene expression cassette encoding a destabilized enhanced green fluorescent protein (dsEGFP) with reduced half-life in comparison to wildtype GFP. Wild-type U2OS*gfp* cell populations are >95% GFP-positive (Fig. 3B). Co-expression of a nuclease, in particular a Cas9 nuclease, and an egfp-specific sgRNA, in particular an egfp-specific Cas9-sgRNA, in U2OS*gfp* cells, which act as target cells, results in *egfp* gene inactivation as a consequence of insertion/deletion mutations (InDel) arising from nuclease/sgRNA-ribonucleoprotein complex-induced chromosomal double-strand breaks and subsequent "non-homologous end-joining" (NHEJ) DNA repair (Fig. 3A). The *egfp* gene inactivation can be easily examined by flow cytometric analysis of the cells (Fig. 3B). The assay is used to quantify the efficiency of reporter gene inactivation after transduction of the cells with different foamy virus (FV)-derived CRISPR vector systems.

When CRISPR components are delivered into the target cells through viral transduction, two scenarios are possible: transferring the nuclease component and the sgRNA component in the same viral vector particle ("One-Virus" vector system, "One-Virus") or in separate viral vector particles ("Two-Virus" vector system, "Two-Virus") (Fig. 3A). For example, a prototype foamy virus (PFV)-derived "Two-Virus" "CRISPR 1.0" vector system consists of Cas9 nuclease encoding PFV RNA transfer vector particles (TraFo C9) and integration-deficient PFV (IDPV) sgRNA-encoding vector particles (IDPV C9-sgRNA) produced by transient transfection of HEK293T packaging cells (Lindel et al. 2019) (Fig. 3D). A single co-transduction of U2OS*gfp* cells with a mixture of these two types of vector particles results in *egfp* gene inactivation in more than 85% of the cells (Fig. 3B), as monitored by flow cytometric analysis of transduced target cells as early as 3 days post infection (dpi) (Fig. 3C). Generally, U2OS*gfp* target cells are transduced by incubation with viral supernatants and GFP fluorescence is determined by flow cytometry analysis at 3 and/or 7 days post infection (dpi) (Fig. 3C).

Fig. 3D shows the composition of different FV-derived CRISPR vector systems. There are two generations of vector systems, namely the "CRISPR 1.0" vector system and the "CRISPR 2.0" vector system. The "CRISPR 1.0" vector system is a "Two-Virus" vector system composed of separate TraFo nuclease-encoding virus vector particles and IDPV sgRNA-encoding virus vector particles. The FV "CRISPR 2.0" vector system comes in two variants, namely the "Two-Virus" vector system and the "One-Virus" vector system. The "Two-Virus" vector system is composed of separate TraFo combinuclease-encoding virus vector particles and TraFo pre-sgRNA-containing, in particular pre-C4/C9-sgRNA-containing, virus vector particles. The "One-Virus" vector system consists of a single TraFo virus vector particle harboring both combinuclease and (pre-)sgRNA components. The two components may be present as a ribonucleoprotein (RNP) complex or they may be provided in the form of nucleic acids, the nucleic acids either encoding the combinuclease or being the pre-sgRNA or mature sgRNA (Fig. 3A).

The experimental results will now be described in detail.

### Gene inactivation by "Two-Virus" CRISPR vector systems

In a first set of experiments, the gene editing efficiency of different FV "Two-Virus" CRISPR/Cas9 or CRISPR/Cas9-Cas4 vector systems was compared (Fig. 4). Prototype FV (PFV) vector particles were generated by transient transfection of different combinations of transfer vectors (Fig. 2A, B) and packaging plasmids (Fig. 2C) into HEK293T packaging cells. In case of TraFo vector supernatants, a PFV Gag (pcoPG4 or pcoPG4 M6) and Env (PFV Env: pcoPE; SFVmac Env: pcoSE; VSV-G: pcziVSV-G) encoding packaging plasmid were cotransfected with the respective nuclease-encoding or sgRNA-encoding transfer vector of choice, which express transcripts having no retroviral genomic sequences to be encapsidated. For integration-deficient PFV (IDPV) sgRNA-encoding vector supernatants, a PFV Gag (pcoPG4), a FV Env (PFV Env: pcoPE; SFVmac Env: pcoSE) and a PFV Pol packaging plasmid encoding an enzymatically inactive integrase (pcoPP3) were cotransfected with the respective sgRNA-encoding transfer vector of choice. In this case, the transfer vector expresses transcripts to be encapsidated in vector particles that contain cis-active FV sequences essential for packaging and reverse transcription.

In this first set of experiments, two types of sgRNA vector particles were employed. The first type were IDPV RNA polymerase (RNAP)-III C9-gfp-148 vector particles that harbored an RNAP-III U6 promoter driven expression cassette for a C9-sgRNA specifically guiding Cas9 to the egfp-ORF at position 148 (gfp-148) and an RNAP-II driven reporter gene expression cassette for a DsRed Express 2 (DsRed Ex2) protein - both embedded in a standard PFV transfer vector backbone (Fig. 2B). Upon packaging of the PFV vector RNA genome (vgRNA), it will be reverse transcribed by the co-encapsidated integrase-deficient PFV Pol protein into double-stranded vector DNA (vgDNA). Due to the enzymatically inactive integrase of the PFV Pol protein employed to deliver vgDNA into target cells, the vgDNA cannot be actively inserted into target cell chromosomes. The vgDNA is largely present transiently in episomal form in the nucleus of transduced target cells. This results in a predominant transient nuclear expression of RNAP-III driven sgRNA and/or RNAP-II driven reporter gene transcripts. However, at low frequency a permanent sgRNA and/or reporter gene expression is possible due to non-viral mediated vgDNA chromosome integration as a consequence of non-homologous recombination.

The second type were TraFo RNAP-II pre-C4/C9-gfp+174-112, TraFo RNAP-II pre-C4/C9-gfp-148, or TraFo RNAP-II pre-C4/C9-stuffer vector particles that harbored a RNAP-II derived non-viral transfer vgRNA representing a pre-C4/C9-sgRNA that lacked any retroviral sequences (Fig. 2B). They were composed of a *DsRed Ex2* reporter gene with downstream chimeric sgRNA sequences, consisting of either one or two C9-sgRNA elements specific for either the egfp-ORF (+174-112; -148) or having no target sequence specificity (stuffer). All C9-sgRNA sequences were flanked by upstream and downstream C4-sR elements. In addition, the 3' terminus of all vgRNA sequences had an optimized woodchuck hepatitis virus posttranscriptional regulatory sequence element (WPRO).

For the transduction of target cells, the sgRNA-encoding or -containing vector supernatants were each combined with four different types of TraFo nuclease-encoding vector supernatants (Fig. 2A) at a 1:1 volume ratio if not mentioned otherwise. The first type were TraFo C9-GFP particles that contained non-viral mRNAs harboring a Cas9-T2A-GFP reporter ORF and resulting in co-expression of separate Cas9 and GFP proteins. The second type were TraFo C4-C9 particles that harbored mRNAs harboring a Csy4-T2A-Cas9-ORF resulting in expression of separate C4- and C9-nucleases. The third and fourth type were TraFo C4C9v1 particles and TraFo C9C4v1 particles, respectively, that harbored mRNAs encoding for either C4C9- or C9C4-combinucleases with C4- and C9-nucleases fused and arranged in different N- to C-terminal orientation and in each case covalently linked by glycine-serine-comprising peptide linkers as shown in Fig. 2A.

The quantitative analysis of the gene inactivation efficiencies of the different "Two-Virus" vector system variations using the GFP knockout (KO) assay revealed significant differences depending on the combination of nuclease-encoding and sgRNA-encoding vector supernatant used (Fig. 4A). U2OS*gfp* cells were incubated with 1:1 ratios of TraFo vector supernatant encoding different nucleases in combination with supernatants of either TraFo vectors containing different RNAP-II derived pre-C4/C9-sgRNA transcripts or IDPV vectors encoding an RNAP-III promoter driven C9-gfp-148 sgRNA expression cassette, followed by flow cytometric GFP KO quantification 7 days post infection (dpi). Importantly, for the following comparisons, it has to be kept in mind that IDPV-derived RNAP-III C9-sgRNA transcripts are de novo expressed in the target cell nucleus, whereas TraFo-derived RNAP-II pre-C4/C9-sgRNA transcripts are introduced by viral transduction and not amplified in the target cell. TraFo C9 vector only enabled highly efficient GFP KO in combination with the IDPV RNAP-III C9-gfp-148 sgRNA vector (Fig. 4A, set 1), whereas it was non-functional in combination with TraFo RNAP-II pre-C4/C9-gfp+174-112 vector (Fig. 4A, set 2). This is in line with the suspected dependence of classical Cas9 on mature C9-sgRNAs and its inability to functionally use C9-sgRNAs embedded in RNAP-II transcripts. TraFo C4-C9 vector was slightly less efficient in combination with IDPV RNAP-III C9-gfp-148 sgRNA vector (Fig. 4A, set 1), but showed a weak but clearly measurable activity in combination with TraFo RNAP-II pre-C4/C9-gfp+174-112 vector (Fig. 4A, set 2). This indicates that the combination of separate C4- and C9-nucleases still allows quite efficient use of RNAP-III C9-sgRNA transcripts and to a much lower level also RNAP-II pre-C4/C9-sgRNA transcripts for gene editing. TraFo C4C9v1 vectors displayed a further reduced GFP KO efficiency in combination with IDPV RNAP-III C9-gfp-148 sgRNA vector (Fig. 4A, set 1), but a strongly increased efficiency when combined with TraFo RNAP-II pre-C4/C9-gfp+174-112 vector (Fig. 4A, set 2). This demonstrates that C4C9v1-combinuclease can efficiently use pre-C4/C9-sgRNA transcripts for gene editing but at the expense of less efficient utilization of C9-sgRNA transcripts expressed in the nucleus compared with C9-GFP- or C4-C9-nucleases.

TraFo C9C4v1 vectors were also tested in this analysis and also showed GFP-KO. The efficiency of the GFP-KO obtained with TraFo C9C4v1 vectors was lower than the GFP-KO efficiency obtained with TraFo C4C9v1 vectors, i.e. the C4C9v1-combinuclease was more effective than the C9C4v1-combinuclease (data not shown).

The analysis further demonstrated that the TraFo C4C9v1 combinuclease supernatant enabled comparable gene editing efficiencies in combination with TraFo supernatant containing different RNAP-II-derived pre-C4/C9-sgRNAs specific for the egfp-ORF. Their GFP KO efficiencies were at similar or slightly higher levels as compared to the respective TraFo nuclease combination with IDPV RNAP-III supernatant encoding an egfp-specific C9-sgRNA (Fig. 4A, compare set 1 to set 2 and 3). No gene editing was observed using TraFo supernatants harboring non-egfp-specific pre-C4/C9-sgRNA RNAP-II transcripts (Fig. 4A, set 4).

Next, the effect of the relative ratios of the TraFo supernatant encoding C4C9v1-combinuclease to TraFo RNAP-II pre-C4/C9-gfp+174-112 supernatants on the gene editing efficiency was examined (Fig. 4B). This was done by adding to U2OS*gfp* cells constant amounts of plain vector supernatants of one component in combination with identical amounts of plain vector supernatant or 10-fold dilutions of the other component. Thereby, the GFP KO determination was conducted 7 dpi by flow cytometry. This analysis revealed that a 1:1 ratio, as used in the previous experiments (Fig. 4A), yielded the highest GFP KO levels (Fig. 4B). The same was found for the C4C9v1-combinuclease (data not shown). Furthermore, lowering the amount of pre-C4/C9-sgRNA vector supernatant relative to combinuclease supernatant led to stronger decrease in the gene editing efficiency than vice versa. This led to the conclusion that the TraFo sgRNA component is more limiting for the gene editing efficiency than the TraFo nuclease component.

### Gene inactivation by "One-Virus" CRISPR vector systems

After successful development of a fully transient "Two-Virus" TraFo "CRISPR 2.0" vector system (Fig. 4), the inventors explored whether establishment of a "One-Virus" variant thereof might be possible (Fig. 3A, 3D). Therefore, "One-Virus" TraFo vector supernatants were produced by transient co-transfection of C4/C9-combinuclease-encoding transfer vectors and pre-C4/C9-sgRNA-encoding transfer vectors together with various packaging plasmid combinations in HEK293T packaging cells (Fig. 2; Fig. 5). The transfer vectors are also referred to as expression vectors. Different amounts of C4/C9-combinuclease-encoding transfer vectors and different amounts of pre-C4/C9-sgRNA-encoding transfer vectors were used. As pre-C4/C9-sgRNA, pre-C4/C9-sgRNA gfp-148 (pre-gfp-148) (Fig. 5A, 5B) or pre-C4/C9-sgRNA gfp-197 (pre-gfp-197) (Fig. 5B) were used. FV packaging constructs (packaging plasmids) encoding wildtype PFV Gag (wt) or a nucleic acid binding deficient (iNAB) mutant thereof as well as wildtype SFVmac Env (wt) were used in fixed amounts. Replacement of one or both FV packaging constructs by non-coding plasmids is indicated by "-". For comparison, "Two-Virus" TraFo vector supernatants encoding the same combinucleases or containing pre-C4C9-sgRNA gfp-148 RNAP-II transcripts were produced in parallel and plain supernatants used at a 1:1 ratio for transduction of U2OS*gfp* cells. GFP inactivation in transduced U2OS*gfp* cells was determined by flow cytometry 7 days post infection (dpi).

Strikingly, transduction of U2OS*gfp* cells and flow cytometric analysis of GFP KO 7 dpi demonstrated a significant gene editing capacity for "One-Virus" TraFo "CRISPR 2.0" supernatants that were generated by co-transfection of identical amounts of either C4/C9-combinuclease-encoding and pre-C4/C9-sgRNA gfp-148-encoding transfer vector (Fig. 5A, set 2). In contrast, employing the C4-C9 nuclease-encoding transfer vector instead only yielded very low GFP KO levels, hardly above background. When comparing the gene editing efficiency of respective "One-Virus" and "Two-Virus" TraFo vector supernatants, the gene editing efficiency of C9C4v1-combinuclease containing vector supernatants was similar, whereas that of "One-Virus" TraFo C4C9v1-combinuclease supernatants was about 2-fold lower compared to respective "Two-Virus" TraFo vector transductions (Fig. 5A, compare set 2 and set 7). Remarkably, the gene editing efficiency of "One-Virus" TraFo vector supernatants that resulted in the delivery of either C4C9v1- or C9C4v1-combinucleases could be strongly increased by reducing the relative ratio of C4/C9-combinuclease transfer vector amounts to pre-C4/C9-sgRNA transfer vector amounts during vector supernatant production (Fig. 5A, set 1, set 2 and set 3; Fig. 5B). The highest efficiency was obtained using a 1:5 ratio of C4/C9-combinuclease transfer vector to pre-C4/C9-sgRNA transfer vector and this effect was independent of the type of egfp-specific pre-C4/C9-sgRNA variant used (Fig. 5A, 5B). The results thus show that gene inactivation by "One-Virus" CRISPR vector systems is influenced in its efficiency by the ratio of nuclease to pre-sgRNA encoding expression constructs during vector production (Fig. 5A, 5B). In case of using the C4C9v1-combinuclease, gene editing efficiencies of "One-Virus" and "Two-Virus" vector supernatants were comparable, whereas that of C9C4v1-combinuclease expressing "One-Virus" supernatants was superior to respective "Two-Virus" supernatants (Fig. 5A, set 3 and set 7). The results further show that both C4/C9-combinucleases have a similar efficiency when using the "One-Virus" TraFo system (Fig. 5A, set 2 and set 3; Fig. 5B, set 2, set 3, set 5 and set 6).

The inventors also explored the requirements and features of the TraFo packaging components for "One-Virus" TraFo vector functionality and efficiency by comparing the gene editing efficiency of vector supernatants generated using different combinations of packaging plasmid (Fig. 2C) at optimized combinuclease- to pre-C4/C9-sgRNA transfer vector ratios (Fig. 5A, set 3 to set 6). The analysis demonstrated that functional "One-Virus" TraFo "CRISPR 2.0" vector particles are dependent on the nucleic acid binding activity of the FV Gag protein, as use of a PFV Gag packaging construct with inactivated nucleic acid binding domain (iNAB) (Hamann et al. 2014) completely abolished the gene editing capacity (Fig. 5A, set 4). Similarly, capsidless PFV subviral particles (SVPs), generated by using only Env- and omitting PFV Gag packaging plasmid during virus production, did not show any gene editing activity (Fig. 5A, set 5). This was also the case when employing control supernatants of HEK293T packaging cells co-transfected with only combinuclease- and pre-C4/C9-sgRNA and omitting all packaging constructs (Fig. 5A, set 6). The latter demonstrates that any gene editing activity observed by transduction with any other vector supernatant (Fig. 5A, set 1 to set 3, set 7) is not due to plasmid transfer of any transfer vector construct used for virus production into reporter cells, which has also been previously demonstrated for TraFo vector particles (Lindel et al. 2019).

### Efficient Genome Editing by "One-Virus" TraFo "CRISPR 2.0" vector systems

The inventors examined whether the remarkably high gene editing capacity of "One-Virus" TraFo "CRISPR 2.0" vector supernatants was due to and dependent on the covalent linkage of C4- and C9-nucleases in the C4C9v1- or C9C4v1-combinuclease transfer vector constructs. Furthermore, the inventors explored the "One-Virus" TraFo "CRISPR 2.0" gene editing capacity with respect to the pre-C4/C9-sgRNA structure used. For that purpose, "One-Virus" TraFo supernatants were generated using different nuclease transfer vectors, encoding either C4C9v1- or C9C4v1-combinucleases or bicistronic-like expressed separate C4- and C9-nucleases (C4-C9), in combination with different egfp-specific sgRNA transfer vectors at a 2:10 ratio. The different egfp-specific sgRNA transfer vectors expressed either a RNAP-II-driven pre-C4/C9-sgRNA (RNAP-II pre-C4/C9) or a RNAP-III-driven C9-sgRNA (RNAP-III C9) (Fig. 2A, B). Subsequently, the gene editing activity of the "One-Virus" TraFo supernatants was determined using the GFP KO assay (Fig. 6). U2OS*gfp* target cells were incubated with 1 ml of either plain (10⁰),10-fold (10⁻¹) or 100-fold (10⁻²) diluted "One-Virus" TraFo supernatant. GFP inactivation was determined by flow cytometry 7 days post infection (dpi). The results demonstrated that generation of functional "One-Virus" TraFo "CRISPR 2.0" vector supernatants is strictly dependent on the use of a pre-C4/C9-sgRNA design, and the gene editing efficiency achieved using C4/C9-combinucleases (C4C9v1, C9C4v1) is at least 100-fold higher than expressing separate C4- and C9-nucleases (C4-C9) (Fig. 6, compare set 1 to set 2 and set 3). In more detail, comparing the GFP KO value of C4-C9 in the 10° dilution of approximately 15% with that of the 10² dilution of C4C9v1 or C9C4v1 with 15-20% GFP KO each shows that similar KO efficiencies are achieved with 100-times less C4C9v1 or C9C4v1 supernatant than with C4-C9 supernatant. Therefore, the C4C9v1 and C9C4v1 variants are 100-times more active than C4-C9. It is noted that at KO values of 80-90%, the system is in saturation, i.e. the target cells are showered with "One-Virus" particles, masking possible differences in the activity of the different RNP complexes.

Thus, a fully transient "One-Virus" TraFo "CRISPR 2.0" vector systems is possible, enabling gene editing efficiencies at similar or even higher levels than respective "Two-Virus" vector systems. The "One-Virus" TraFo "CRISPR 2.0" vector system gene editing capacity depends on covalent linkage of C4- and C9-nucleases in a C4/C9-combinuclease design, which must be combined with a sgRNA transcript of the pre-C4/C9-sgRNA design. Furthermore, its efficiency could be optimized by using high relative ratios of pre-C4/C9-sgRNA to C4/C9-combinuclease transfer vector during viral vector production, which indicates the pre-C4/C9-sgRNA component is the limiting functional component for this type of transfer.

### Comparison of genome editing efficiency of "One-Virus" vector systems based on different retrovirus and foamy virus species

The inventors examined whether the ability to produce "One-Virus" TraFo "CRISPR 2.0" vector supernatants was restricted to the PFV Gag packaging constructs used in all experiments so far or whether it is a general feature of FV Gag or even other retrovirus Gag proteins (Fig. 2C). Therefore, "One-Virus" TraFo "CRISPR 2.0" supernatants were produced by transient transfection of HEK293T packaging cells using C4C9v1- or C9C4v1-combinuclease transfer vector together with egfp-specific pre-C4/C9-sgRNA-gfp-148 in combination with PFV Env and different Gag packaging constructs, and their gene editing activity determined using the GFP KO assay using U2OS*gfp* reporter cells (Fig. 7). This analysis showed that packaging construct encoding different primate FV Gag proteins readily supported generation of functional "One-Virus" TraFo "CRISPR 2.0" vector particles (Fig. 7A). The FV Gag proteins tested included the Gag proteins of prototype foamy virus (PFV, SFVpsc), Bornean orangutan simian foamy virus (SFVora, SFVppy), Taiwanese macaque simian foamy virus (SFVmac, SFVmcy), Grivet simian foamy virus (SFVagm; SFVcae), Spider monkey simian foamy virus (SFVspm, SFVaxx), Squirrel monkey simian foamy virus (SFVsqu, SFVssc), or White-tufted-ear marmoset simian foamy virus (SFVmar, SFVcja). In contrast, neither murine leukemia virus (MLV) Gag nor human immunodeficiency virus type 1 (HIV-1) Gag-expressing packaging constructs did yield vector particles with any gene editing activity, whereas PFV Gag-expressing packaging constructs readily did so using conditions of identical amounts of C4/C9-combinuclease, pre-C4/C9-sgRNA transfer vectors and respective packaging constructs (Fig. 7B). Thus, the ability to enable "One-Virus" TraFo "CRISPR 2.0" vector supernatants is a feature of Gag proteins of many if not all FV species, but not of gamma-retroviral or lentiviral Gag proteins.

The dependence of the "One-Virus" TraFo "CRISPR 2.0" vector particle gene editing capacity on the nucleic acid binding capacity of their Gag protein (Fig. 5A) suggested that nucleic acid - Gag protein interactions are essential for functional, simultaneous transfer of CRISPR components in target cells. However, this raises the question in which format they are packaged and transferred by "One-Virus" TraFo particles? Due to the co-transfection of C4/C9-combinuclease encoding plasmids and pre-C4/C9-sgRNAs encoding plasmids in HEK293T packaging cells, both components are co-expressed during vector particle production. This allows for their interaction, pre-C4/C9-sgRNA processing and assembly of a functional C4/C9-combinuclease-C4/C9-sgRNA RNP complex. Consequently, several possibilities are conceivable for the way the CRISPR components are transferred. The Gag-nucleic binding dependence may suggest that the CRISPR components are either encapsidated in the format of C4/C9-combinuclease-encoding mRNA and the pre-C4/C9-sgRNA transcripts, as assembled functional RNP complexes, or a combination of both (Fig. 3A). In the former case, C4/C9-combinuclease encoding mRNAs would be translated in transduced target cells followed by pre-C4/C9-sgRNA recognition, processing and assembly of a functional CRISPR RNP complex. In the second case, functionally assembled CRISPR RNP complex would be transferred into target cells and allow gene editing after its transport into the nucleus. Thereby, the TraFo-mediated transfer would likely be due to an interaction of PFV Gag with the negative charge of the C4/C9-sgRNA as part of the stable RNP complex.

### Functionally active CRISPR RNP complexes are mainly responsible for the "One-Virus" TraFo-mediated gene editing activity

The inventors examined which CRISPR component format is responsible for the majority of gene editing capacity conferred by "One-Virus" TraFo vector particles. To do so, the inventors examined the nucleic acid composition of "One-Virus" and "Two-Virus" TraFo "CRISPR 2.0" vector particles (Fig. 8). TraFo vector particles were produced by transient transfection of HEK293T packaging cells using the appropriate nuclease-, sgRNA-, and packaging plasmid combinations. In case of "One-Virus" TraFo particles a 1:11 ratio of C4/C9-combinuclease to pre-C4/C9-sgRNA transfer vector was used. Since for "Two-Virus" TraFo vector production the maximum possible amount of 12 µg transfer vector is generally employed for both types of TraFo particles, the inventors generated a second set of TraFo combinuclease vectors in the "Two-Virus" setting using only 1 µg C4/C9-combinuclease transfer vector, the same amount as used for production of "One-Virus" TraFo supernatants. As control for non-particle associated nucleic acids, mock supernatants for "One-Virus" and "Two-Virus" TraFo configurations were produced using identical amounts and ratios of C4/C9-combinuclease and/or pre-C4/C9-sgRNA transfer vectors, but omitting the PFV packaging constructs (pack: -). After TraFo vector particle concentration by ultracentrifugation and a DNase I digest, the viral nucleic acids were extracted and the composition of the samples was examined by RT-qPCR using (i) C4/C9-combinuclease-specific (Fig. 8A) or (ii) pre-C4/C9-sgRNA-specific or (iii) total C4/C9-sgRNA (pre-sgRNA + sgRNA)-specific (Fig. 8B) primer-probe sets.

Use of 1 µg C4/C9-combinuclease transfer vector for TraFo production resulted in similar amounts of its transcripts in "One-Virus" or "Two-Virus" particles, which were always 1,000-fold higher as respective mock supernatants produced in the absence of packaging constructs (Fig. 8A, columns 1, 2, 5, 6). Use of 12 µg C4/C9-combinuclease transfer vector instead of 1 µg for generation of "Two-Virus" TraFo nuclease particles resulted in a 10-fold increase in its amounts of transcripts while keeping the signal at 1,000-fold above respective mock controls (Fig. 8A, columns 3, 4).

Thus, "One-Virus" and "Two-Virus" TraFo vector particles encapsidate similar numbers of combinuclease RNAP-II transcripts and "Two-Virus" TraFo RNAP-II transcript encapsidation correlates with the amount of transfer vector transfected during vector production.

The sgRNA composition of TraFo vector particles was analyzed by using two sets of sgRNA-specific primer-probes (Fig. 8B). Set 1 was specific for pre-C4/C9-sgRNA transcripts as its sense primer bound to the overlap of the 3' end of the C9-sgRNA element and 3' located C4-sR element, its antisense primer bound to the 5' part of the WPRO element located 3' of the latter, and the probe spanned the boundary of 3' C4-sR and WPRO elements. Mature C4/C9-sgRNAs lack the WPRO element due to C4-mediated processing of the pre-C4/C9-sgRNA immediately 3' of the C4-sR elements. Therefore this primer-probe set can only use pre-C4/C9-sgRNAs transcripts as template. Primer-probe set 2 bound and in combination with polymerase amplified the C9-sgRNA backbone and therefore detected both precursor and mature C4/C9-sgRNA transcripts. The analysis of nucleic acids extracts from "One-Virus" and "Two-Virus" TraFo particles revealed dramatic differences in the pre- and mature C4/C9-sgRNA composition (Fig. 8B). "Two-Virus" TraFo sgRNA particles mainly contained pre-C4/C9-sgRNA transcripts with the signals of both primer-probe sets being at least 1,000-fold above respective mock controls (Fig. 8B, columns 7, 8). In contrast, "One-Virus" TraFo particles contained 40-fold more mature as pre-C4/C9-sgRNA copies (Fig. 8B, column 1, 2). Here, pre-C4/C9-sgRNA copy numbers of respective mock controls were 500-fold lower whereas mature C4/C9-sgRNA copies were only 19-fold lower than in the corresponding "One-Virus" TraFo samples. This indicates that coexpression of C4/C9-combinuclease and pre-C4/C9-sgRNA in HEK293T packaging cells results in higher non-virus particle associated release of mature C4/C9-sgRNAs than pre-C4/C9-sgRNAs. However, even when taking this into account, levels of mature C4/C9-sgRNAs in "One-Virus" TraFo samples were still 37-fold higher than pre-C4/C9-sgRNAs (Fig. 8B, column 1), indicating that functionally active CRISPR RNPs rather than transferred combinuclease-encoding mRNA and mature C4/C9-sgRNA molecules are responsible for the "One-Virus" TraFo-mediated gene editing activity. This was supported by the analysis of the gene editing efficiency of the respective TraFo supernatants using the GFP KO assay (Fig. 8C). The GFP KO efficiency of "One-Virus" TraFo "CRISPR 2.0" supernatants (Fig. 8C, columns 1 to 3) was more than 5-fold higher than respective "Two-Virus" TraFo "CRISPR 2.0" supernatant combinations (Fig. 8C, columns 4, 5), with "One-Virus" supernatants containing similar or even lower amounts of RNAP-II combinuclease- (Fig. 8A, columns 1, 2, 5, 6) and total C4/C9-sgRNA transcripts (Fig. 8B, columns 1, 2, 7, 8) than "Two-Virus" supernatants.

### Genome editing by "Two-Virus" but not "One-Virus" TraFo "CRISPR 2.0" vector supernatants is inhibited by target cell expression of CRISPR-Cas9 specific shRNAs

The inventors further examined whether target cell translation of transcripts delivered by TraFo-mediated transduction is required for TraFo-mediated gene editing. Therefore, U2OS*gfp* reporter cell variants were generated by lentiviral vector transduction that stably expressed shRNAs specific for the C4-ORF (C4-688) or the C9-sgRNA (sgRNA-947) (Table 4) and a *mCerulean-2A-puromycin* reporter-resistance gene ORF (Fig. 2D). Subsequently, these U2OS*gfp*-shRNA reporter cells and appropriate controls expressing a scrambled (scr. Ctrl.) (Table 4) or no (no) shRNA were transduced with "One-Virus" and "Two-Virus" TraFo "CRISPR 2.0" supernatants at different dilutions and GFP KO was quantified at 7 dpi (Fig. 9). It was found that "Two-Virus" TraFo gene inactivation was reduced by up to 40% relative to the scrambled control by either C4-ORF-specific (C4-688) or C9-sgRNA-specific (sgRNA-947) shRNAs (Fig. 9, set 4 and set 5). In contrast, both specific shRNAs did not inhibit "One-Virus" TraFo gene inactivation or resulted in a maximum reduction of 13% (Fig. 9, set 1 to set 3). Thus, the "One-Virus" TraFo-mediated gene inactivation appears to be mainly due to CRISPR RNP complex transfer as it is largely insensitive to inhibition of de novo C4/C9-combinuclease translation. Furthermore, it is also resistant to C4/C9-sgRNA inactivation suggesting that functionally transferred C4/C9-sgRNAs are protected due to enclosure in the RNP complex.

**Table 4**

| shRNA | shRNA sequence (5'-3') (*) |
|---|---|
| scrambled | GAAACACCGGCAACAAGATGAAGAGCACCAAgttaatattcatagc- TTGGTGCTCTTCATCTTGTTG (SEQ ID NO.: 40) |
| C4-688 | GAAGGAGGCTTTACTTGTTATgttaatattcatagc- ATAACAAGTAAAGCCTCCTTC (SEQ ID NO.: 41) |
| sgRNA-947 | GAGCTAGAAATAGCAAGTTAAgttaatattcatagc- TTAACTTGCTATTTCTAGCTC (SEQ ID NO.: 42) |

| | |
|---|---|
| (*) Uppercase at the beginning indicates target sequence (5'-3'). Lowercase in the middle indicates hairpin sequence. Uppercase at the end indicates antisense sequence (5'-3'). | |

### Improved gene inactivation by chimeric C4/C9 nucleases having optimized peptide linkers

The inventors also intended to further improve the gene editing efficiency of the C4/C9-combinucleases in the "One-Virus" and "Two-Virus" TraFo setting. To do so, the inventors evaluated whether the design and amino acid composition of the peptide linker connecting C4- and C9-nuclease domains as well as the type of expression-optimization of the C4-ORF might influence the C4/C9-combinuclease activity.

Therefore, the inventors generated a 2^{nd} and 3^{rd} generation (v2, v3) of combinuclease transfer vector expression constructs (Fig. 2A). Unlike the 1^{st} generation (v1), they encoded peptide linkers that were largely composed of different numbers of G₄S₁ peptide modules (v2: seven; v3: three) and lacked NLS peptide sequences. The latter were relocated from the peptide linker to either the N- or C-terminus of the C4/C9-combinuclease. Furthermore, these new C4/C9-combinucleases had the original humanized C4-ORF (hCsy4) of the first generation (v1) replaced by a C4-ORF (coCsy4), which had been expression-optimized by a different algorithm. To allow the examination of the influence of C4-ORF optimization alone on any phenotypes observed, the inventors generated variants (v2-1) with the original humanized C4-ORF for the respective new C4/C9-combinuclease variants with the longest peptide linker (v2).

The inventors then produced "One-Virus" and "Two-Virus" TraFo "CRISPR 2.0" supernatants by cotransfecting 1^{st}, 2^{nd} or 3^{rd} generation C4/C9-combinuclease transfer vectors with respective packaging constructs into HEK293T packaging cells, and compared their gene editing efficiency incubating U2OS*gfp* cells with serial dilutions of vector supernatants and flow cytometric analysis of GFP KO at 7 dpi (Fig. 10). For the "Two-Virus" TraFo "CRISPR 2.0" system, only the C4C9v2 and C4C9v2-1 variants led to a significant increase of the respective supernatants gene editing efficiency, at a comparable level (Fig. 10A, compare set 2, set 3 to set 1, set 4). Strikingly, all C9C4 variants displayed similar gene editing activities (Fig. 10A, set 5 to set 8), with overall reduced activity in comparison to corresponding C4C9 variants (Fig. 10A). In contrast, the use of all new C4/C9-combinuclease variants (v2, v3, v2-1) enabled significantly higher gene editing efficiency than the corresponding 1^{st} generation variants (v1) in the context of "One-Virus" TraFo vector particles, regardless of the order of the C4- and C9-nuclease domains (C4C9 and C9C4). (Fig. 10B, compare set 2 to 4 to set 1 and set 6 to 8 to set 5). No significant difference was detected between v2 and v2-1 C4/C9-combinuclease variants, indicating that both variations of the C4-ORF expression optimization led to similar gene inactivation activities (Fig. 10). In summary, alterations in the peptide linker design and composition had a beneficial effect on the C4/C9-combinuclease-mediated gene editing efficiency in context of "One-Virus" TraFo delivery to target cells.

With respect to the figure legend of Fig. 10A, it is noted that mixing, for example, 500 µl undiluted virus 1 with 500 µl undiluted virus 2 gives a 1:2 dilution of both viruses. This corresponds to a 10^{0.3} dilution. A 1:1 mixture of 1:10 dilutions of both viruses gives a 1:20 dilution of both viruses, which corresponds to a 10^{1.3} dilution.

### Comparison of genome editing efficiency of "One-Virus" TraFo pseudotype vector systems

Naturally, FV vectors cannot be pseudotyped as their budding and release process requires a very specific, direct interaction of the FV capsid with its cognate glycoprotein (FV Env). Heterologous viral glycoproteins cannot functionally replace the FV glycoprotein's function in virus release. Furthermore, the FV Gag protein lacks a membrane targeting domain (MTD), which is the reason for the failure of FVs to release virus-like particles (VLP) in the absence of FV glycoprotein coexpression as observed for orthoretroviruses. Covalent fusion of heterologous MTDs to PFV Gag allowed FV Env-independent particle release. However, due to aberrant capsid structures such PFV vector particles are non-infectious as they fail to reverse transcribe their encapsidated vector genome. To enable pseudotyping of FV vectors, the inventors therefore previously developed a small-molecule dependent association of a heterologous MTD to PFV Gag (Ho et al. 2012). Pseudotyping capability of TraFo "CRISPR 2.0" vectors could potentially broaden their future application in cases where the broad tropism mediated by use of FV Env proteins might not be suitable for specific target tissues and should be replaced by alternative viral glycoproteins or retargeted glycoproteins with cell-specific receptor use as for example on basis of measles- or Nipah virus glycoproteins. Therefore, the inventors examined the compatibility of PFV Gag packaging component variants with either covalently fused N-terminal HIV-1 Gag matrix subunit MTD (M6) or its small molecule-dependent transiently association variant (iDim) (Fig. 2C) with "One-Virus" TraFo "CRISPR 2.0" gene editing. Respective "One-Virus" TraFo "CRISPR 2.0" vector supernatants either harboring the SFVmac Env (SE) glycoprotein or vesicular stomatitis glycoprotein (VSV) were produced by transiently cotransfecting C4/C9-combinuclease and pre-C4/C9-sgRNA transfer vector together with the appropriate packaging constructs into HEK293T packaging cells and if necessary including addition of "Dimerizer". Subsequently, the gene editing efficiency mediated by the different "One-Virus" TraFo vector supernatants was examined by titration on U2OS*gfp* reporter cells as described earlier (Fig. 11). The analysis revealed that both pseudotyping system variants can be used to produce pseudotyped "One-Virus" TraFo "CRISPR 2.0" vector particles with only slightly reduced gene editing capabilities compared to the parental PFV Gag packaging construct when employing the SFVmac glycoprotein (Fig. 11, set 1 to set 3). Furthermore, the results demonstrated that VSV-G pseudotype titers on U2OS*gfp* target cells are slightly lower than that of respective vectors harboring the SFVmac glycoprotein (Fig. 11, compare set 2 and set 3 to set 4 and set 5).

### T-cell receptor inactivation by "One-Virus" TraFo "CRISPR 2.0" vector supernatants in Jurkat cells

The inventors examined whether TraFo "CRISPR 2.0" vector particles enable an efficient gene inactivation of the human T-cell receptor alpha chain (TRAC) gene in human T-cells. To do so, VSV-G pseudotyped "One-Virus" TraFo "CRISPR 2.0" vector particles encoding C4C9v1 nuclease were produced in combination with human TRAC gene specific pre-C4/C9-sgRNAs (TRAC1 or TRAC3) at a 2:10 ratio by transient transfection using the appropriate FV packaging constructs. For comparison, "Two-Virus" TraFo "CRISPR 1.0" vector particles, composed of TraFo particles containing C9-nuclease mRNA and integration-deficient lentiviral (IDLV) vector particles containing RNAP-III-driven C9-sgRNA expression cassettes for the identical TRAC target sequences were produced. Jurkat cells, which are T-cells of a human tumor cell line derived from T-cell acute lymphoblastic leukemia, were transduced with plain "One-Virus" TraFo "CRISPR 2.0" supernatants or a 4:1 mixture (nuclease-encoding to sgRNA-encoding vector supernatant) of plain "Two-Virus" TraFo "CRISPR 1.0" supernatants. TCR cell surface expression of Jurkat target cells was monitored by determining CD3 cell surface expression. More specifically, TRAC gene inactivation was determined by flow cytometry 6 days post infection employing a CD3-specific antibody staining. The results revealed that "One-Virus" TraFo "CRISPR 2.0" vector supernatants were similarly effective (TRAC1) or even more effective (TRAC3) in TRAC KO as the combination of "Two-Virus" TraFo/IDLV "CRISPR 1.0" vectors (Fig. 12). "One-Virus" TraFo "CRISPR 2.0" vector supernatants showed between 40 and 50% TRAC KO for both TRAC1 and TRAC3. "Two-Virus" TraFo/IDLV "CRISPR 1.0" vectors showed between 40 and 50% TRAC KO for TRAC1 and between 30 and 35% TRAC KO for TRAC3. Taken together, this demonstrates that a fully transient efficient delivery of CRISPR genome engineering tools for efficient editing of human genes can be achieved by the "One-Virus" TraFo "CRISPR 2.0" vector system.

Importantly, the "One-Virus" TraFo "CRISPR 2.0" vector system established by the inventors is the first fully transient TraFo vector system with which a functional gene editing in Jurkat cells could be demonstrated. The "Two-Virus" TraFo/IDLV "CRISPR 1.0" vector system, which can achieve a comparable gene editing efficiency in Jurkat cells, requires integration-deficient lentiviral (IDLV) vector particles for expressing the C9-sgRNA component and is thus not fully transient. IDLV vector particles show a low, non-viral mediated chromosome integration of the DNA vector genome as a consequence of non-homologous recombination events. Therefore, the use of IDLV vector particles poses a residual risk for unintended permanent genetic modifications of the target cell. In contrast, the "One-Virus" TraFo "CRISPR 2.0" vector system is completely transient so that unintended permanent genetic modifications of the target cell do not occur. At the same time, the "One-Virus" TraFo "CRISPR 2.0" vector system facilitates the introduction of both CRISPR components, nuclease and sgRNA, in one virus particle into the target cell. This is particularly advantageous in case of target cells that generally show a poor target cell transduction such as, for example, T-cells.

Regarding the observation that the TRAC gene inactivation efficiency achieved in Jurkat cells is considerably lower than the GFP gene inactivation efficiency achieved in U2OS*gfp* cells, the following is noted: In general, lymphocytes (primary lymphocytes or tumor cells) are much less transducible with retroviral vector particles than other cell types such as fibroblasts (e.g. U2OS cells). This explains the lower gene inactivation efficiency in Jurkat cells compared to U2OS*gfp* cells. In addition, it needs to be considered that in Jurkat cells, at least two copies of the target gene (TRAC) are present and must be inactivated to prevent expression of the TCR alpha chain. In contrast, the U2OS*gfp* reporter cells only have a single copy of a constitutively expressing expression cassette for an EGFP protein.

In addition, the inventors assumed that the RNA stability (and thus the stability of the pre-sgRNA) and/or the translation efficiency (and thus the translation efficiency of the nuclease) may be lower in Jurkat cells compared to U2OS*gfp* cells. This may contribute to the lower gene inactivation efficiency in Jurkat cells compared to U2OS*gfp* cells. The inventors were able to confirm their assumption by using the "Two-Virus" TraFo "CRISPR 2.0" vector system and by comparing a simultaneous with a consecutive application of the two components thereof. Accordingly, the inventors either (i) simultaneously applied the TraFo nuclease and the TraFo sgRNA supernatants or (ii) first applied the TraFo nuclease and 24 h later the TraFo sgRNA supernatants of the "Two-Virus" TraFo "CRISPR 2.0" vector system to Jurkat cells. The latter transduction scheme enabled a more effective TRAC KO compared to the simultaneous application scheme (data not shown).

### Gene editing by "One-Virus" TraFo "CRISPR 2.0" base editor vector supernatants

The inventors examined whether the C4/C9 combinuclease design is compatible with the addition of further functional protein domains. Therefore, the inventors covalently connected a GS-linker-Csy4 domain module to the C-terminus of the adenine base editor (ABE) ABE8e (Richter et al. 2020), generating the C9C4v2 BE1 combinuclease (Fig. 2A). The C9C4v2 BE1 combinuclease is composed of covalently connected N-terminal TadA-8e (*Escherichia coli* tRNA adenosine deaminase variant 8e), central Cas9 D10A nickase and C-terminal Csy4 nuclease domains. "One-Virus" TraFo "CRISPR 2.0" vector supernatants were generated by coexpression of C9C4v2 BE1 or C9C4v2 and pre-C4/C9-sgRNAs specific for GFP (gfp-148, c.187 A>G) (Fig. 13A). The c.187 A>G sgRNA was previously shown to result in a Y66H amino acid mutation due to base editor mediated editing of the gfp ORF, thereby converting GFP into BFP (blue fluorescent protein). GFP and BFP fluorescence of U2OS*gfp* target cells transduced with the different "One-Virus" TraFo "CRISPR 2.0" supernatants was examined seven days post infection by flow cytometry. GFP KO analysis revealed that pre-C4/C9 gfp-148 resulted in significant gene inactivation activities when combined with either C9C4v2 or C9C4v2 BE1 nucleases, though inactivation was reduced for "One-Virus" TraFo C9C4v2 BE1 supernatants (Fig. 13B). In gfp c.187 A>G pre-C4/C9 sgRNA transduced samples also a significant GFP KO was detectable either in combination with C9C4v2 or C9C4v2 BE1 nucleases, though at a lower level than when using pre-C4/C9 gfp-148 sgRNA. At the same time in about 1/3 of the GFP-negative cells, a blue fluorescent signal was detectable in cells transduced in the gfp c.187 A>G sgRNA containing TraFo supernatants, which was absent in all other samples including those transduced with gfp-148 containing TraFo supernatants (Fig. 13C). This indicated a GFP to BFP color-switch due to successful base editing at codon 66 of the *gfp* ORF. Thus, the functionality of the C4/C9 combinuclease design can be further expanded by covalent coupling of additional protein domains such as an adenosine deaminase.

### References

Hamann, M.V. et al. Efficient transient genetic manipulation in vitro and in vivo by prototype foamy virus-mediated nonviral RNA transfer. Molecular Therapy 2014;22(8):1460-1471.
Ho, Y.P. et al. A small-molecule-controlled system for efficient pseudotyping of prototype foamy virus vectors. Molecular Therapy 2012;20(6):1167-1176.
Lindel, F. et al. TraFo-CRISPR: Enhanced Genome Engineering by Transient Foamy Virus Vector-Mediated Delivery of CRISPR/Cas9 Components. Molecular Therapy Nucleic Acids 2019;18:708-726.
Richter, M.F. et al. Phage-assisted evolution of an adenine base editor with improved Cas domain compatibility and activity. Nature Biotechnology 2020;38(7):883-891.
Tsai, S.Q. et al. Dimeric CRISPR RNA-guided Fokl nucleases for highly specific genome editing. Nature Biotechnology 2014;32(6):569-576.
EP 3 822 346 A1
WO 2012/152632 A1

## Claims

1. Use of an artificial protein or of a nucleic acid encoding the artificial protein for providing a functional ribonucleoprotein (RNP) complex, wherein the artificial protein comprises
a Csy4 protein,
a Cas9 protein, and
a linker of 5 to 60 amino acids connecting the Csy4 protein and the Cas9 protein,
wherein the linker does not comprise a 2A peptide.

2. The use according to claim 1, wherein the linker links the C-terminus of the Csy4 protein to the N-terminus of the Cas9 protein or wherein the linker links the C-terminus of the Cas9 protein to the N-terminus of the Csy4 protein.

3. The use according to claim 1 or claim 2, wherein the linker comprises at least one GGGGS (SEQ ID NO.: 2) motif, preferably at least three GGGGS (SEQ ID NO.: 2) motifs, more preferred seven GGGGS (SEQ ID NO.: 2) motifs.

4. The use according to any one of claims 1 to 3, wherein the functional RNP complex is provided in a target cell and the artificial protein further comprises at least one nuclear localization sequence.

5. The use according to claim 4, wherein the at least one nuclear localization sequence is located at the N-terminus of the artificial protein or at the C-terminus of the artificial protein.

6. The use according to claim 4 or claim 5, wherein the target cell preferably is a mammalian cell, more preferred a human cell.

7. An *in vitro* method for providing a functional ribonucleoprotein (RNP) complex in a target cell, the method comprising the step of introducing a component (a) and a component (b) into the target cell,
wherein the component (a) is an artificial protein or a nucleic acid encoding the artificial protein, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide;
and wherein the component (b) is
either
(b-1) a Csy4/Cas9-single guide RNA (Csy4/Cas9-sgRNA), wherein the Csy4/Cas9-sgRNA comprises
a classical Cas9-sgRNA sequence, and
a Csy4 cleavage sequence located downstream of the classical Cas9-sgRNA sequence,
or
(b-2) a pre-Csy4/Cas9-sgRNA or a nucleic acid encoding the pre-Csy4/Cas9-sgRNA, wherein the pre-Csy4/Cas9-sgRNA comprises a classical Cas9-sgRNA sequence,
a Csy4 cleavage sequence located downstream of the classical Cas9-sgRNA sequence, and
an additional Csy4 cleavage sequence located directly upstream of the classical Cas9-sgRNA sequence.

8. The method according to claim 7, wherein the component (a) and the component (b) are introduced into the target cell by transduction of the target cell using at least one recombinant virus.

9. The method according to claim 8, wherein the component (a) and the component (b) are separately introduced into the target cell by using a first virus and a second virus for the transduction of the target cell.

10. The method according to claim 9, wherein the first virus is a first retrovirus and the second virus is a second retrovirus, wherein the first retrovirus is a reverse transcriptase-deficient prototype foamy virus (PFV) that is based on a minimal PFV vector system and/or the second retrovirus is a reverse transcriptase-deficient PFV that is based on a minimal PFV vector system, wherein the minimal PFV vector system comprises at least one viral packaging vector and a minimal transfer vector, wherein the at least one viral packaging vector comprises sequences encoding at least a FV Gag protein and an Env protein, wherein the minimal transfer vector comprises a nucleic acid encoding the artificial protein or a nucleic acid encoding the pre-Csy4/Cas9-sgRNA, as applicable, and wherein the minimal transfer vector does not comprise any sequences of the PFV.

11. The method according to claim 8, wherein the component (a) and the component (b) are introduced into the target cell together by using one virus for the transduction of the target cell.

12. The method according to claim 11, wherein the virus is a foamy virus, wherein the foamy virus is a reverse transcriptase-deficient PFV that is based on a minimal PFV vector system, wherein the minimal PFV vector system comprises at least one viral packaging vector, a first minimal transfer vector and a second minimal transfer vector, wherein the at least one viral packaging vector comprises sequences encoding at least a FV Gag protein and an Env protein, wherein the first minimal transfer vector comprises a nucleic acid encoding the artificial protein, wherein the second minimal transfer vector comprises a nucleic acid encoding the pre-Csy4/Cas9-sgRNA, and wherein the first and the second minimal transfer vector do not comprise any sequences of the PFV.

13. The method according to any one of claims 7 to 12, wherein the Csy4 cleavage sequence comprises the sequence
GUUCACUGCCGUAUAGGCAG (SEQ ID NO.: 11),
GUUCACUGCCGUAUAGGCAGCUAAGAAA (SEQ ID NO.: 12),
GUACACUGCCGUAUAGGCAG (SEQ ID NO.: 13),
GUAAACUGCCGUAUAGGCAG (SEQ ID NO.: 14), or
GUUCCCUGCCGUAUAGGCAG (SEQ ID NO.: 15).

14. The method according to any one of claims 7 to 13, wherein the pre-Csy4/Cas9-sgRNA further comprises at least one additional classical Cas9-sgRNA sequence arranged downstream of the Csy4 cleavage sequence that is located downstream of the classical Cas9-sgRNA sequence, wherein each additional classical Cas9-sgRNA sequence is accompanied by an additional Csy4 cleavage sequence located downstream of the additional classical Cas9-sgRNA sequence.

15. Use of a nucleic acid encoding an artificial protein for producing recombinant virus particles, wherein the recombinant virus particles are suitable for providing a functional ribonucleoprotein (RNP) complex in a target cell, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide.

16. An *in vitro* method for producing recombinant virus particles, wherein the recombinant virus particles are suitable for providing a functional ribonucleoprotein (RNP) complex in a target cell, the method comprising the steps of
(a) introducing
(i) a nucleic acid encoding an artificial protein, wherein the artificial protein comprises
a Csy4 protein,
a Cas9 protein,
a linker of 5 to 60 amino acids connecting the Csy4 protein and the Cas9 protein, and
at least one nuclear localization sequence,
wherein the linker does not comprise a 2A peptide;
and
(ii) at least one viral packaging vector
into a plurality of packaging cells, thereby obtaining a plurality of producer cells;
(b) culturing the producer cells in a medium, thereby obtaining the recombinant virus particles in the medium; and
(c) separating the medium from the producer cells.

17. The method according to claim 16, wherein step (a) further comprises introducing either
(iii-1) a Csy4/Cas9-single guide RNA (Csy4/Cas9-sgRNA), wherein the Csy4/Cas9-sgRNA comprises
a classical Cas9-sgRNA sequence, and
a Csy4 cleavage sequence located downstream of the classical Cas9-sgRNA sequence,
or
(iii-2) a pre-Csy4/Cas9-sgRNA or a nucleic acid encoding the pre-Csy4/Cas9-sgRNA, wherein the pre-Csy4/Cas9-sgRNA comprises a classical Cas9-sgRNA sequence,
a Csy4 cleavage sequence located downstream of the classical Cas9-sgRNA sequence, and
an additional Csy4 cleavage sequence located directly upstream of the classical Cas9-sgRNA sequence,
into the plurality of packaging cells.
